# EUROPÄISCHE PATENTSCHRIFT

(11) **EP 2 254 864 B1**
(45) Veröffentlichungstag und Bekanntmachung des Hinweises auf die Patenterteilung: **14.05.2014**
(21) Anmeldenummer: 09713839.0
(22) Anmeldetag: 25.02.2009
(51) Int. Cl.: C07D 209/08, C07D 209/12, C07D 403/12, C07D 471/04, C07F 7/08, A61K 31/404, A61K 31/437, A61P 25/04, A61P 29/00

(54) **SUBSTITUIERTE 4-AMINOCYCLOHEXAN-DERIVATE ZUR BEHANDLUNG VON SCHMERZ**
SUBSTITUTED 4-AMINOCYCLOHEXANE DERIVATIVES FOR THE TREATMENT OF PAIN
DÉRIVÉS DE 4-AMINOCYCLOHEXANE SUBSTITUÉS POUR LE TRAITEMENT DE LA DOULEUR

(30) Priorität: 26.02.2008 EP 08003442
(43) Veröffentlichungstag der Anmeldung: 01.12.2010
(73) Patentinhaber: Grünenthal GmbH, 52078 Aachen (DE)
(72) Erfinder: SCHUNK, Stefan, 52080 Aachen (DE); ZEMOLKA, Saskia, 52066 Aachen (DE); LINZ, Klaus, 53359 Rheinbach (DE); ENGLBERGER, Werner, 52223 Stolberg (DE); THEIL, Fritz, 12247 Berlin (DE)
(74) Vertreter: Bülle, Jan
(86) Internationale Anmeldenummer: PCT/EP2009/001329
(87) Internationale Veröffentlichungsnummer: WO 2009/106307

(56) Entgegenhaltungen:
- WO-A-03/080557
- WO-A-2004/043902
- PALIN R ET AL: "Synthesis and SAR studies of 3-phenoxypropyl piperidine analogues as ORL1 (NOP) receptor agonists" BIOORGANIC & MEDICINAL CHEMISTRY LETTERS, OXFORD, GB, Bd. 15, Nr. 3, 1. Februar 2005 (2005-02-01), Seiten 589-593, XP004739660 ISSN: 0960-894X

## Beschreibung

Die vorliegende Erfindung betrifft substituierte 4-Aminocyclohexan-Derivate, Verfahren zu deren Herstellung, Arzneimittel enthaltend diese Verbindungen und die Verwendung von substituierten 4-Aminocyclohexan-Derivaten zur Herstellung von Arzneimitteln.

Das Heptadekapeptid Nociceptin ist ein endogener Ligand des ORL1 (Opioid-Receptor-Like)-Rezeptors (Meunier et al., Nature 377, 1995, S. 532-535), der zu der Familie der Opioid Rezeptoren gehört und in vielen Regionen des Gehirns und des Rückenmarks zu finden ist und eine hohe Affinität für den ORL1-Rezeptor aufweist. Der ORL1-Rezeptor ist homolog zu den µ, κ und δ Opioid-Rezeptoren und die Aminosäuresequenz des Nociceptin-Peptids weist eine starke Ähnlichkeit mit denen der bekannten Opioidpeptide auf. Die durch das Nociceptin induzierte Aktivierung des Rezeptors führt über die Kopplung mit G_{i/o}-Proteinen zu einer Inhibierung der Adenylatcyclase (Meunier et al., Nature 377, 1995, S. 532-535).

Das Nociceptin-Peptid zeigt nach intercerebroventicularer Applikation eine pronociceptive und hyperalgetische Aktivität in verschiedenen Tiermodellen (Reinscheid et al., Science 270, 1995, S. 792-794). Diese Befunde können als Hemmung der stressinduzierten Analgesie erklärt werden (Mogil et al., Neuroscience 75, 1996, S. 333-337). In diesem Zusammenhang konnte auch eine anxiolytische Aktivität des Nociceptin nachgewiesen werden (Jenck et al., Proc. Natl. Acad. Sci. USA 94, 1997, 14854-14858).

Auf der anderen Seite konnte in verschiedenen Tiermodellen, insbesondere nach intrathekaler Applikation, auch ein antinociceptiver Effekt von Nociceptin gezeigt werden. Nociceptin wirkt antinociceptiv in verschiedenen Schmerzmodellen, beispielsweise im Tail Flick-Test in der Maus (King et al., Neurosci. Lett., 223, 1997, 113-116. In Modellen für neuropathische Schmerzen konnte ebenfalls eine antinociceptive Wirkung von Nociceptin nachgewiesen, die insofern besonders interessant ist, als dass die Wirksamkeit von Nociceptin nach Axotomie von Spinalnerven zunimmt. Dies steht im Gegensatz zu den klassischen Opioiden, deren Wirksamkeit unter diesen Bedingungen abnimmt (Abdulla und Smith, J. Neurosci., 18, 1998, S. 9685-9694).

Der ORL1-Rezeptor ist außerdem noch an der Regulation weiterer physiologischer und pathophysiologischer Prozesse beteiligt. Hierzu gehören unter anderem Lernen und Gedächtnisbildung (Manabe et al., Nature, 394, 1997, S. 577-581), Hörvermögen (Nishi et al., EMBO J., 16, 1997, S. 1858-1864) sowie zahlreiche weitere Prozesse. In einem Übersichtsartikel von Calo et al. (Br.J. Pharmacol., 129, 2000, 1261 - 1283) wird ein Überblick über die Indikationen oder biologischen Vorgänge gegeben, in denen der ORL1-Rezeptor eine Rolle spielt oder mit hoher Wahrscheinlichkeit spielen könnte. Genannt werden u.a.: Analgesie, Stimulation und Regulation der Nahrungsaufnahme, Einfluß auf µ-Agonisten wie Morphin, Behandlung von Entzugserscheinungen, Reduzierung des Suchtpotentials von Opioiden, Anxiolyse, Modulation der Bewegungsaktivität, Gedächtnis-Störungen, Epilepsie; Modulation der Neurotransmitter-Ausschüttung, insbesondere von Glutamat, Serotonin und Dopamin, und damit neurodegenerative Erkrankungen; Beeinflussung des cardiovaskulären Systems, Auslösung einer Erektion; Diurese, Antinatriurese, Elektrolyt-Haushalt, arterieller Blutdruck, Wasserspeicher-Krankheiten, intestinale Motilität (Diarrhoe), relaxierende Effekte auf die Atemwege, Mikturations Reflex (Harninkontinenz). Weiter wird die Verwendung von Agonisten und Antagonisten als Anoretika, Analgetika (auch in Coadministration mit Opioiden) oder Nootropika diskutiert.

Entsprechend vielfältig sind die Anwendungsmöglichkeiten von Verbindungen, die an den ORL1-Rezeptor binden und diesen aktivieren oder inhibieren. Neben diesem spielen aber gerade im Bereich der Schmerztherapie aber auch anderen der genannten Indikationen Opioidrezeptoren wie der µ-Rezeptor, aber auch die anderen Subtypen dieser Opioidrezeptoren, nämlich δ und κ eine große Rolle. Entsprechend ist es günstig, wenn die Verbindung auch Wirkung an diesen Opioidrezeptoren zeigen.

Bei der Therapie von neuropathischem Schmerz mit Opioiden müssen die Opioide üblicherweise höher dosiert werden, als dies zur Behandlung von Akutschmerz nötig wäre. Der Nachteil für die Patienten liegt darin, dass sie üblicherweise an stärkeren opioidtypischen Nebenwirkungen leiden. Daher wäre es von Vorteil, Verbindungen zu finden, bei der möglichst eine geringere, zumindest aber keine höhere Dosis als die zur Behandlung von Akutschmerz notwendige Dosis zur Behandlung von Neuropathieschmerz verabreicht werden muss.

In WO2004043902 werden 4-Hydroxymethyl-1-Aryl-Cyclohexylamin-Verbindungen. Es werden zwar u.a. auch Indol-Derivate erwährt, deratige Beispiele sind jedoch nicht offenbart und auch die Art der Verknüpfung des Indol-Restes an die Leitstruktur wird nicht näher erläutert. Für vier Verbindungen, worin R⁵ Phenyl oder 4-Fluorphenyl bedeutet, sind Rezeptor-Bindungsdaten und für zwei Verbindungen eine Wirksamkeit im Tail-flick Test, einem Akutschmerzmodell, beschrieben. Akutschmerz ist jedoch im Vergleich zu chronischem bzw. neuropathischem Schmerz häufig gut therapierbar, so dass insbesondere ein Bedarf besteht an neuen Arzneimitteln zur Behandlung von chronischem und neuropathischem Schmerz.

Ferner besteht ein Bedarf an verbindungen, welche eine allenfalls geringe Affinität zum hERG-Ionenkanal, zum L-Typ Calcium-Ionenkanal (Phenylalkylamin-, Benzothiazepin-, Dihydropyridin-Bindungsstellen) bzw. zum Natrium-Kanal im BTX-Assay (Batrachotoxin) aufweisen, da dies sonst jeweils als Anzeichen für kardiovaskuläre Nebenwirkungen gedeutet werden kann. Ferner sollte die Löslichkeit in wässrigen Medien ausreichend sein, da sich dies sonst u.a. negativ auf die Bioverfügbarkeit auswirken kann. Darüber hinaus sollte die chemische Stabilität der Verbindungen ausreichend sein. Zeigen die Verbindungen keine ausreichende pH-, UV- bzw. Oxidationsstabilität, so kann sich dies u.a. negativ auf die Lagerstabilität und auch auf die orale Bioverfügbarkeit auswirken.Auch ein günstiges PK/PD (Pharmakokinetik/Pharmakodynamik)-Profil ist wünschenswert, damit z.B. die Wirdauer nicht zu lang ist. Ferner sollte auch die metabolische Stabilität nicht zu gering sein, was sich auf eine erhöhte Bioverfügbarkeit auswirken kann. Auch eine schwache oder nicht vorhandene Wechselwirkung mit Transportermolekülen, die an der Aufnahme und der Ausscheidung von Arzneistoffen beteiligt sind, ist als Hinweis auf eine verbesserte Bioverfügbarkeit und allenfalls geringe Arzneimittelwechselwirkungen zu werten. Ferner sollten auch die Wechselwirkungen mit den am Abbau und der Ausscheidung von Arzneistoffen beteiligten Enzymen möglichst gering sein, da solche Testergebnisse ebenfalls darauf hindeuten, dass allenfalls geringe oder überhaupt keine Arzneimittelwechselwirkungen zu erwarten sind.

Der Erfindung liegt die Aufgabe zugrunde, Verbindungen zur Verfügung zu stellen, welche sich zu pharmazeutischen Zwecken eignen und Vorteile gegenüber den Verbindungen des Standes der Technik aufweisen. Die Verbindungen sollten sich insbesondere zur Behandlung von chronischem und neuropathischem Schmerz eignen.

Diese Aufgabe wird durch den Gegenstand der Patentansprüche gelöst.

Es wurde überraschenderweise gefunden, dass sich substituierte 4-Aminocyclohexan-Derivate der allgemeinen Formel I besonders gur zur Behandlung von chronischem und neuropathischem Schmerz eignen.

Gegenstand der Erfindung sind substituierte 4-Aminocyclohexan-Derivate der allgemeinen Formel I, 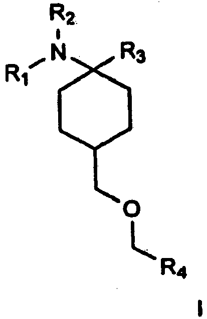 worin
R¹ und R² unabhängig voneinander Methyl oder H bedeuten oder die Reste R¹ und R² unter Einschluss des N-Atoms einen Ring bilden und gemeinsam (CH₂)₃ bedeuten;
R³ für Phenyl, unsubstituiert oder einfach substituiert mit F, Cl, CN, CH₃; Thienyl; oder n-Butyl, unsubstituiert oder einfach oder mehrfach substituiert mit OCH₃, OH oder OC₂H₅, insbesondere mit OCH₃; steht.
R⁴ für 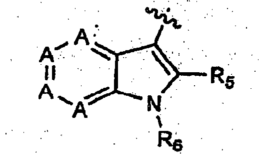 steht,
worin
A für N oder CR⁷⁻¹⁰ steht, wobei A höchstens einmal N bedeutet;
R⁵_{.} R⁷, R⁸, R⁹ und R¹⁰ unabhängig voneinander für H, F, Cl, Br, CN, CH₃, C₂H₅ NH₂, *tert*.-Butyl, Si(Ethyl)₃, Si(Methyl)₂(*tert*.-Butyl), SO₂CH₃, C(O)CH₃, NO₂, SH, CF₃, OH, OCH₃, OC₂H₅ oder N(CH₃)₂, insbesondere Si(Ethyl)₃, Si(Methyl)₂(*tert*.-Butyl), CN, CF₃, F, C(O)CH₃, SO₂CH₃ oder CH₃, und
R⁸ für H, CH₃ oder C(O)CH₃ steht.
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Die Ausdrücke "C₁₋₆-Alkyl" und "C₁₋₃-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit mit 1, 2, 3, 4, 5 oder 6 C-Atomen bzw. 1, 2 oder 3 C-Atomen, d. h. C₁₋₅-Alkanyle, C₂₋₅-Alkenyle und C₂₋₅-Alkinyle bzw. C₁₋₃-Alkanyle, C₂₋₃-Alkenyle und C₂₋₃-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, n-Hexyl, 2-Hexyl; Ethylenyl (Vinyl), Ethinyl, Propenyl (-CH₂CH=CH₂, -CH=CH-CH₃, -C(=CH₂)-CH₃), Propinyl (-CH-C≡CH, - C≡C-CH₃), 1,1-Dimethylethyl, 1,1-Dimethylpropyl, Butenyl, Butinyl, Pentenyl, Pentinyl, Hexyl,

Hexenyl oder Hexinyl umfaßt. Besonders bevorzugt im Sinne dieser Erfindung sind Methyl und Ethyl.

Der Ausdruck "Cycloalkyl" bedeutet für die Zwecke dieser Erfindung cyclische Kohlenwasserstoffe mit 3, 4, 5, 6, 7 oder 8 Kohlenstoffatomen, wobei die Kohlenwasserstoffe gesättigt oder ungesättigt (aber nicht aromatisch), unsubstituiert oder ein- oder mehrfach substituiert sein können. Vorteilhaft ist Cycloalkyl aus der Gruppe ausgewählt, die Cyclopropyl, Cyclo-butyl, Cyclopentyl, Cyclohexyl, Cycloheptyl, Cyclooctyl, Cyclopentenyl, Cyclohexenyl, Cyclo-heptenyl und Cyclooctenyl enthält. Besonders bevorzugt im Sinne dieser Erfindung sind Cyclobutyl, Cyclopentyl und Cyclohexyl.

Unter dem Begriff (CH₂)₃₋₆ ist -CH₂-CH₂-CH₂-, -CH₂-CH₂-CH₂-CH₂-, -CH₂-CH₂-CH₂-CH₂-CH₂-und CH₂-CH₂-CH₂-CH₂-CH₂-CH₂- zu verstehen.

Der Ausdruck "Aryl" bedeutet im Sinne dieser Erfindung carbocyclische Ringsysteme mit bis zu 14 Ringgliedern mit mindestens einem aromatischen Ring, aber ohne Heteroatome in nur einem der Ringe, u.a. Phenyle, Naphthyle und Phenanthrenyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Besonders vorteilhaft sind Phenyl- oder NaphthylReste.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bi- oder polycyclischen Systems mit bis zu 14 Ringgliedern sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phtalazinyl, Pyrazolyl, Imidazolyt, Thiazolyl, Oxazolyl, Isoxazoyl, Pyridinyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl enthält, wobei die Bindung an die Verbindungen der allgemeinen Struktur I über jedes beliebige und mögliche Ringglied des Heteroaryl-Restes erfolgen kann.

Im Zusammenhang mit Definitionen von Substituenten bedeutet "Alkyl" "C₁₋₆-Alkyl", soweit "Alkyl" nicht näher spezifiziert ist.

Im Zusammenhang mit "Alkyl" versteht man unter dem Begriff "substituiert" im Sinne dieser Erfindung die Substitution eines oder mehrerer Wasserstoffreste durch F, Cl, Br, I, -CN, NH₂, NH-Alkyl, NH-Aryl, NH-Heteroaryl, NH-Cycloalkyl, NH-Alkyl-Aryl, NH-Alkyl-Heteroaryl, NH-Alkyl-OH, N(Alkyl)₂, N(Alkyl-Aryl)₂, N(Alkyl-Heteroaryl)₂, N(Cycloalkyl)₂, N(Alkyl-OH)₂, NO₂, SH, S-Alkyl, S-Aryl, S-Heteroaryl, S-Alkyl-Aryl, S-Alkyl-Heteroaryl, S-Cycloalkyl, S-Alkyl-OH, S-Alkyl-SH, OH, O-Alkyl, O-Aryl, O-Heteroaryl, O-Alkyl-Aryl, O-Alkyl-Heteroaryl, O-Cycloalkyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, C(=S)C₁₋₆-Alkyl-Aryl, C(=O)-Heteroaryl, C(=S)-Heteroaryl, C(=O)-Cycloalkyl, C(=S)-Cycloalkyl, CO₂H, CO₂-Alkyl, CO₂-Alkyl-Aryl, C(=O)NH₂, C(=O)NH-Alkyl, C(=O)NHAryl, C(=O)NH-Cycloalkyl, C(=O)N(Alkyl)₂, C(=O)N(Alkyl-Aryl)₂, C(=O)N(Alkyl-Heteroaryl)₂, C(=O)N(Cycloalkyl)₂, SO-Alkyl, SO₂-Alkyl, SO₂NH₂, SO₃H, PO(O-C₁₋₆-Alkyl)₂ =O, =S, wobei unter mehrfach substituierten Resten solche Reste zu verstehen sind, die entweder an verschiedenen oder an gleichen Atomen mehrfach, z. B. zwei- oder dreifach, substituiert sind, beispielsweise dreifach am gleichen C-Atom wie im Falle von CF₃ oder -CH₂CF₃ oder an verschiedenen Stellen wie im Falle von -CH(OH)-CH=CH-CHCl₂. Die Mehrfachsubstitution kann mit dem gleichen oder mit verschiedenen Substituenten erfolgen. Ggf. kann ein Substituent auch seinerseits substituiert sein; so umfaßt -OAlkyl u.a. auch -0-CH₂-CH₂-O-CH₂-CH₂-OH. Bevorzugt im Sinne dieser Erfindung ist es, wenn Alkyl substituiert ist mit F, Cl, Br, I, CN, CH₃, C₂H₅, NH₂, NO₂, SH, CF₃, OH, OCH₃, Cyclopentyl, Cyclohexyl, OC₂H₅ oder N(CH₃)₂, vorzugsweise F, Cl, Br, I, CN, CH₃, C₂H₅, NH₂, NO₂, SH, CF₃, OH, OCH₃, OC₂H₅ oder N(CH₃)₂. Ganz besonders bevorzugt ist es, wenn Alkyl substituiert ist mit OH, OCH₃ oder OC₂H₅.

In Bezug auf "Aryl", _{"}Indol" "Pyrrolo[2,3-b]pyridin", "Pyrrolo[2,3-c]pyridin, "Pyrrolo[3,2-c]pyridin", "Pyrrolo[3,2-b]pyridin" oder "Heteroaryl" versteht man im Sinne dieser Erfindung unter "ein- oder mehrfach substituiert" die ein- oder mehrfache, z.B. zwei-, drei- vier- oder fünffache, Substitution eines oder mehrerer Wasserstoffatome des Ringsystems durch F, Cl, Br, I, CN, NH₂, NH-Alkyl, NH-Aryl, NH-Heteroaryl, NH-Alkyl-Aryl, NH-Alkyl-Heteroaryl, NH-Cycloalkyl, NH-Alkyl-OH, N(Alkyl)₂, N(Alkyl-Aryl)₂, N(Alkyl-Heteroaryl)₂, N(Cycloalkyl)₂, N(Alkyl-OH)₂, NO₂, SH, S-Alkyl, S-Cycloalkyl, S-Aryl, S-Heteroaryl, S-Alkyl-Aryl, S-Alkyl-Heteroaryl, S-Cycloalkyl, S-Alkyl-OH, S-Alkyl-SH, OH, O-Alkyl, O-Cycloalkyl, O-Aryl, O-Heteroaryl, O-Alkyl-Aryl, O-Alkyl-Heteroaryl, O-Cycloalkyl, O-Alkyl-OH, CHO, C(=O)C₁₋₆-Alkyl, C(=S)C₁₋₆-Alkyl, C(=O)Aryl, C(=S)Aryl, C(=O)-C₁₋₆-Alkyl-Aryl, C(=S)C₁₋₆-Alkyl-Aryl, C(=O)-Heteroaryl, C(=S)-Heteroaryl, C(=O)-Cycloalkyl, C(=S)-Cycloalkyl, CO₂H, CO₂-Alkyl, CO₂-Alkyl-Aryl, C(=O)NH₂, C(=O)NH-Alkyl, C(=O)NHAryl, C(=O)NH-Cycloalkyl, C(=O)-N(Alkyl)₂, C(=O)N(Alkyl-Aryl)₂, C(=O)N(Alkyl-Heteroaryl)₂, C(=O)N(Cycloalkyl)₂, S(O)-Alkyl, S(O)-Aryl, SO₂-Alkyl, SO₂-Aryl, SO₂NH₂, SO₃H, Si(Alkyl)₃, CF₃; Alkyl, Cycloalkyl, Aryl und/oder Heteroaryl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten. Wenn "Aryl", "Indol" "Pyrrolo[2,3-b]pyridin", "Pyrrolo[2,3-c]pyridin, "Pyrrolo[3,2-c]pyridin", "Pyrrolo[3,2-b]pyridin" oder "Heteroaryl" seinerseits mit einem gegebenenfalls über eine Brücke verknüpften Aryl- oder Heteroarylrest substituiert ist, so ist dieser Substituient vorzugweise seinerseits unsubstituiert oder einfach oder mehrfach substituiert mit F, Cl, Br, I, CN, CH₃, C₂H₅, NH₂, NO₂, SH, CF₃, OH, OCH₃, OC₂H₅ oder N(CH₃)₂.

Besonders bevorzugt im Sinne dieser Erfindung ist es, wenn "Aryl", "Indol" "Pyrrolo[2,3-b]pyridin", "Pyrrolo[2,3-c]pyridin, "Pyrrolo[3,2-c]pyridin", "Pyrrolo[3,2-b]pyridin" oder "Heteroaryl" substituiert sind mit F, Cl, Br, I, CN, CH₃, C₂H_{5'} NH₂, tert.-Butyl, Si(Ethyl)₃, Si(Methyl)₂-(*tert*.-Butyl), SO₂CH₃, C(O)CH₃, NO₂, SH, CF₃, OH, OCH₃, OC₂H₅ oder N(CH₃)₂.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - verträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Acetylsalicylsäure, Hippursäure und/oder Asparaginsäure. Besonders bevorzugt ist das Hydrochlorid-Salz, das Citrat und das Hemicitrat.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid und das Citrat. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier- verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch- insbesondere bei Anwendung im Menschen und/oder Säugetier - veträglich ist. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Bevorzugt im Sinne dieser Erfindung sind substituierte 4-Aminocyclohexan-Derivate, worin "Alkyl substituiert" für die Substitution eines Wasserstoffrestes durch F, Cl, Br, I, -CN, NH₂, NH-C₁₋₆-Alkyl, NH-C₁₋₆-Alkyl-OH, C₁₋₆-Alkyl, N(C₁₋₆-Alkyl)₂, N(C₁₋₆-Alkyl-OH)₂, NO₂, SH, S-C₁₋₈-Alkyl, S-Benzyl, O-C₁₋₆-Alkyl, OH, O-C₁₋₆-Alkyl-OH, =O, O-Benzyl, C(=O)C₁₋₆-Alkyl, C(=O)OC₁₋₆-Alkyl, Phenyl oder Benzyl steht,
und "Aryl substituiert", "Indol substituiert" "Pyrrolo[2,3-b]pyridin substituiert*, "Pyrrolo[2,3-c]pyridin substituiert", "Pyrrolo[3,2-c]pyridin substituiert", "Pyrrolo[3,2-b]pyridin substituiert" oder "Heteroaryl substituiert" für die ein- oder mehrfache, z. B. zwei-, drei- oder vierfache, Substitution eines oder mehrerer Wasserstoffatome des Ringsystems durch F, Cl, Br, I, CN, NH₂, NH-C₁₋₆-Alkyl, NH- C₁₋₆-Alkyl-OH, N(C₁₋₆Alkyl)₂, N(C₁₋₆-Alkyl-OH)₂, NO₂, SH, S-C₁₋₆-Alkyl, OH, O-_{C1-6}-_{Alk}yl, O-C₁₋₆Alkyl-OH, C(=O)-Aryl; C(=O)C₁₋₆-Alkyl, C(=O)NHC₁₋₆-Alkyl; C(=O)-N-Morpholin; C(=O)-Piperidin; (C=O)-Pyrrolidin; (C=O)-Piperazin; NHSO₂C₁₋₆-Alkyl, NHCOC₁₋₆-Alkyl, CO₂H, CH₂SO₂-Phenyl, SO₂-Alkyl, Si(Alkyl)₃ CO₂-C₁₋₆-Alkyl, OCF₃, CF₃, 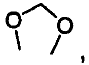 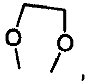 C₁₋₆-Alkyl, Benzyloxy, Phenoxy, Phenyl, Pyridyl, Alkylaryl, Thienyl oder Furyl, wobei Aryl- und Heteroaryl-Substituenten ihrerseits mit F, Cl, Br, I, CN, CH₃, C₂H₅' NH₂, NO₂, SH, CF₃, OH, OCH₃, OC₂H₅ oder N(CH₃)₂ substituiert sein können; steht.
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Ganz besonders bevorzugt sind substituierte 4-Aminocyclohexan-Derivate, worin R¹ und R² unabhängig voneinander Methyl oder H bedeuten, vorzugsweise Methyl.

Insbesondere bevorzugt sind Strukturen, in denen
R⁴ einer der Strukturen L, M oder N bedeutet 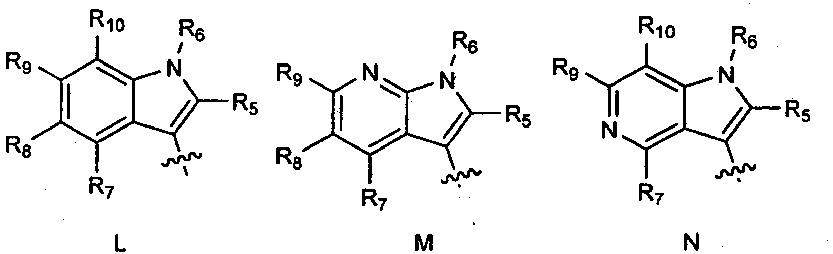 wobei die Reste R⁵, R⁷, R⁸, R⁹ und R¹⁰ unabhängig voneinander für H, F, Cl, Br, CN, CH₃, C₂H₅' NH₂, tert.-Butyl, Si(Ethyl)₃, Si(Methyl)₂(tert.-Butyl), SO₂CH₃, C(O)CH₃, NO₂, SH, CF₃, OH, OCH₃, OC₂H₅ oder N(CH₃)₂, insbesondere Si(Ethyl)₃, Si(Methyl)₂(tert.-Butyl), CN, CF₃, F, C(O)CH₃, SO₂CH₃ oder CH₃, und
R⁶ für H, CH₃ oder C(O)CH₃ steht,
wobei es bevorzugt ist, dass R⁴ für die Substruktur der allgemeinen Formel L steht.

Bevorzugt sind auch substituierte 4-Aminocyclohexan-Derivate, worin R⁴ für 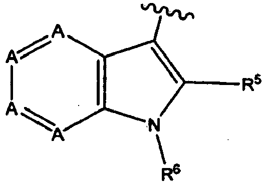 für eine der Strukturen O₃, P₃, Q₃, R₃ oder S₃ steht.

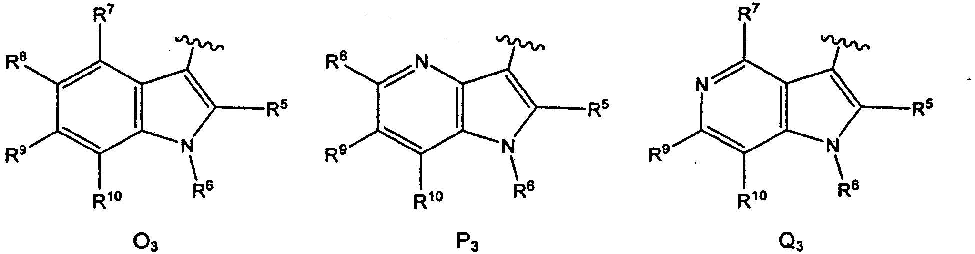

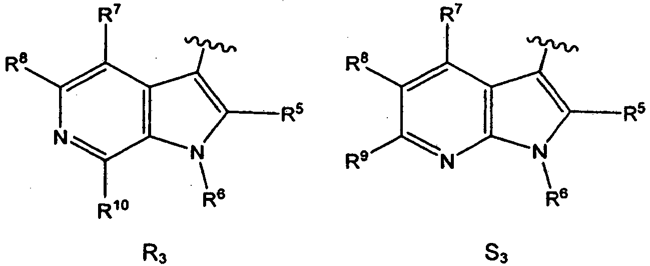

Ganz besonders bevorzugt sind substituierte 4-Aminocyclohexan-Derivate aus der Gruppe:
1 [1-Phenyl-4-(2-triethylsilanyl-1H-indol-3-ylmethoxymethyl)cyclohexyl]dimethyl-amin (ggf. eines von zwei möglichen Diastereomeren)
2 [1-Phenyl-4-(1H-indol-3-ylmethoxymethyl)cyclohexyl]dimethylamin (ggf. eines von zwei möglichen Diastereomeren)
3 1-Phenyl-4-(((2-(tert-butyldimethylsilyl)-5-cyano-1H-indol-3-yl)methoxy)methyl)-N,N-dimethylcyclohexanamin (ggf. eines von zwei möglichen Diastereomeren)
4 1-Phenyl-4-(((5-cyano-2-(triethylsilyl)-1H-indol-3-yl)methoxy)methyl)-N,N-dimethylcyclohexanamin (ggf. eines von zwei möglichen Diastereomeren)
5 1-Phenyl-4-(((5-cyano-1H-indol-3-yl)methoxy)methyl)-N,N-dimethyl-cyclohexanamin (ggf. eines von zwei möglichen Diastereomeren)
6 1-Phenyl-4-(((2-(tert-butyldimethylsilyl)-5-trifluormethyl-1H-indol-3-yl)methoxy)-methyl)-N,N-dimethylcyclohexanamin (ggf. eines von zwei möglichen Diastereomeren)
7 1-Phenyl-4-(((5-trifluormethyl-2-(triethylsilyl)-1H-indol-3-yl)methoxy)methyl)-N,N-dimethylcyclohexanamin (ggf. eines von zwei möglichen Diastereomeren)
8 1-Phenyl-4-(((5-trifluormethyl-1H-indol-3-yl)methoxy)methyl)-N, N-dimethyl-cyclohexanamin (ggf. eines von zwei möglichen Diastereomeren)
9 1-Phenyl-4-(((5-fluor-2-(triethylsilyl)-1H-indol-3-yl)methoxy)methyl)-N,N-dimethylcyclohexanamin (ggf. eines von zwei möglichen Diastereomeren)
10 1-Phenyl-4-(((5-fluor-1H-indol-3-yl)methoxy)methyl)-N,N-dimethyl-cyclohexanamin (ggf. eines von zwei möglichen Diastereomeren)
11 1-Phenyl-N,N-dimethyl-4-(((2-(triethylsilyl)-1H-pyrrolo[2,3-b]pyridin-3-yl)methoxy)-methyl)cyclohexanamin (ggf. eines von zwei möglichen Diastereomeren)
12 4-(((1H-Pyrrolo[2,3-b]pyridin-3-yl)methoxy)methyl)-1-phenyl-N,N-dimethylcyclohexanamin (ggf. eines von zwei möglichen Diastereomeren)
13 1-Phenyl-4-(((2-(tert-butyldimethylsilyl)-1H-pyrrolo[3,2-c]pyridin-3-yl)methoxy)methyl)-N,N-dimethylcyclohexanamin (ggf. eines von zwei möglichen Diastereomeren)
14 1-Phenyl-N,N-dimethyl-4-(((2-(triethylsilyl)-1H-pyrrolo[3,2-c]pyridin-3-yl)methoxy)-methyl)cyclohexanamin (ggf. eines von zwei möglichen Diastereomeren)
15 4-(((1H-Pyrrolo[3,2-c]pyridin-3-yl)methoxy)methyl)-1-phenyl-N,N-dimethylcyclohexanamin (ggf. eines von zwei möglichen Diastereomeren)
16 1-(3-(((4-(Dimethylamino)-4-phenylcyclohexyl)methoxy)methyl)-5-fluor-2-(triethylsilyl)-1H-indol-9-yl)ethanon
17 4-(((5-Fluor-1-(methylsulfonyl)-2-(triethylsilyl)-1H-indol-3-yl)methoxy)methyl)-N,N-dimethyl-1-phenylcyclohexanamin (ggf. eines von zwei möglichen Diastereomeren)
18 1-Phenyll-4-[2-(*tert*-butyldimethylsilanyl)-1*H*-indol-3-ylmethoxy-methyl]cyclohexyldimethylamin (ggf. eines von zwei möglichen Diastereomeren)
19 1-Phenyl-4-(((2-(tert-butyldimethylsilyl)-5-fluor-1H-indol-3-yl)methoxy)methyl)-N,N-dimethylcyclohexanamin (ggf. eines von zwei möglichen Diastereomeren)
20 1-Phenyl-4-(((2-(tert-butyldimethylsilyl)-1H-pyrrolo[2,3-b]pyridin-3-yl)methoxy)methyl)-N,N-dimethylcyclohexanamin (ggf. eines von zwei möglichen Diastereomeren)
22 {1-Benzyl-4-[2-(*tert*-butyldimethylsilanyl)-1*H*-indol-3-ylmethoxymethyl]cyclohexyl}-dimethylamin (ggf. unpolareres Diastereomer)
23 [1-Benzyl-4-(2-triethylsilanyl-1*H*-indol-3-ylmethoxymethyl)cyclohexyl] dimethylamin (ggf. unpolareres Diastereomer)
24 [1-Benzyl-4-(1*H*-indol-3-ylmethoxymethyl)cyclohexyl]dimethylamin (ggf. unpolareres Diastereomer)
25 1-Benzyl-4-(((5-fluor-2-(triethylsilyl)-1H-indol-3-yl)methoxy)methyl)-N,N-dimethylcyclohexanamin (ggf. unpolareres Diastereomer)
26 1-Benzyl-4-(((2-(tert-butyldimethylsilyl)-5-fluor-1H-indol-3-yl)methoxy)methyl)-N,N-dimethylcyclohexanamin (ggf. unpolareres Diastereomer)
27 1-Benzyl-4-(((5-fluor-1H-indol-3-yl)methoxy)methyl)-N,N-dimethylcyclohexanamin (ggf. unpolareres Diastereomer)
28 1-Benzyl-N,N-dimethyl-4-(((2-(triethylsilyl)-1H-pyrrolo[2,3-b]pyridin-3-yl)methoxy)-methyl)cyclohexanamin (ggf. unpolareres Diastereomer)
29 1-Benzyl-4-(((2-(tert-butyldimethylsilyl)-1H-pyrrolo[2,3-b]pyridin-3-yl)methoxy)methyl)-N,N-dimethylcyclohexanamin (ggf. unpolareres Diastereomer)
30 4-(((1H-Pyrrolo[2,3-b]pyridin-3-yl)methoxy)methyl)-1-benzyl-N,N-dimethylcyclohexanamin (ggf. unpolareres Diastereomer)
31 1-Benzyl-N,N-dimethyl-4-(((2-(triethylsilyl)-1H-pyrrolo[3,2-c]pyridin-3-yl)methoxy)-methyl)cyclohexanamin (ggf. unpolareres Diastereomer)
32 1-Benzyl-4-(((2-(tert-butyldimethylsilyl)-1H-pyrrolo[3,2-c]pyridin-3-yl)methoxy)methyl)-N,N-dimethylcyclohexanamin (ggf. unpolareres Diastereomer)
33 4-(((1H-Pyrrolo[3,2-c]pyridin-3-yl)methoxy)methyl)-1-benzyl-N,N-dimethylcyclohexan-amin (ggf. unpolareres Diastereomer)
34 1-Benzyl-N,N-dimethyl-4-(((2-(triethylsilyl)-1H-indol-3-yl)methoxy)methyl)cyclohexan-amin (ggf. polareres Diastereomer)
35 1-Benzyl-4-(((2-(tert-butyldimethylsilyl)-1H-indol-3-yl)methoxy)methyl)-N,N-dimethyl-cyclohexanamin (ggf. polareres Diastereomer)
36 4-(((1H-Indol-3-yl)methoxy)methyl)-1-benzyl-N,N-dimethylcyclohexanamin (ggf. polareres Diastereomer)
37 1-Benzyl-4-(((5-fluor-2-(triethylsilyl)-1H-indol-3-yl)methoxy)methyl)-N,N-dimethyl-cyctohexanamin (ggf. polareres Diastereomer)
38 1-Benzyl-4-(((2-(tert-butyldimethylsilyl)-5-fluor-1H-indol-3-yl)methoxy)methyl)-N,N-dimethylcyclohexanamin (ggf. polareres Diastereomer)
39 1-Benzyl-4-(((5-fluor-1H-indol-3-yl)methoxy)methyl)-N, N-dimethylcyclohexanamin (ggf. polareres Diastereomer)
40 1-Benzyl-N,N-dimethyl-4-(((2-(triethylsilyl)-1H-pyrrolo[2,3-b]pyridin-3-yl)methoxy)-methyl)cyclohexanamin hydrochlorid (ggf. polareres Diastereomer)
41 4-(((1H-Pyrrolo[2,3-b]pyridin-3-yl)methoxy)methyl)-1-benzyl-N, N-dimethylcyclohexanamin (ggf. polareres Diastereomer)
42 1-Benzyl-4-(((2-(tert-butyldimethylsilyl)-1H-pyrrolo[3,2-c]pyridin-3-yl)methoxy)methyl)-N,N-dimethylcyclohexanamin (ggf. polareres Diastereomer)
43 1-Butyl-N,N-dimethyl-4-(((2-(triethylsilyl)-1H-indol-3-yl)methoxy)methyl)cyclohexanamin (ggf. unpolareres Diastereomer)
44 4-(((1H-Indol-3-yl)methoxy)methyl)-1-butyl-N,N-dimethylcyclohexanamin (ggf. unpolareres Diastereomer)
45 1-Butyl-4-(((5-fluor-2-(triethylsilyl)-1H-indol-3-yl)methoxy)methyl)-N,N-dimethylcyclohexanamin (ggf. unpolareres Diastereomer)
46 1-Butyl-4-(((5-fluor-1H-indol-3-yl)methoxy)methyl)-N,N-dimethylcyclohexanamin (ggf. unpolareres Diastereomer)
47 1-Butyl-N,N-dimethyl-4-(((2-(triethylsilyl)-1H-pyrrolo[2,3-b]pyridin-3-yl)methoxy)-methyl)cyclohexanamin (ggf. unpolareres Diastereomer)
48 4-(((1H-Pyrrolo[2,3-b]pyridin-3-yl)methoxy)methyl)-1-butyl-N,N-dimethylcyclohexanamin (ggf. unpolareres Diastereomer);
49 1-Butyl-N,N-dimethyl-4-(((2-(triethylsilyl)-1H-pyrrolo[3,2-c]pyridin-3-yl)methoxy)-methyl)cyclohexanamin (ggf. unpolareres Diastereomer)
50 4-(((1H-Pyrrolo[3,2-c]pyridin-3-yl)methoxy)methyl)-1-butyl-N,N-dimethylcyclohexanamin (ggf. unpolareres Diastereomer)
51 1-Butyl-4-(((5-fluor-2-(triethylsilyl)-1H-indol-3-yl)methoxy)methyl)-N-methylcyclohexanamin (ggf. unpolareres Diastereomer)
52 1-Butyl-N-methyl-4-(((2-(triethylsilyl)-1H-indol-3-yl)methoxy)methyl)cyclohexanamin (ggf. unpolareres Diastereomer)
53 1-Butyl-N-methyl-4-(((2-(triethylsilyl)-5-(trifluormethyl)-1H-indol-3-yl)methoxy)-methyl)cyclohexanamin (ggf. unpolareres Diastereomer)
54 1-Butyl-N-methyl-4-(((5-(trifluormethyl)-1H-indol-3-yl)methoxy)methyl)cyclohexanamin (ggf. unpolareres Diastereomer)
55 4-(((5-Fluor-2-(triethylsilyl)-1H-indol-3-yl)methoxy)methyl)-N,N-dimethyl-1-(thiophen-2-yl)cyclohexanamin (ggf. eines von zwei möglichen Diastereomeren) "
56 4-(((5-Fluor-1H-indol-3-yl)methoxy)methyl)-N,N-dimethyt-1-(thiophen-2-yl)cyclohexanamin (ggf. eines von zwei möglichen Diastereomeren)
57 3-(((4-(Azetidin-1-yl)-4-phenylcyclohexyl)methoxy)methyl)-5-fluor-2-(triethylsilyl)-1 H-indol (ggf. eines von zwei möglichen Diastereomeren)
58 3-(((4-(Azetidin-1-yl)-4-phenylcyclohexyl)methoxy)methyl)-5-fluor-1H-indol (ggf. eines von zwei möglichen Diastereomeren)
60 4-(((5-Fluor-1-methyl-2-(triethylsilyl)-1H-indol-3-yl)methoxy)methyl)-N,N-dimethyl-1-phenylcyclohexanamin (ggf. eines von zwei möglichen Diastereomeren)
61 4-(((5-Fluor-1-methyl-1H-indol-3-yl)methoxy)methyl)-N,N-dimethyl-1-phenylcyclohexanamin (ggf. eines von zwei möglichen Diastereomeren)
62 4-(((5-Fluor-1-(methylsulfonyl)-1H-indol-3-yl)methoxy)methyl)-N,N-dimethyl-1-phenylcyclohexanamin (ggf. eines von zwei möglichen Diastereomeren)
63 1-(3-(((4-(Dimethylamino)-4-phenylcyclohexyl)methoxy)methyl)-5-fluor-1H-indol-1-yl)-ethanon (ggf. eines von zwei möglichen Diastereomeren)
64 4-(((2-tert-Butyl-5-fluor-1H-indol-3-yl)methoxy)methyl)-N,N-dimethyl-1-phenylcyclohexanamin (ggf. eines von zwei möglichen Diastereomeren)
65 3-(((4-(Azetidin-1-yl)-4-phenylcyclohexyl)methoxy)methyl)-2-(triethylsilyl)-1H-pyrrolo-[2,3-b]pyridin (ggf. eines von zwei möglichen Diastereomeren)
66 3-(((4-(Azetidin-1-yl)-4-phenylcyclohexyl)methoxy)methyl)-1H-pyrrolo[2,3-b]pyridin (ggf. eines von zwei möglichen Diastereomeren)
67 4-(((2-*tert*-Butyl-1H-pyrrolo[2,3-b]pyridin-3-yl)methoxy)methyl)-N,N-dimethyl-1-phenylcyclohexanamin (ggf. eines von zwei möglichen Diastereomeren)
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Die erfindungsgemäßen Substanzen eignen sich besonders zur Behandlung von neuropathischem Schmerz. Ein weiterer Gegenstand der Erfindung sind daher Arzneimittel enthaltend wenigstens ein erfindungsgemäßes substituiertes 4-Aminocyclohexan-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen substituierten 4-Aminocyclohexan-Derivat gegebenenfalls geeignete Zusatz- und/ oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte 4-Aminocyclohexan-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten 4-Aminocyclohexan-Derivate verzögert freisetzen. Die erfindungsgemäßen substituierten 4-Aminocyclohexan-Derivate können auch in parenteralen Langzeitdepotformen wie z. B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,001 bis 0,5 mg/kg wenigstens eines erfindungsgemäßen substituierten 4-Aminocyclohexan-Derivats appliziert.

In einer bevorzugten Form des Arzneimittels liegt ein enthaltenes erfindungsgemäßes substituiertes 4-Aminocyclohexan-Derivat als reines Diastereomer und/oder Enantiomer, als Razemat oder als nicht-äquimolare oder äquimolare Mischung der Diastereomere und/oder Enantiomere vor.

Die Verbindungen eigenen sich besonders zur Behandlung von neuropathischem Schmerz, insbesondere diabetischem Polyneuropathieschmerz oder Schmerzen bei postzosterischer Neuralgie. Entsprechend können erfindungsgemäße substituierte 4-Aminocyclohexan-Derivate zur Herstellung eines Arzneimittels zur Behandlung von Schmerz, besonders aber neuropathischem oder chronischem Schmerz, insbesondere diabetischem Polyneuropathieschmerz oder Schmerzen bei postzosterischer Neuralgie, verwendet werden.

Ein weiterer Gegenstand der Erfindung ist daher die Verwendung eines erfindungsgemäßen substituierten 4-Aminocyclohexan-Derivats zur Herstellung eines Arzneimittels zur Behandlung von Schmerz, besonders neuropathischem oder chronischem Schmerz, insbesondere diabetischem Polyneuropathieschmerz oder Schmerzen bei postzosterischer Neuralgie,

Ein weiterer Gegenstand der Erfindung ist die Verwendung eines erfindungsgemäßen substituierten 4-Aminocyclohexan-Derivats zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/ oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn ein verwendetes substituiertes 4-Aminocyclohexan-Derivat als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist Verbindungen der Formel (I) zur Verwendung in einem Verfahren zur Behandlung, insbesondere in einer der vorgenannten Indikationen, eines nichthumanen Säugetieres oder Menschen, das oder der eine Behandlung von Schmerzen, insbesondere neuropathischer Schmerzen, benötigt, durch Verabreichung einer therapeutisch wiksamen Dosis eines erfindungsgemäßen substituierten 4-Aminocyclohexan-Derivats, oder eines erfindungsgemäßen Arzneimittels.

Ein weiterer Gegenstand der vorliegenden Erfindung ist ein Verfahren zur Herstellung der erfindungsgemäßen substituierten 4-Aminocyclohexan-Verbindungen. Die in den beschriebenen Umsetzungen zum Einsatz kommenden Chemikalien und Reaktionskomponenten sind käuflich erhältlich oder können jeweils nach dem Fachmann bekannten Methoden hergestellt werden.

### Allgemeines Verfahren zur Darstellung der Verbindungen der allgemeinen Formel I

In den Verfahren I und II wird das ketalgeschützte 4-Hydroxymethyl-cyclohexanon **2** verwendet, das aus dem kommerziell erhältlichen 4-Cyclohexanoncarbonsäureethylester **1** durch Umsetzung mit Ethylenglykol in Anwesenheit von Brönsted-Säuren, beispielsweise Camphersulfonsäure, p-Toluolsulfonsäure oder festen Säuren wie sauren lonentauscherharzen in höhersiedenden, organischen Lösungsmitteln, beispielsweise in Benzol, Toluol oder Xylolen bei Temperaturen zwischen 20 und 130 °C und nachfolgender Umsetzung mit einem Reduktionsmittel, beispielsweise einem Hydrid wie Natrium- oder Lithiumborhydrid, Natriumcyanoborhydrid, Natriumtriacetoxyborhydrid, Diisobutylaluminiumhydrid, Lithium-tri-(sec.-butyl)borhydrid (L-Selectride^{®}) oder Lithiumaluminiumhydrid, gegebenenfalls in Anwesenheit von Lewis-Säuren, beispielsweise ZnCl₂, Ni(OAc)₂ oder CoCl₂ in organischen Lösungsmittel, beispielsweise Ether, Diisopropylether, Tetrahydrofuran oder Methyl-t-Butylether bei Temperaturen zwischen 0 und 100 °C erhalten werden kann.

### Verfahren I:

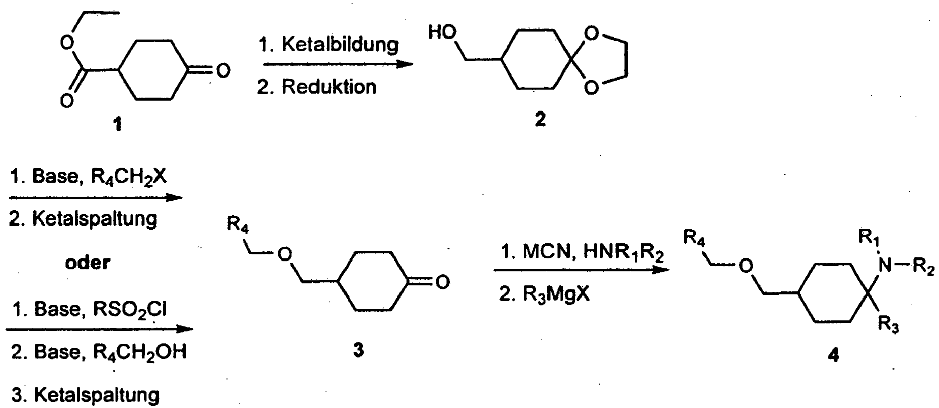

Die Umsetzung des ketalgeschützten 4-Hydroxymethyl-cyclohexanon **2** zu substituierten Cyclohexanonen der allgemeinen Formel **3** kann in organischen Lösungsmitteln, beispielsweise Ether, Tetrahydrofuran, Dimethylformamid, Methanol, Ethanol oder Dichlormethan in Gegenwart von einer anorganischen oder organischen Base, beispielsweise Kalium-tert-butanolat, Natriummethanolat oder -ethanolat, Lithium- oder Natriumhydrid, Kalium- oder Natriumhydroxid, Protonenschwamm, Butyllithium, oder anderen basischen Organometallverbindungen wie Grignardreagenzien, beispielsweise Ethylmagnesiumchlorid oder -bromid, gegebenenfalls in Anwesenheit von Phasentransferkatalysatoren, beispielsweise Tetra-*n-*butylammoniumchlorid, Tetra-*n*-butylammoniumhydroxid oder Tetra-*n*-butylammoniumiodid und mit einem Alkylierungsmittel

R⁴CH₂X mit X = Cl, Br bei Temperaturen zwischen -10 und 120 °C erfolgen. Abschließend kann in wässrigem Medium oder organischen Lösungsmitteln, beispielsweise Tetrahydrofuran, Aceton oder Butanon, oder Mischungen dieser beiden Medien in Gegenwart einer anorganischen Säure, beispielsweise HCl, HBr, H₂SO₄, HClO₄ oder H₃PO₄ oder einer organischen Säure, beispielweise Trifluormethansäure, Methansulfonsäure, Trifluoressigsäure, Camphersulfonsäure, *p*-Toluolsulfonsäure oder Pyridinium-Tosylat, gegebenenfalls in Anwesenheit von PdCl₂, Pd(OAc)₂, PdCl₂(MeCN)₂ oder PdCl₂(PPh₃)₂, bei Temperaturen zwischen 20 und 80 °C eine Ketalspaltung erfolgen.

Alternativ dazu kann die Umsetzung des ketalgeschützten 4-Hydroxymethyl-cyclohexanon 2 zu substituierten Cyclohexanonen der allgemeinen Formel 3 in organischen Lösungsmitteln, beispielsweise Ether, Tetrahydrofuran, Dimethylformamid, Methanol, Ethanol oder Dichlormethan in Gegenwart einer organischen Base, beispielsweise Hünig's Base, *N*-Methyl-Piperidin, Pyridin, Triethylamin oder *N*-Methyl-morpholin und einem Sulfonsäurechlorid, beispielsweise p-Toluolsulfonsäurechlorid, Methansulfonsäurechlorid oder Trifluormethansulfonsäurechlorid, bei Temperaturen zwischen 20 und 80 °C erfolgen. Die erhaltene Zwischenstufe kann mit einer Mischung eines Alkohols

R⁴CH₂OH

in organischen Lösungsmitteln, beispielsweise Ether, Tetrahydrofuran, Dimethylformamid, Methanol, Ethanol oder Dichlormethan in Gegenwart von einer anorganischen oder organischen Base, beispielsweise Kalium-tert-butanolat, Natriummethanolat oder -ethanolat, Lithium- oder Natriumhydrid, Kalium- oder Natriumhydroxid, Protonenschwamm, Butyllithium, oder anderen basischen Organometallverbindungen wie Grignardreagenzien, beispielsweise Ethylmagnesiumchlorid oder -bromid, gegebenenfalls in Anwesenheit von Phasentransferkatalysatoren, beispielsweise Tetra-*n*-butylammoniumchlorid, Tetra-n-butylammoniumhydroxid oder Tetra-*n*-butylammoniumiodid bei Temperaturen zwischen -10 und 120 °C umgesetzt werden. Abschließend kann in wässrigem Medium oder organischen Lösungsmitteln, beispielsweise Tetrahydrofuran, Aceton oder Butanon, oder Mischungen dieser beiden Medien in Gegenwart einer anorganischen Säure, beispielsweise HCl, HBr, H₂SO₄, HClO₄ oder H₃PO₄ oder einer organischen Säure, beispielweise Trifluormethansäure, Methansulfonsäure, Trifluoressigsäure, Camphersulfonsäure, *p*-Toluolsulfonsäure oder Pyridinium-Tosylat, gegebenenfalls in Anwesenheit von PdCl₂, Pd(OAc)₂, PdCl_{2z}(MeCN)₂ oder PdCl₂(PPh₃)₂, bei Temperaturen zwischen 20 und 80 °C eine Ketalspaltung erfolgen.

Die Umsetzung der substituierten Cyclohexanone der allgemeinen Formel 3 zu den erfindungsgemäßen 1-substituierten-4-Hydroxymethyl-Cyclohexylamin-Derivaten 4 kann in wässrigem Medium oder organischen Lösungsmitteln, beispielsweise Methanol, Ethanol, *n-*Propanol, Tetrahydrofuran, Acetonitril oder Dichlormethan, oder Mischungen dieser beiden Medien in Gegenwart einer anorganischen Säure, beispielsweise HCl, HBr, H₂SO₄, HClO₄ oder H₃PO4, einer Cyanidquelle, beispielsweise Natrium- oder Kaliumcyanid, Zinkcyanid, Trimethylsilylcyanid, Acetoncyanhydrin, und einem Amin

HNR¹R²

oder dessen Hydrochlorid

HCl·HNR¹R²

bei Temperaturen zwischen 20 und 60 °C erfolgen. Abschließend kann die erhaltene Zwischenstufe in organischen Lösungsmitteln, beispielsweise Hexan, Pentan, Toluol, Ether, Diisopropylether, Tetrahydrofuran oder Methyl-t-Butylether, mit einem Grignardreagenz

MgXR³ mit X = Cl, Br, I

bei Temperaturen zwischen -10 °C und 40 °C umgesetzt werden.

### Verfahren II:

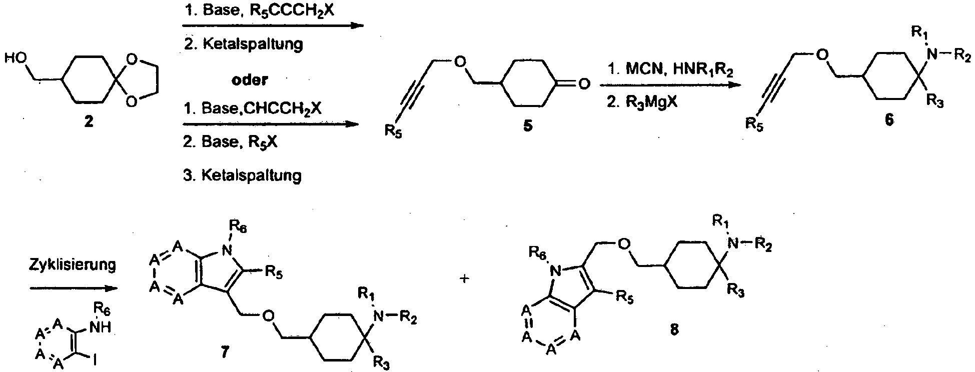

Die Umsetzung des ketalgeschützten 4-Hydroxymethyl-cyclohexanon 2 zu substituierten Cyclohexanonen der allgemeinen Formel 5 kann in organischen Lösungsmitteln, beispielsweise Ether, Tetrahydrofuran, Dimethylformamid, Methanol, Ethanol oder Dichlormethan in Gegenwart von einer anorganischen oder organischen Base, beispielsweise Kalium-tert-butanolat, Natriummethanolat oder-ethanolat, Lithium- oder Natriumhydrid, Kalium- oder Natriumhydroxid, Protonenschwamm, Butyllithium, oder anderen basischen Organometallverbindungen wie Grignardreagenzien, beispielsweise Ethylmagnesiumchlorid oder -bromid, gegebenenfalls in Anwesenheit von Phasentransferkatatysatoren, beispielsweise Tetra-n-butylammoniumchlorid, Tetra-n-butylammoniumhydroxid oder Tetra-n-butylammoniumiodid und mit einem Alkylierungsmittel

R⁵CCCH₂X mit X = Cl, Br

bei Temperaturen zwischen -10 und 120 °C erfolgen. Abschließend kann in wässrigem Medium oder organischen Lösungsmitteln, beispielsweise Tetrahydrofuran, Aceton oder Butanon, oder Mischungen dieser beiden Medien in Gegenwart einer anorganischen Säure, beispielsweise HCl, HBr, H₂SO₄, HClO₄ oder H₃PO₄ oder einer organischen Säure, beispielsweise Trifluormethansäure, Methansulfonsäure, Trifluoressigsäure, Camphersulfonsäure, *p-*Toluolsulfonsäure oder Pyridinium-Tosylat, gegebenenfalls in Anwesenheit von PdCl₂, Pd(OAc)₂, PdCl₂(MeCN)₂ oder PdCl₂(PPh₃)₂, bei Temperaturen zwischen 20 und 80 °C eine Ketalspaltung erfolgen.

Alternativ dazu kann die Umsetzung des ketalgeschützten 4-Hydroxymethyl-cyclohexanon 2 zu substituierten Cyclohexanonen der allgemeinen Formel 5 in organischen Lösungsmitteln, beispielsweise Ether, Tetrahydrofuran, Dimethylformamid, Methanol, Ethanol oder Dichlormethan in Gegenwart von einer anorganischen oder organischen Base, beispielsweise Kalium-tert-butanolat, Natriummethanolat oder-ethanolat, Lithium- oder Natriumhydrid, Kalium- oder Natriumhydroxid, Protonenschwamm, Butyllithium, oder anderen basischen Organometallverbindungen wie Grigṅardreagenzien, beispielsweise Ethylmagnesiumchlorid oder -bromid, gegebenenfalls in Anwesenheit von Phasentransferkatalysatoren, beispielsweise Tetra-*n*-butylammoniumchlorid, Tetra-*n*-butylammoniumhydroxid oder Tetra-*n*-butylammoniumiodid und mit einem Alkylierungsmittel

HCCCH₂X mit X = Cl, Br, OSO₂CH₃, OSO₂-*p*-Toluol

bei Temperaturen zwischen -10 und 120 °C erfolgen. Die erhaltene Zwischenstufe kann in organischen Lösungsmitteln, beispielsweise Hexan, Pentan, Ether, Diisopropylether, Tetrahydrofuran oder Methyl-*t*-Butylether, in Gegenwart von einer Base, beispielsweise Natriumamid, Lithium-diisopropylamid Butyllithium, oder anderen basischen Organometallverbindungen wie Grignardreagenzien, beispielsweise Ethylmagnesiumchlorid oder -bromid, mit Elektrophilen

R⁵X mit X = Cl, Br, I 1

bei Temperaturen zwischen -80 und 20 °C umgesetzt werden.

Abschließend kann in wässrigem Medium oder organischen Lösungsmitteln, beispielsweise Tetrahydrofuran, Aceton oder Butanon, oder Mischungen dieser beiden Medien in Gegenwart einer anorganischen Säure, beispielsweise HCl, HBr, H₂SO₄, HClO₄ oder H₃PO₄ oder einer organischen Säure, beispielweise Trifluormethansäure, Methansulfonsäure, Trifluoressigsäure, Camphersulfonsäure, *p*-Toluolsulfonsäure oder Pyridinium-Tosylat, gegebenenfalls in Anwesenheit von PdCl₂, Pd(OAc)₂, PdCl₂(MeCN)₂ oder PdCl₂(PPh₃)₂, bei Temperaturen zwischen 20 und 80 °C eine Ketalspaltung erfolgen.

Die Umsetzung der substituierten Cyclohexanone der allgemeinen Formel 5 zu den 1-substituierten-4-Alkinyloxymethyl-Cyclohexylamin-Derivaten der allgemeinen Formel 6 kann in wässrigem Medium oder organischen Lösungsmitteln, beispielsweise Methanol, Ethanol, *n*-Propanol, Tetrahydrofuran, Acetonitril oder Dichlormethan, oder Mischungen dieser beiden Medien in Gegenwart einer anorganischen Säure, beispielsweise HCl, HBr, H₂SO₄, HClO₄ oder H₃PO₄, einer Cyanidquelle, beispielsweise Natrium- oder Kaliumcyanid, Zinkcyanid, Trimethylsilylcyanid, Acetoncyanhydrin, und einem Amin

HNR¹R²

oder dessen Hydrochlorid

HCl·HNR¹R²

bei Temperaturen zwischen 20 und 60 °C erfolgen. Abschließend kann die erhaltene Zwischenstufe in organischen Lösungsmitteln, beispielsweise Hexan, Pentan, Toluol, Ether, Diisopropylether, Tetrahydrofuran oder Methyl-*t*-Butylether, mit einem Grignardreagenz

MgXR³ mit X = Cl, Br, I

bei Temperaturen zwischen -10 °C und 40 °C umgesetzt werden.

Die Umsetzung der 1-substituierten-4-Alkinyloxymethyl-Cyclohexylamin-Derivaten der allgemeinen Formel **6** zu den erfindungsgemäßen 1-substituierten-4-hydroxymethyl-Cyclohexylamin-Derivaten der allgemeinen Formeln **7** und 8 kann in organischen Lösungsmitteln, beispielsweise Tetrahydrofuran, Dimethylformamid, Benzol, Toluol, Xylolen, Dimethoxyethan oder Diethylenglykol-dimethylether, in Gegenwart einer anorganischen Base, beispielsweise Natrium-, Kalium- oder Cäsiumcarbonat oder Kaliumphosphat, in Anwesenheit von PdCl₂, Pd(OAc)₂, PdCl₂(MeCN)₂, PdCl₂(PPh₃)₂ oder [1,3-Bis-(2,6-diisopropylphenyl)imidazol-2-yliden]-(3-chlorpyridyl)palladium(II)-chlorid (PEPPSI^{®}), gegebenenfalls in Anwesenheit von zusätzlichen Liganden, beispielsweise Triphenyl-, Tri-o-tolyl-, Tricyclohexyl oder Tri-*t*-butylphosphin, gegebenenfalls in Anwesenheit von Phasentransferkatalysatoren, beispielsweise Tetra-*n*-butylammoniumchlorid, Tetra-*n-*butylammoniumhydroxid oder Tetra-*n*-butylammoniumiodid, bei Temperaturen zwischen 60 °C und 180°C, auch mikrowellenunterstützt, umgesetzt werden. Die Produkte **7** und **8** können einzeln oder als Gemische erhalten und ggf. durch Säulenchromatographie gereinigt werden.

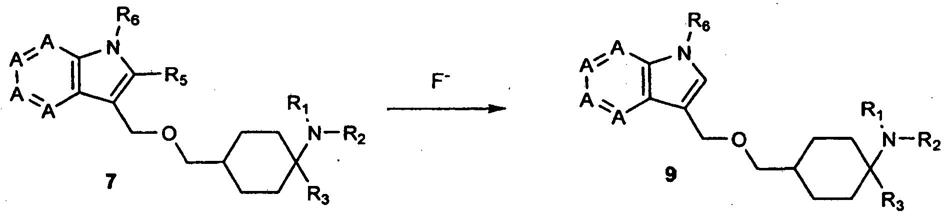

Die Umsetzung der 1-substituierten-4-hydroxymethyl-Cyclohexylamin-Derivaten der allgemeinen Formel **7** zu den erfindungsgemäßen 1-substituierten-4-hydroxymethyl-Cyclohexylamin-Derivaten allgemeinen Formel **9** kann in organischen Lösungsmitteln, beispielsweise Hexan, Pentan, Ether, Diisopropylether, Tetrahydrofuran, Methyl-*t*-Butylether, Acetonitril, Dimethylformamid oder Dichlormethan in Gegenwart einer Fluoridquelle beispielsweise Tetra-*n*-butylammoniumfluorid, Tetramethylammoniumfluorid, Benzyltrimethylammoniumfluorid oder HF-Pyridin, gegebenenfalls in Anwesenheit von wasserentziehenden Reagenzien, beispielsweise Molekularsieb 4 A, bei Temperaturen zwischen 20 °C und 80 °C umgesetzt werden.

### Verfahren III:

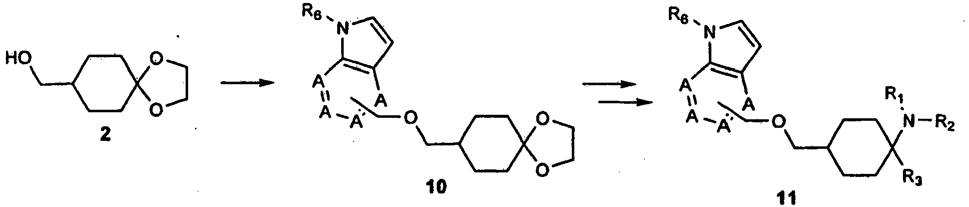

Die Umsetzung des ketalgeschützten 4-Hydroxymethyl-cyclohexanon **2** zu substituierten Cyclohexanonen allgemeinen Formel **10** kann in organischen Lösungsmitteln, beispielsweise Ether, Tetrahydrofuran, Dimethylformamid, Methanol, Ethanol oder Dichlormethan in Gegenwart von einer anorganischen oder organischen Base, beispielsweise Kalium-tert-butanolat, Natriummethanolat oder -ethanolat, Lithium- oder Natriumhydrid, Kalium- oder Natriumhydroxid, Protonenschwamm, Butyllithium, oder anderen basischen Organometallverbindungen wie Grignardreagenzien, beispielsweise Ethylmagnesiumchlorid oder -bromid, gegebenenfalls in Anwesenheit von Phasentransferkatalysatoren, beispielsweise Tetra-n-butylammoniumchlorid, Tetra-*n*-butylammoniumhydroxid oder Tetra-n-butylammoniumiodid und mit einem Alkylierungsmittel

R⁴CH₂X mit X = Cl, Br

bei Temperaturen zwischen -10 und 120 °C erfolgen.

Die weiteren Schritte zu den erfindungsgemäßen 1-substituierten-4-hydroxymethyl-Cyclo-hexylamin-Derivaten der allgemeinen Formel **11** können dann in Analogie zu den Verfahren I und II erfolgen.

Die nachfolgenden Beispiele dienen der Erläuterung der Erfindung, sind jedoch nicht einschränkend auszulegen.

### Beispiele

### 1.) Synthese der Bausteine

### Nitrilbaustein 1: 4-((3-(tert-Butyldimethylsilyl)prop-2-ynyloxy)methyl)-1-(dimethylamino)cyclohexancarbonitril (Nit-01)

### Stufe 1: 1,4-Dioxaspiro[4.5]decan-8-carbonsäureethylester

Eine Lösung von 4-Oxocyclohexancarbonsäureethylester (27.6 g, 162 mmol), Ethylenglykol (35.3 g, 31.7 ml, 569 mmol) und *p*-Toluolsulfonsäure (360 mg, 1.89 mmol) in Toluol (80 ml) wurde 20 h bei Raumtemperatur gerührt. Anschließend wurde die Reaktionslösung in Diethylether (150 ml) gegossen und mit Wasser, 5 % Natriumhydrogencarbonat-Lösung und gesättigter Natriumchloridlösung (je 150 ml) gewaschen. Die organische Phase wurde mit Natriumsulfat getrocknet und i. Vak. eingeengt.

### Ausbeute: 33 g (95 %), farbloses Öl

*¹H-NMR (DMSO-d₆):* 1.17 (t, 3H,J = 7.3 Hz); 1.41-1.69 (m, 6H); 1.77-1.82 (m, 1 H); 2.28-2.40 (m, 2H); 3.84 (s, 4H); 4.04 (dd, 2H, J = 6.8, 14.6 Hz).

### Stufe 2: (1,4-Dioxaspiro[4.5]dec-8-yl)methanol

Eine Aufschlämmung von Lithiumaluminiumhydrid (3.50 g, 93.3 mmol) in wasserfreiem Tetrahydrofuran (100 ml) wurde unter Argon bei 65 °C tropfenweise mit einer Lösung von 1,4-Dioxaspiro[4.5]decan-8-carbonsäureethylester (9.72g, 45.4 mmol) in wasserfreiem Tetrahydrofuran (50 ml) versetzt und anschließend 3 h unter Rückfluss gerührt. Nach Zugabe von Wasser (6 ml) und 4 *N* Natronlauge (1.5 ml) wurde der Niederschlag abfiltriert und mit Tetrahydrofuran gewaschen. Das Filtrat wurde mit Natriumsulfat getrocknet und i. Vak. eingeengt.

### Ausbeute: 6.53 g (84 %), farbloses Öl

*¹H-NMR (DMSO-d₆):* 1.03-1.18 (m, 2H); 1.30-1.18 (m, 3H); 1.59-1.70 (m, 4H); 3.21 (t, 2H, J = 6.0 Hz); 3.83 (s, 4H); 4.40 (t, 1H, J = 5.3 Hz).

¹³C-NMR (DMSO-d₆): 26.4; 33.8; 38.7; 63.4; 63.5; 66.7; 108.3.

### Stufe 3: 8-((Prop-2-ynyloxy)methyl)-1,4-dioxaspiro[4.5]decan

### Zu einer auf 0 °C gekühlten Lösung von (1,4-Dioxaspiro[4.5]dec-8-yl)methanol (5.24 g,

30.4 mmol) in wasserfreiem Tetrahydrofuran (150 ml) wurde eine 60 % Dispersion von Natriumhydrid (1.40 g, 36.7 mmol) in Mineralöl gegeben und die Mischung 90 min unter Rückfluss gekocht. Anschließend wurde die Reaktionslösung auf 0 °C gekühlt und nacheinander mit Tetra-n-butylammoniumiodid (241 mg, 0.63 mmol) und einer 80 % Lösung von Propargylbromid in Toluol (10.8 g, 8.12 ml, 91.2 mmol) versetzt und bei Raumtemperatur über Nacht gerührt. Das Reaktionsgemisch wurde danach i. Vak. eingeengt, der Rückstand mit Wasser (100 ml) versetzt und mit Dichlormethan (3 × 80 ml) extrahiert. Die vereinigten organischen Phasen wurden mit Natriumsulfat getrocknet und i. Vak. eingeengt. Das Rohprodukt (6.75 g) wurde durch Flashchromatographie (100 g, 20 × 4.0 cm) mit Ethylacetat / Cyclohexan (1:9) gereinigt.

### Ausbeute: 4.31 g (67 %), Öl ¹H-NMR (DMSO-d₆): 1.10-1.24 (m, 2H); 1.42 (dt, 2H, J = 12.6, 3.7 Hz); 1.49-1.60 (m, 1H); 1.65 (d, 4H, J = 10.1 Hz); 3.26 (d, 2H, J = 6.3 Hz); 3.39 (t, 1 H, J = 2.3 Hz); 3.83 (s, 4H); 4.09 (d, 2H, J = 2.4 Hz).

### Stufe 4: (3-(1,4-Dioxaspiro[4.5]decan-8-ylmethoxy)prop-1-ynyl)(tert-butyl)dimethylsilan

Zu einer Lösung von 8-((Prop-2-ynyloxy)methyl)-1,4-dioxaspiro[4.5]decan (4.30 g, 20.4 mmol) in wasserfreiem Tetrahydrofuran (150 ml) wurde bei -75 °C unter Argon eine 2.5 *M* Lösung von *n*-Butyllithium in *n*-Hexan (8.5 ml, 21.2 mmol) getropft. Das Reaktionsgemisch wurde auf 0 °C erwärmt und 1 h bei dieser Temperatur gerührt. Nach erneutem Abkühlen auf -75 °C wurde eine Lösung von *tert*-Butyldimethylchlorsilan (3.16 g, 21 mmol) in wasserfreiem Tetrahydrofuran (50 ml) zugetropft. Das Reaktionsgemisch wurde anschließend auf Raumtemperatur erwärmt und über Nacht gerührt. Nach Zugabe von gesättigter Ammoniumchloridlösung (35 ml) wurde die Suspension mit Diethylether (3 × 100 ml) extrahiert, die vereinigten organischen Phasen wurden mit gesättigter Natriumchloridlösung (50 ml) gewaschen, mit Natriumsulfat getrocknet und i. Vak. eingeengt.

### Ausbeute: 6.08 g (92 %), bräunliches Öl

*¹H-NMR (DMSO-d₆):* 0.09 (s, 6H); 0.91 (s, 9H); 1.10-1.22 (m, 2H); 1.35-1.47 (m, 2H); 1.50-1.60 (m, 1H); 1.64 (d, 4H, J = 10.6 Hz); 3.28 (d, 2H, J = 6.3 Hz); 3.83 (s, 4H); 4.13 (s, 2H).

### Stufe 5: 4-((3-(tert-Butyldimethylsilyl)prop-2-ynyloxy)methyl)cyclohexanon

Eine Lösung von (3-(1,4-Dioxaspiro[4.5]decan-8-ylmethoxy)prop-1-ynyl)(tert-butyl)dimethylsilan (1.55 g, 4.77 mmol) in Aceton (80 ml) wurde mit 1 *N* Salzsäure (15 ml) versetzt und 3 h bei Raumtemperatur gerührt. Das Reaktionsgemisch wurde anschließend mit 1 *N* Natronlauge (16 ml) versetzt und mit Dichlormethan (3 × 50 ml) extrahiert. Die vereinigten organischen Phasen wurden mit Natriumsulfat getrocknet und i. Vak. eingeengt. Das Rohprodukt (1.30 g) wurde durch Flashchromatographie (100 g, 20 × 4.0 cm) mit Ethylacetat / Cyclohexan (1:9) gereinigt.

### Ausbeute: 1.01 g (75 %), farbloses Öl

*¹H-NMR (DMSO-d₆):* 0.09 (s, 6H); 0.92 (s, 9H); 1.66-1.44 (m, 2H); 1.90-2.10 (m, 3H); 2.14-2.23 (m, 2H); 2.37 (dt, 2H, J = 13.9, 5.9 Hz); 3.37 (d, 2H, J = 6.3 Hz); 4.17 (s, 2H). *¹³C-NMR (DMSO-d₆):* -4.8; 16.0; 25.8; 28.8; 35.1; 39.6; 58.2; 73.0; 88.4; 103.5; 210.6.

### Stufe 6: 4-((3-(tert-Butyldimethylsilyl)prop-2-ynyloxy)methyl)-1-(dimethylamino)cyclohexan-carbonitril

Zu einer auf 0 °C gekühlten Lösung von 4 N Salzsäure (4.9 ml) und Methanol (2.9 ml) wurde 40 % wässrige Dimethylaminlösung (11.7 ml, 88.9 mmol) und anschließend eine Lösung von 4-((3-(*tert*-Butyldimethylsilyl)prop-2-ynyloxy)methyl)cyclohexanon (5.19 g, 18.5 mmol) in Methanol (20 ml) gegeben. Dieses Gemisch wurde mit Kaliumcyanid (2.90 g, 44.4 mmol) versetzt und 5 d bei Raumtemperatur gerührt. Nach Zugabe von Wasser (30 ml) wurde mit Diethylether (3 × 50 ml) extrahiert. Die vereinigten organischen Phasen wurden mit Natriumsulfat getrocknet und i. Vak. eingeengt.

### Ausbeute: 5.82 g (94 %), bräunliches Öl

*¹H-NMR (DMSO-d₆):* 0.088 und 0.09 (2 s, 6H); 0.09 und 0.92 (2 s, 9H); 1.05-1.21 (m, 2H); 1.26-1.57 (m, 3H); 1.63-1.83 (m, 2H); 2.20 (s, 2H); 2.24 (s, 6H); 3.27-3.32 (m, 2H); 4.13 und 4.15 (2 s, 2H).

Das Isomerenverhältnis beträgt ca. 2:3.

### Nitrilbaustein 2: 1-(Dimethylamino)-4-((3-(triethylsilyl)prop-2-ynyloxy)methyl)cyclohexancarbonitril (Nit-02)

### Stufe 1: (3-(1,4-Dioxaspiro[4.5]decan-8-ylmethoxy)prop-1-ynyl)triethylsilan

Zu einer Lösung von 8-((Prop-2-ynyloxy)methyl)-1,4-dioxaspiro[4.5]decan (Stufe 3, Nitrilbaustein 1) (6.1 g, 29.0 mmol) in wasserfreiem Tetrahydrofuran (150 ml) wurde bei -75 °C tropfenweise eine 2.5 *M* Lösung von *n*-Butyllithium in *n*-Hexan zugesetzt. Das Gemisch wurde eine Stunde bei 0 °C gerührt. Anschließend wurde die Lösung auf -75 °C abgekühlt und tropfenweise mit einer Lösung von Chlortriethylsilan (4.5 g, 29.8 mmol) in wasserfreiem Tetrahydrofuran (50 ml) versetzt. Das Gemisch wurde über Nacht bei Räumtemperatur gerührt, dann tropfenweise mit gesättigter Ammoniumchloridlösung (50 ml) versetzt und mit Diethylether (3 × 70 ml) extrahiert. Die vereinigten organischen Phasen wurden mit Natriumsulfat getrocknet und i. Vak. eingeengt.

### Ausbeute: 8.92 g (95 %), braunes Öl

*¹H-NMR (DMSO-d₆):* 0.57 (q, 6 H, J = 7.9 , 8.1 Hz); 0.95 (t, 9H, J = 7.8 Hz); 1.10-1.24 (m, 2H); 1.42 (dd, 2H, J = 9.3, 12.5 Hz); 1.52-1.60 (m, 1H); 1.64 (d, 4H, J = 10.6 Hz); 3.29 (d, 2H, J = 6.3 Hz); 3.83 (s, 4H); 4.13 (s, 2H).

### Stufe 2: 4-((3-(Triethylsilyl)prop-2-ynyloxy)methyl)cyclohexanon

Eine Lösung von (3-(1,4-Dioxaspiro[4.5]decan-8-ylmethoxy)prop-1-ynyl)triethylsilan (8.40 g, 25.9 mmol) in Aceton (160 ml) wurde mit einer 1 *M* Salzsäurelösung (70 ml) versetzt und 6 h bei Raumtemperatur gerührt. Das Reaktionsgemisch wurde anschließend mit Dichlormethan (3 × 25 ml) extrahiert. Die vereinigten organischen Phasen wurden mit Natriumsulfat getrocknet und i. Vak. eingeengt. Das Rohprodukt (7.24 g) wurde durch Flashchromatographie (400 g, 7.6 x 20 cm) mit Cyclohexan / Ethylacetat (8.5:1.5) gereinigt.

### Ausbeute: 5.10 g (66 %), gelbliches Öl

*¹H-NMR (DMSO-d₆):* 0.58 (q, 6H, J = 7.9, 8.1 Hz); 0.96 (t, 9H, J = 7.8 Hz); 1.36 (dq, 2H, J = ca. 4, 13.0 Hz); 1.92-2.10 (m, 3H); 2.19 (br d, 2H, J= 14.4 Hz); 2.37 (dt, 2H, J = 5.8, 13.5 Hz); 3.38 (d, 2H, J = 6.3 Hz); 4.17 (s, 2H).

### Stufe 3 (Methode 1): 1-(Dimethylamino)-4-((3-(triethylsilyl)prop-2-ynyloxy)methyl)cyclohexancarbonitril (Nit-02)

Zu einer auf 0 °C gekühlten Lösung von 4 N Salzsäure (10.8 ml) und Methanol (12 ml) wurde 40 % wässrige Dimethylaminlösung (26.4 ml, 192 mmol) und anschließend 4-((3-(Triethylsilyl)prop-2-ynyloxy)methyl)cyclohexanon (12.1 g, 43.1 mmol) in Methanol (50 ml) gegeben. Zu diesem Gemisch wurde Kaliumcyanid (6.81 g, 102 mmol) gegeben und 2 h bei Raumtemperatur gerührt. Nach Zugabe von Wasser (200 ml) wurde mit Diethylether (3 × 60 ml) extrahiert. Die vereinigten organischen Phasen wurden mit Natriumsulfat getrocknet und i. Vak. eingeengt.

### Ausbeute: 12.7 g (88 %), gelbliches Öl

*¹H-NMR (DMSO-d₆):* 0.57 und 0.78 (2 q, 6H, J = 7.8 Hz); 0.95 und 0.96 (2 t, 9H, J = 7.8 Hz); 1.04-1.50 (m, 5H); 1.65-1.85 (m, 3H); 2.07 (br d, 1H, J = 13.6 Hz); 2.20 und 2.24 (2 s, 6H); 3.29 (d, 1 H, J = 6.4 Hz); 3.32 (d, 1 H, J = 6.2 Hz); 4.13 und 4.15 (2 s, 2H).

### Stufe 3 (Methode 2): 1-(Dimethylamino)-4-((3-(triethylsilyl)prop-2-ynyloxy)methyl)cyclohexancarbonitril (Nit-02)

Eine auf 0°C gekühlte Lösung von 4 N Salzsäure (1.1 ml), Methanol (0.6 ml) und 40 % wässriger Dimethylaminlösung (2.5 ml, 19.2 mmol) wurde zu 4-((3-(Triethylsilyl)prop-2-ynyloxy)methyl)cyclohexanon (1.12 mg, 4 mmol) gegeben. Dieses Gemisch wurde mit Kaliumcyanid (624 mg, 9.6 mmol) versetzt und 5 d bei Raumtemperatur gerührt. Nach Zugabe von Wasser (10 ml) wurde mit Diethylether (3 × 10 ml) extrahiert. Die vereinigten organischen Phasen wurden mit Natriumsulfat getrocknet und i. Vak. eingeengt.

### Ausbeute: 1.04 g (78 %), farbloses Öl

### Nitrilbaustein 3: 4-But-2-inyloxymethyl-1-dimethylaminocyclohexancarbonitril (Nit-03) (Diastereomerengemisch)

### Stufe 1: 8-But-2-inyloxymethyl-1,4-dioxaspiro[4.5]decan

Zu einer Lösung von (1,4-Dioxaspiro[4.5]dec-8-yl)methanol (Stufe 2, Nitrilbaustein 1) (8.6 g, 50 mmol) in wasserfreiem Tetrahydrofuran (200 ml) wurde unter Eiskühlung eine 60 %

Dispersion von Natriumhydrid in Mineralöl (2.1 g, 55 mmol) gegeben. Das Gemisch wurde 1.5 h unter Rückfluss gerührt, anschließend auf 0 °C abgekühlt und tropfenweise mit einer Lösung von 1-Brombut-2-in (19.2 g, 144 mmol) in wasserfreiem Tetrahydrofuran (40 ml) versetzt. Das Gemisch wurde über Nacht bei Raumtemperatur gerührt und das Tetrahydrofuran anschließend i. Vak. abdestilliert. Der Rückstand wurde mit Wasser (140 ml) versetzt und mit Dichlormethan (4 × 40 ml) extrahiert. Die vereinigten organischen Phasen wurden mit Natriumsulfat getrocknet und i. Vak. eingeengt. Das Rohprodukt (15.0 g) wurde durch Flashchromatographie (400 g, 20 × 7.6 cm) mit Cyclohexan / Ethylacetat (4:1) gereinigt.

### Ausbeute: 4.47 g (40 %), gelbliches Öl

*¹H-NMR (DMSO-d₆):* 1.10-1.23 (m, 2 H); 1.36-1.47 (m, 2 H); 1.54 (m, 1 H); 1.64 (d, 4H, J = 10.2 Hz); 1.81 (t, 3 H, J = 2.3 Hz); 3.24 (d, 2H, J = 6.4 Hz); 3.83 (s, 4H); 4.04 (q, 2H, J = 2.3 Hz).

### Stufe 2: 4-But-2-inyloxymethylcyclohexanon

Eine Lösung von 8-But-2-inyloxymethyl-1,4-dioxaspiro[4.5]decan (4.75 g, 21.1 mmol) in Aceton (150 ml) wurde mit 2 *M* Salzsäure (56 ml) versetzt und 48 h bei Raumtemperatur gerührt. Das Aceton wurde anschließend i. Vak. abdestilliert, der Rückstand mit 4 *M* Natronlauge alkalisch gestellt und mit Dichlormethan (3 × 30 ml) extrahiert. Die vereinigten organischen Phasen wurden mit Natriumsulfat getrocknet und i. Vak. eingeengt.

### Ausbeute: 3.59 g (94 %), gelbliches Öl

*¹H-NMR (DMSO-d₆):* 1.26-1.44 (m, 2H); 1.82 (t, 3H, J = 2.3 Hz); 1.90-2.02 (m, 3H); 2.14-2.24 (m, 2H); 2.37 (dt, 2H, J = 13.6, 5.9 Hz); 3.33 (d, 2H, J = 6.1 Hz); 4.08 (q, 2H, J = 2.3 Hz).

### Stufe 3: 4-But-2-inyloxymethyl-1-dimethylaminocyclohexancarbonitril (Nit-03) (Diastereomerengemisch)

Zu einer auf 0 °C gekühlten Mischung von 4 M Salzsäure (4.96 ml) und Methanol (5.3 ml) wurde 40 % wässrige Dimethylaminlösung (12.12 ml) und anschließend eine Lösung von 4-But-2-inyloxymethylcyclohexanon (3.57 g, 19.8 mmol) in Methanol (23.6 ml) gegeben. Dieses Gemisch wurde danach mit Kaliumcyanid (3.12 g, 46.8 mmol) versetzt und über Nacht bei Raumtemperatur gerührt. Nach Zugabe von Wasser (90 ml) wurde mit Diethylether (3 × 30 ml) extrahiert. Die vereinigten organischen Phasen wurden mit Natriumsulfat getrocknet und i. Vak. eingeengt. Es handelt sich um ein Diastereoisomerengemisch im Verhältnis von ca. 2:1.

### Ausbeute: 4.16 g (89 %), gelbliches Öl

*¹H-NMR (DMSO-d₆):* 1.06-1.20 (m, 1.3H); 1.22-1.40 (m, 2H); 1.42-1.60 (m, 2.3H), 1.62-1.74 (m, 1 H); 1.816 und 1.823 (2 t, 3H, J = 2.4 Hz); 2.09 (m, 0.7H); 2.18-2.25 (m, 1.7H); 2.21 und 2.24 (2s, 6H); 3.23 (d, 0.7H, J = 6.4 Hz); 3.27 (d, 1.3H; J = 6.4 Hz); 4.04 (q, 0.7H, J = 2.4 Hz); 4.06 (q, 1.3H, J = 2.4 Hz).

### Nitrilbaustein 4: 1-(Dimethylamino)-4-((4,4-dimethylpent-2-ynyloxy)methyl)cyclohexancarbonitril (Nit-04) (Diastereomerengemisch)

### Stufe 1: 4,4-Dimethylpent-2-in-1-ol

Eine 2.5 *M* Lösung von n-Butyllithium (19.5 g, 122 ml, 304 mmol) in Hexan wurde bei 0 °C unter Argon langsam zu einer Lösung von 3,3-Dimethyl-1-butin (25.0 g, 304 mmol) in absolutem Diethylether (125 ml) getropft. Anschließend wurde absolutes Tetrahydrofuran (100 ml) dazu getropft, die Reaktionslösung auf Raumtemperatur erwärmt, über einen Zeitraum von 45 min portionsweise mit Paraformaldehyd (11.5 g, 383 mmol) versetzt, dann 4 h unter Rückfluss und danach über Nacht bei Raumtemperatur gerührt. Das Reaktionsgemisch wurde in Wasser (1 L) gegossen, die Phasen getrennt und die wässrige Phase mit Diethylether extrahiert (3 × 100 ml). Die vereinigten organischen Phasen wurden mit gesättigter Ammoniumchlorid-Lösung und gesättigter Natriumchlorid-Lösung (je 100 ml) gewaschen, mit Natriumsulfat getrocknet und wegen des geringen Siedepunktes des Produktes (Kₚ = 140 °C bei Normaldruck, 68-69 °C bei 17 Torr) vorsichtig i. Vak. eingeengt. Da das Produkt (31.6 g) sehr rein anfiel, konnte es direkt weiter umgesetzt werden.

### Ausbeute: 31.6 g (93 %), gelbes Öl

*¹H-NMR (DMSO-d₆):* 1.17 (s, 9H); 4.01 (d, 2H, J = 5.4 Hz); 5.00 (t, 1 H, J = 5.6 Hz).

### Stufe 2: 1-Brom-4,4-dimethylpent-2-in

Zu einer Aufschlämmung von Triphenylphosphindibromid (107 g, 253 mmol) in absolutem Acetonitril (750 ml) und Imidazol (17.2 g, 253 mmol) wurde unter Argon eine Lösung von 4,4-Dimethylpent-2-in-1-ol (25.0 g, 223 mmol) in absolutem Acetonitril (100 ml) getropft. Anschließend wurde über Nacht bei Raumtemperatur gerührt. Wegen des geringen Siedepunktes des Produktes (Kₚ = 60-64 °C bei 20 Torr) wurde bei Raumtemperatur i. Vak. eingeengt. Der Rückstand wurde mit Pentan (1 L) gewaschen und das Filtrat wiederum bei Raumtemperatur i. Vak. eingeengt. Der Rückstand enthielt noch etwas Feststoff und wurde deshalb nochmals auf die eben beschriebene Weise mit Pentan (100 ml) behandelt. Da das Produkt (14.7 g) sehr rein anfiel, konnte es direkt weiter umgesetzt werden.

### Ausbeute: 14.7 g (38 %), hellgelbes Öl

*¹H-NMR (DMSO-d₆):* 1.18 (s, 9H); 4.19 (s, 2H).

### Stufe 3: 8-(4,4-Dimethylpent-2-inyloxymethyl)-1,4-dioxaspiro[4.5]decan

Zu einer 60 % Natriumhydrid-Suspension in Mineralöl (1.02 g, 25.5 mmol) in absolutem Tetrahydrofuran (50 ml) wurde unter Argonspülung eine Lösung von (1,4-Dioxaspiro[4.5]dec-8-yl)methanol (3.67 g, 21.3 mmol) in absolutem Tetrahydrofuran (25 ml) getropft, welche anschließend 90 min unter Rückfluss gekocht wurde. Nach Abkühlung auf Raumtemperatur wurde eine Lösung von 1-Brom-4,4-dimethylpent-2-in (11.2 g, 64.0 mmol) in absolutem Tetrahydrofuran (25 ml) dazu getropft und anschließend über das Wochenende gerührt. Die Reaktionslösung wurde i. Vak. eingeengt, mit Wasser (100 ml) versetzt und die Suspension mit Ethylacetat (3 × 50 ml) extrahiert. Die vereinigten organischen Phasen wurden mit gesättigter Natriumchlorid-Lösung (50 ml) gewaschen, mit Natriumsulfat getrocknet und i. Vak. eingeengt. Das Rohprodukt (11.3 g) wurde mittels Flashchromatographie (400 g, 20 × 7.5 cm) mit Cyclohexan/ Ethylacetat (9:1) gereinigt.

### Ausbeute: 1.70 g (30 %), hellgelbes Öl

*¹H-NMR (DMSO-d₆):* 1.18 (s, 9H); 1.35-1.47 (m, 2H); 1.49-1.58 (m, 1 H); 1.59-1.70 (m, 6H); 3.32 (s, 2H, J = 6.3 Hz); 3.83 (s, 4H); 4.02 (s, 2H).

### Stufe 4: 4-(4,4-Dimethylpent-2-inyloxymethyl]cyclohexanon

Eine Lösung von 8-(4,4-Dimethylpent-2-inyloxymethyl)-1,4-dioxaspiro[4.5]decan (1.70 g, 6.4 mmol) in Aceton (30 ml) wurde mit 1 *N* Salzsäure (6 ml) versetzt und über Nacht bei Raumtemperatur gerührt. Da noch Edukt nachweisbar war (LC-MS) wurde weitere 24 h gerührt. Die Reaktionslösung wurde danach mit 1 *N* Natronlauge auf pH 8 eingestellt und i. Vak. eingeengt. Der Rückstand wurde mit Wasser (20 ml) versetzt und anschließend mit Dichlormethan (3 × 20 ml) extrahiert. Die vereinigten organischen Phasen wurden mit gesättigter Natriumchlorid-Lösung (20 ml) gewaschen, mit Natriumsulfat getrocknet und i. Vak. eingeengt. Das Rohprodukt (1.49 g; enthielt lt. ¹H-NMR-Spektrum ca. 5 % 8-(4,4-Dimethylpent-2-inyloxymethyl)-1,4-dioxaspiro[4.5]decan Dieses wurde ohne Reinigung weiter umgesetzt.

### Ausbeute: 1.49 g (100 %), hellgelbes Öl

*¹H-NMR (DMSO-d₆):* 1.19 (s, 9H); 1.37-1.43 (m, 2H); 1.92-2.08 (m, 3H); 2.16-2.24 (m, 2H); 2.32-2.41 (m, 2H); 3.33 (d, 2H, J = 6.3 Hz); 4.08 (s, 2H).

LC-MS : m/z: [M+1]⁺ = 223.2, Rₜ 3.4 min.

### Stufe 5: 1-(Dimethylamino)-4-((4,4-dimethylpent-2-ynyloxy)methyl)cyclohexancarbonitril (Nit-04) (Diastereomerengemisch)

Zu einer eisgekühlten Mischung von 4 N Salzsäure (1.68 ml, 6.7 mmol) und Methanol (7 ml) wurde zuerst 40 % Dimethylaminlösung (1.64 ml, 26.6 mmol), dann 4-(4,4-Dimethylpent-2-inyloxymethyl]cyclohexanon (1.49 g, 6.7 mmol) und Kaliumcyanid (1.05 g, 16.1 mmol) gegeben. Die entstandene Suspension wurde 66 h bei Raumtemperatur gerührt. Die Suspension wurde mit Wasser (50 ml) versetzt und anschließend mit Diethylether (3 × 50 ml) extrahiert. Die vereinigten organischen Phasen wurden mit Natriumsulfat getrocknet und i. Vak. eingeengt. Das Rohprodukt (1.54 g) wurde direkt weiter umgesetzt. Es handelt sich um ein Diastereoisomerengemisch im Verhältnis 2:1.

### Ausbeute: 1.54 g (83 %), hellgelbes Öl

*¹H-NMR (DMSO-d₆):* 1.18 und 1.19 (2s, 9H); 1.24-2.12 (m, 8H); 2.16-2.20 (m, 1 H); 2.21 und 2.24 (2s, 6H); 3.23 und 3.27 (2d, 2H, J = 6.3 Hz); 4.05 und 4.06 (2s, 2H).

### Triazolbaustein 1

### 1-Dimethylamino-1-[1,2,3]triazol-1-yl-4-(3-triethylsilanylprop-2-ynyloxymethyl)cyclohexan (Tri-01)

Zu einer Lösung von zu 4-((3-(Triethylsilyl)prop-2-ynyloxy)methyl)cyclohexanon (2.00 g, 7.1 mmol) in wasserfreiem Tetrahydrofuran (50 ml) wurde eine 2 M Lösung von Dimethylamin in Tetrahydrofuran (4.4 ml, 8.8 mmol), 1,2,3-Triazol (540 mg, 7.8 mmol) und 4 A Molsieb (2.70 g) gegeben. Das Gemisch wurde über Nacht bei Raumtemperatur gerührt, anschließend filtriert und sofort weiter umgesetzt.

### Ausbeute: 2.67 g, (100 %)

*¹H NMR (DMSO-d₆):* 0.57 (q, 6H, J = 7.7 Hz); 0.95 (t, 9H, J = 7.8 Hz); 1.12-1.30 (m 1H); 1.55-1.82 (m, 4H); 1.90-2.25 (m, 4H); 2.47 (s, 6H); 3.27-3.41 (m, 2H); 4.15 (s, 2H); 6.87 (s, 1 H); 7.84 (s, 1 H).

### Alkinbaustein 1 (Methode 1):

### 1-Phenyl-4-((3-(triethylsilanyl)prop-2-ynyloxy)methyl)-N,N-dimethylcyclohexanamin (Ain-01; eines von zwei Diastereomeren)

Eine 2 *M* Lösung von Phenylmagnesiumchlorid (16.8 ml, 33.6 mmol) in Tetrahydrofuran wurde unter Argon und bei 0 °C tropfenweise mit einer Lösung von von 1-(Dimethylamino)-4-((3-(triethylsilyl)prop-2-ynyloxy)methyl)cyclohexancarbonitril **(Nit-02)** (4.50 g, 13.4 mmol) in wasserfreiem Tetrahydrofuran (40 ml) versetzt und anschließend über Nacht bei Raumtemperatur gerührt. Nach Zugabe von gesättigter Ammoniumchloridlösung und Wasser (je 20 ml) wurden die Phasen getrennt und die wässrige Phase mit Diethylether (3 × 30 ml) extrahiert. Die vereinigten organischen Phasen wurden mit Natriumsulfat getrocknet und i. Vak. eingeengt. Das Rohprodukt (5.00 g) wurde durch Flashchromatographie (100 g, 20 × 4.0 cm) mit Ethylacetat / Cyclohexan (1:9) und die erhaltenen Mischfraktionen (2.00 g) durch erneute Flashchromatographie (100 g, 20 × 4.0 cm) mit Chloroform / Isopropanol (300:1) gereinigt.

### Ausbeute: 2.67 g (52 %), farbloses Öl

*¹H-NMR (DMSO-d₆):* 0.58 (q, 6H, J = 7.8 Hz); 0.96 (t, 9H, J = 7.8 Hz); 1.25-1.65 (m, 7H); 1.91 (s, 6H); 2.57 (br d, 2H, J = 11.4 Hz); 3.35 (d, 2H, J = 6.1 Hz); 4.15 (s, 2H); 7.21-7.38 (m, 5H).

Es wurde nur eines der beiden möglichen Diastereoisomeren isoliert.

### Alkinbaustein 1 (Methode 2):

### 1-Phenyl-4-((3-(triethylsilanyl)prop-2-ynyloxy)methyl)-N,N-dimethylcyclohexanamin (Ain-01; eines von zwei Diastereomeren)

Eine 2 *M* Lösung von Phenylmagnesiumchlorid (8.9 ml, 18 mmol) in Tetrahydrofuran wurde unter Argon und bei 0 °C tropfenweise mit einer Lösung von 1-Dimethylamino-1-[1,2,3]triazol-1-yl-4-(3-triethylsilanylprop-2-ynyloxymethyl)cyclohexan **(Tri-01)** (2.67 g, 7.1 mmol) in wasserfreiem Tetrahydrofuran (50 ml) versetzt und anschließend bei Raumtemperatur über Nacht gerührt. Nach Zugabe von gesättigter Ammoniumchloridlösung und Wasser (je 10 ml) wurden die Phasen getrennt und die wässrige Phase mit Diethylether (3 × 30 ml) extrahiert. Die organische Phase wurde mit Natriumsulfat getrocknet und i. Vak. eingeengt. Das Rohprodukt (2.60 g) wurde durch Flashchromatographie (100 g, 20 × 4.0 cm) mit Ethylacetat / Cyclohexan (1:9) und Ethylacetat / Methanol (1:9) gereinigt.

### Ausbeute: 779 mg (28 %), Öl

*¹H-NMR (DMSO-d₆):* 0.58 (q, 6H, J = 7.9 Hz); 0.96 (t, 9H, J = 7.9 Hz); 1.30-1.54 (m, 7H); 1.91 (s, 6H); 2.58 (br d, 2H, J = 11.3 Hz); 3.35 (d, 2H, J = 6.2 Hz); 4.15 (s, 2H); 7.20-7.40 (m, 5H).

### Alkinbaustein 2:

### 1-Phenyl-4-((3-(tert-butyldimethylsilyl)prop-2-ynyloxy)methyl)-N,N-dimethylcyclohexanamin (Ain-02; eines von zwei Diastereomeren)

Eine 2 *M* Lösung von Phenylmagnesiumchlorid (20.5 ml, 41 mmol) in Tetrahydrofuran wurde unter Argon bei 0 °C tropfenweise mit einer Lösung von 4-((3-(*tert*-Butyldimethylsilyl)prop-2-ynyloxy)methyl)-1-(dimethylamino)cyclohexancarbonitril (Nit-01) (5.50 g, 16.4 mmol) in wasserfreiem Tetrahydrofuran (80 ml) versetzt und anschließend bei Raumtemperatur über Nacht gerührt. Nach Zugabe von gesättigter Ammoniumchloridlösung (15 ml) und Wasser (15 ml) wurden die Phasen getrennt und die wässrige Phase mit Diethylether (3 × 30 ml) extrahiert. Die vereinigten organischen Phasen wurden mit Natriumsulfat getrocknet und i. Vak. eingeengt. Das Rohprodukt (5.73 g) wurde durch Flashchromatographie (200 g, 20 × 5.7 cm) mit Ethylacetat / Cyclohexan (1:9→1:2) gereinigt.

### Ausbeute: 3.98 g (63 %), gelbliches Öl

*¹H-NMR (DMSO-d₆):* 0.10 (s, 6H); 0.93 (s, 9H); 1.30-1.65 (m, 7H); 1.92 (s, 6H); 2.57 (d, 2H, J = 12.4 Hz); 3.35 (d, 2H, J = 6.3 Hz); 4.15 (s, 2H); 7.29-7.36 (m, 5H).

*¹³C-NMR (DMSO-d₆):* -4.8; 16.1; 24.4; 25.8; 32.0; 36.7, 37.4; 58.1; 58.5; 74.5; 88.2; 103.8; 126.1; 126.5; 127.2; 139.3.

### Alkinbausteine 4 & 7:

### 1-Benzyl-4-((3-(tert-butyldimethylsilyl)prop-2-ynyloxy)methyl)-N,N-dimethylcyclo-hexanamin (Ain-04; Unpolareres Diastereomer und Ain-07; Polareres Diastereomer)

In einem ausgeheizten Kolben wurde zu einer 2 M Lösung von Benzylmagnesiumchlorid in Tetrahydrofuran (22.8 ml, 45.6 mmol) unter Argon und Eiskühlung eine Lösung von 4-((3-(*tert*-Butyldimethylsilyl)prop-2-ynyloxy)methyl)-1-(dimethylamino)cyclohexancarbonitril (Nit-01) (5.10 g, 15.2 mmol) in Tetrahydrofuran (25 ml) getropft. Das Gemisch wurde 20 h bei Raumtemperatur gerührt und danach unter Eis-Wasser-Kühlung mit 20 % Ammoniumchloridlösung (20 ml) versetzt und mit Diethylether (3 × 50 ml) extrahiert. Die vereinigten organischen Phasen wurden mit gesättigter Natriumchloridlösung gewaschen, mit Natriumsulfat getrocknet und i. Vak. eingeengt. Das Rohprodukt (6 g) wurde durch Flashchromatographie (360 g, 20 × 7.5 cm) mit Ethylacetat / Cyclohexan (1:4→1:2→1:1 und danach mit Methanol / 25 % wässriger Ammoniaklösung (9:1) gereinigt. Das polare Diastereoisomer wurde erneut mittels Flashchromatographie (90 g, 20 × 4 cm) mit Ethylacetat / Methanol (4:1) gereinigt.

### Ain-04, Unpolares Diastereoisomer

### Ausbeute: 2.25 g (37 %), farbloses Öl

*¹H-NMR (DMSO-d₆):* 0.05 (s, 6H); 0.88 (s, 9H); 0.90-1.01 (m, 2H); 1.16-1.34 (m, 5H); 1.78 (d, J = 13.0 Hz, 2H); 2.22 (s, 6H); 2.56 (s, 2H); 3.20 (d, J = 4.4 Hz, 2H); 4.06 (s, 2H);7.09-7.27 (m, 5H).

### Ain-07, Polareres Diastereomer Ausbeute: 1.5 g (25 %), farbloses Öl

*¹H-NMR (DMSO-d₆):* 0.08 (s, 6H); 0.91 (s, 9H); 1.14-1.77 (m, 9H); 2.25 (s, 6H); 2.71 (br s, 2H); 3.25 (d, J = 6.5 Hz, 2H); 4.09 (s, 2H); 7.12-7.33 (m, 5H).

### Alkinbausteine 5 & 6:

### 1-Benzyl-N,N-dimethyl-4-((3-(triethylsilyl)prop-2-ynyloxy)methyl)cyclohexanamin (Ain-05, Unpolareres Diastereomer und Ain-06, Polareres Diastereomer)

Eine 2 M Lösung von Benzylmagnesiumchlorid in Tetrahydrofuran (22.4 ml, 44.8 mmol) wurde unter Argon und Eiskühlung tropfenweise mit einer Lösung von 1-(Dimethylamino)-4-((3-(triethylsilyl)prop-2-ynyloxy)methyl)cyclohexancarbonitril **(Nit-02)** (5.0 g, 14.9 mmol) in Tetrahydrofuran (25 ml) versetzt und 20 h bei Raumtemperatur gerührt. Anschließend wurde das Reaktionsgemisch unter Eis-Wasser-Kühlung mit 20 % Ammoniumchloridlösung (20 ml) versetzt. Die Phasen wurden getrennt und die wässrige Phase mit Diethylether (3 × 50 ml) extrahiert. Die vereinigten organischen Phasen wurden mit gesättigter Natriumchloridlösung gewaschen, mit Natriumsulfat getrocknet und i. Vak. eingeengt. Das Rohprodukt (6.8 g) wurde durch Flashchromatographie (250 g, 23 × 5.5 cm) mit Ethylacetat / Cyclohexan (1:4→1:1→1:0), danach mit Ethylacetat/Methanol (4:1) gereinigt. Das polare Diastereoisomer wurde erneut mittels Flashchromatographie (40 g, 9 × 4 cm) mit Ethylacetat / Methanol (4:1) gereinigt.

### Ain-05 (Unpolareres Diastereomer)

### Ausbeute: 2.08 g (35 %), farbloses Öl

*¹H-NMR (DMSO-d₆):* 0.54 (q, J = 8.2, 7.9 Hz, 6H); 0.92 (t, J = 7.9 Hz, 9H); 0.92-1.01 (m, 2H); 1.17-1.34 (m, 5H); 1.78 (d, J = 12.9 Hz, 2H); 2.22 (s, 6H); 2.56 (s, 2H); 3.21 (d, J = 4.7 Hz, 2H); 4.07 (s, 2H); 7.09-7.27 (m, 5H).

### Ain-06 (Polareres Diastereomer)

### Ausbeute: 1.78 g (30 %), farbloses Öl

*¹H NMR (DMSO-d₆):* 0.56 (q, J = 7.8 Hz, 6H); 0.94 (t, J = 7.8 Hz, 9H); 1.16-1.35 (m, 4H); 1.40-1.53 (m, 2H); 1.55-1.73 (m, 3H); 2.21 (s, 6H); 2.68 (s, 2H); 3.25 (d, J = 6.4 Hz, 2H); 4.09 (s, 2H); 7.10-7.27 (m, 5H).

### Alkinbaustein 8:

### 1-Butyl-N,N-dimethyl-4-((3-(triethylsilyl)prop-2-ynyloxy)methyl)cyclohexanamin (Ain-08, Unpolareres Diastereomer)

Eine 2 *M* Lösung von *n*-Butylmagnesiumchlorid in Tetrahydrofuran (18.7 ml, 37.5 mmol) wurde unter Eiskühlung tropfenweise mit einer Lösung von **Nit-02** (5.02 g, 15.0 mmol) in wasserfreiem Tetrahydrofuran (80 ml) versetzt und über Nacht bei Raumtemperatur gerührt. Das Reaktionsgemisch wurde anschließend unter Eiskühlung tropfenweise mit gesättigter Ammoniumchloridlösung (15 ml) und Wasser (15 ml) versetzt. Die Phasen wurden getrennt und die wässrige Phase mit Diethylether (3 × 30 ml) extrahiert. Die vereinigten organischen Phasen wurden mit Natriumsulfat getrocknet und i. Vak. eingeengt. Das Rohprodukt (5.28 g) wurde durch Flashchromatographie (420 g, 30 × 7.6 cm) mit Cyclohexan / Ethylacetat (4:1)→Cyclohexan / Ethylacetat (1:1)→Ethylacetat / Methanol (1:1) gereinigt.

### Ain-08, Unpolareres Diastereomer

### Ausbeute: 3.14 g (57 %), gelbliches Öl

*¹H-NMR (DMSO-d₆):* 0.57 (q, 6H, J= 7.9 Hz); 0.87 (t, 3H, J = 7.1 Hz); 0.95 (t, 9H, J = 7.8 Hz); 1.10-1.56 (m, 13H); 1.66 (d, 2H, J = 11.8 Hz); 2.12 (s, 6H); 3.27 (d, 2H, J = 6.3 Hz); 4.12 (s, 2H).

*¹³C-NMR (DMSO-d₆):* 3.7; 7.2; 13.9; 23.3; 23.9; 25.4; 26.3; 28.8; 30.6; 31.4; 35.8; 36.7; 37.1; 55.6; 58.1; 74.9; 87.2; 104.4.

### Alkinbaustein 9:

### N,N-Dimethyl-1-(thiophen-2-yl)-4-((3-(triethylsilyl)prop-2-ynyloxy)methyl)cyclohexanamin (Ain-09; Eines von zwei möglichen Diastereomeren)

Eine 1 M Lösung von 2-Thienylmagnesiumbromid in Tetrahydrofuran (15.8 ml, 15.8 mmol) wurde bei 0 °C unter Argon tropfenweise mit einer Lösung von **Nit-02** (2.12 g, 6.34 mmol) in wasserfreiem Tetrahydrofuran (20 ml) versetzt und anschließend bei Raumtemperatur über Nacht gerührt. Nach Zugabe von gesättigter Ammoniumchloridlösung (5 ml) und Wasser (10 ml) wurden die Phasen getrennt und die wässrige Phase mit Diethylether (3 × 20 ml) extrahiert. Die vereinigten organischen Phasen wurden mit Natriumsulfat getrocknet und i. Vak. eingeengt. Das Rohprodukt (2.40 g) wurde durch Flashchromatographie (200 g, 20 × 5.7 cm) mit Ethylacetat / Cyclohexan (1:9) gereinigt.

### Ausbeute: 1.36 g (55 %), bräunliches Öl

*¹H-NMR (DMSO-d₆):* 0.53-0.63 (m, 6H); 0.92-1.00 (m, 9H); 1.33-1.70 (m, 7H); 1.99 (s, 6H); 2.39 (br d, 2H, J = 11.9 Hz); 3.27-3.35 (m, 2H vom HDO-Signal überlagert); 4.15 (s, 2H); 6.91 (dd, 1H, J = 3.5, 1.0 Hz); 7.03 (dd, 1 H, J = 5.1, 3.5 Hz); 7.39 (dd, 1 H, J = 5.1, 0.9 Hz). *¹³C-NMR (DMSO-d₆):* 3.7; 7.2; 24.2; 34.6; 36.6; 37.4; 58.1; 58.2; 74.4; 87.3; 104.3; 123.0; 123.8; 126.1; 145.0.

*LC-MS:* (Methode: ASCA-7MIN-80GRAD.M): m/z: [M+H]⁺ = 392.3, Rₜ 3.5 min

### Alkinbaustein 10:

### 1-(1-Phenyl-4-((3-(triethylsilyl)prop-2-ynyloxy)methyl)cyclohexyl)azetidin (Ain-10, Eines von zwei möglichen Diastereomeren)

### Stufe 1: 1-(Azetidin-1-yl)-4-((3-(triethylsilyl)prop-2-ynyloxy)methyl)cyclohexancarbonitril

Zu einer auf 0 °C gekühlten Mischung von 4 N Salzsäure (2 ml) und Methanol (1 ml) wurde Azetidin (2.18 g, 2.58 ml, 38.3 mmol) und anschließend eine Lösung von 4-((3-(Triethylsilyl)prop-2-ynyloxy)methyl)cyclohexanon (2.24 g, 8 mmol) in Methanol (5 ml) gegeben. Zu diesem Gemisch wurde eine Lösung von Kaliumcyanid (1.24 g, 19.1 mmol) in Wasser (5 ml) gegeben und danach 3 h bei Raumtemperatur gerührt. Nach Zugabe von Wasser (10 ml) zum Reaktionsgemisch wurde mit Diethylether (3 × 20 ml) extrahiert. Die vereinigten organischen Phasen wurden mit Natriumsulfat getrocknet und i. Vak. eingeengt.

### Ausbeute: 2.52 g (91 %), gelbliches Öl

*¹H-NMR (DMSO-d₆): 0.54-0.61* (m, 6H); 0.96 (t, 9H, J = 7.8 Hz); 1.05-1.85 (m, 9H); 1.92-2.02 (m, 2H); 3.15-3.23 (m, 4H); 3.27-3.33 (m, 2H); 4.13 und 4.15 (2 s, 2H).

Es handelt sich um ein Diastereoisomerengemisch.

### Stufe 2: 1-(1-Phenyl-4-((3-(triethylsilyl)prop-2-ynyloxy)methyl)cyclohexyl)azetidin (Ain-10, Eines von zwei möglichen Diastereomeren)

Eine 2 *M* Lösung von Phenylmagnesiumchlorid in Tetrahydrofuran (9 ml, 18 mmol) wurde bei 0 °C unter Argon tropfenweise mit einer Lösung von 1-(Azetidin-1-yl)-4-((3-(triethylsilyl)prop-2-ynyloxy)methyl)cyclohexancarbonitril (2.52 g, 7.2 mmol) in wasserfreiem Tetrahydrofuran (20 ml) versetzt und anschließend bei Raumtemperatur über Nacht gerührt. Nach Zugabe von gesättigter Ammoniumchloridlösung (5 ml) und Wasser (10 ml) wurden die Phasen getrennt und die wässrige Phase mit Diethylether (3 × 20 ml) extrahiert. Die vereinigten organischen Phasen wurden mit Natriumsulfat getrocknet und i. Vak. eingeengt. Das Rohprodukt (2.72 g) wurde durch Flashchromatographie (100 g, 20 × 4.0 cm) mit Ethylacetat / Cyclohexan (1:9) gereinigt.

### Ausbeute: 1.76 g (61 %), gelbliches Öl

*¹H-NMR (DMSO-d₆):* 0.53-0.63 (m, 6H); 0.92-1.00 (m, 9H); 1.25-1.35 (m, 2H); 1.45-1.53 (m, 5H); 1.61 (quin, 2H, J = 7.1 Hz); 2.19 (br d, 2H, J = 13.0 Hz); 2.83 (t, 4H, J = 6.8 Hz); 3.34-3.40 (m, 2H); 4.16 (s, 2H); 7.22-7.42 (m, 5H).

*¹³C-NMR (DMSO-d₆):* 3.7; 7.2; 15.6; 24.7; 28.8; 30.3; 35.1; 36.4; 45.8; 57.2; 58.2; 72.9; 74.4; 87.3; 104.3; 126.3; 126.6; 127.4; 140.1.

*LC-MS:* (Methode: ASCA-7MIN-80Grad.M): m/z: [M+H]⁺ = 398.3, Rₜ 3.5 min.

Es konnte nur ein Diastereoisomer gewonnen werden. Bereits das ¹H-NMR-Spektrum des Rohproduktes zeigte, dass es sich im Wesentlichen nur um ein Diastereoisomer handeln muss.

### Alkinbaustein 12:

### 4-((But-2-ynyloxy)methyl)-N,N-dimethyl-1-phenylcyclohexanamin (Ain-12, Eines von zwei möglichen Diastereomeren)

Eine 2 *M* Lösung von Phenylmagnesiumchlorid in Tetrahydrofuran (26.6 ml, 53.3 mmol) wurde unter Eiskühlung tropfenweise mit einer Lösung von 4-But-2-inyloxymethyl-1-dimethylaminocyclohexancarbonitril **(Nit-03)**(4.16 g, 17.8 mmol) in wasserfreiem Tetrahydrofuran (80 ml) versetzt und über Nacht bei Raumtemperatur gerührt. Das Gemisch wurde anschließend unter Eiskühlung tropfenweise mit gesättigter Ammoniumchloridlösung und Wasser (jeweils 15 ml) versetzt. Das Tetrahydrofuran wurde i. Vak. abdestilliert und der Rückstand mit Diethylether (3 × 40 ml) extrahiert. Die vereinigten organischen Phasen wurden mit Natriumsulfat getrocknet und i. Vak. eingeengt. Das Rohprodukt (3.53 g) wurde durch Flashchromatographie (370 g, 20 × 7.6 cm) mit Ethylacetat / Methanol (9:1) gereinigt. Es wurde nur ein Diastereoisomer isoliert.

### Ausbeute: 2.47 g (48 %), gelbliches Öl

*¹H-NMR (DMSO-d₆):* 1.30-1.64 (m, 7H); 1.83 (t, 3H, J = 2.3 Hz); 1.92 (s, 6H); 2.58 (br d, 2H, J = 12.5 Hz), 3.29 (d, 2H, J = 6.3 Hz); 4.06 (q, 2H, J = 2.3 Hz); 7.23 (m, 1 H); 7.29-7.37 (m, 4H).

### Alkinbaustein 13:

### 4-((4,4-dimethylpent-2-ynyloxy)methyl)-N,N-dimethyl-1-phenylcyclohexanamin (Ain-13, Eines von zwei möglichen Diastereomeren)

Zu einer eisgekühlten 2 *M* Lösung von Phenylmagnesiumchlorid (1.92 g, 7.0 ml, 14.0 mmol) in Tetrahydrofuran wurde unter Argon langsam eine Lösung von 1-(dimethylamino)-4-((4,4-dimethylpent-2-ynyloxy)methyl)cyclohexancarbonitril **(Nit-04)** (1.54 g, 5.6 mmol) in absolutem Tetrahydrofuran (30 ml) getropft und anschließend über Nacht bei Raumtemperatur gerührt. Die Reaktionslösung wurde anschließend mit gesättigter Ammoniumchlorid-Lösung und Wasser (je 20 ml) versetzt, die Phasen wurden getrennt und die wässrige Phase mit Diethylether (3 × 30 ml) extrahiert. Die vereinigten organischen Phasen wurden mit gesättigter Natriumchlorid-Lösung (30 ml) gewaschen, mit Natriumsulfat getrocknet und i. Vak. eingeengt. Es fielen 1.61 g Rohprodukt an, welches mittels Flashchromatographie (100 g, 20 × 4.0 cm) mit Dichlormethan / Methanol (95:5) gereinigt wurde.

### Ausbeute: 942 mg (51 %), farbloses Öl, welches langsam kristallisiert

*¹H-NMR (DMSO-d₆):* 1.20 (s, 9H); 1.27-1.64 (m, 7H); 1.92 (s, 6H); 2.54-2.59 (m, 2H); 3.29 (d, 2H, J = 6.1 Hz); 4.07 (s, 2H); 7.20-7.27 (m, 1H); 7.29-7.37 (m, 4H)

Es wurde nur ein Diastereoisomer isoliert. Das ¹H-NMR-Spektrum des Rohproduktes zeigte, dass das mögliche zweite Isomer zu höchstens 5 % vorhanden war.

### Iodanilinbaustein 1:

### 2-Iodanilin (Ian-01)

Cas Nr.: 615-43-0; zur Zeit der Synthese kommerziell erhältlich bei z.B. Aldrich

### Iodanilinbaustein 2:

### 4-Amino-3-iodbenzonitril (Ian-02)

Cas Nr.: 33348-34-4; zur Zeit der Synthese kommerziell erhältlich bei z.B. Aldrich

### Iodanilinbaustein 3:

### 4-Amino-3-iodbenzotrifluorid (Ian-03)

Cas Nr.: 163444-17-5; zur Zeit der Synthese kommerziell erhältlich bei z.B. ABCR

### Iodanilinbaustein 4:

### 4-Fluor-2-iodanilin (Ian-04)

Cas Nr.: 61272-76-2; zur Zeit der Synthese kommerziell erhältlich bei z.B. ABCR

### Iodanilinbaustein 5:

### 2-Amino-3-iodpyridin (Ian-05)

Cas Nr.: 104830-06-0; zur Zeit der Synthese kommerziell erhältlich bei z.B. ABCR

### Iodanilinbaustein 6:

### 4-Amino-3-iodpyridin (Ian-06)

Cas Nr.: 88511-27-7; zur Zeit der Synthese kommerziell erhältlich bei z.B. ABCR

### Iodanilinbaustein 8:

### N-(4-Fluor-2-iodphenyl)acetamid (Ian-08)

Eine Lösung von 4-Fluor-2-iodanilin **(Ian-04)** (5.93 g, 25 mmol) und Triethylamin (3.5 ml) in wasserfreiem Dichlormethan (100 ml) wurde bei 0 °C mit einer Lösung von Acetylchlorid (2.55 g, 25.2 mmol) in wasserfreiem Dichlormethan (10 ml) versetzt. Das Gemisch wurde 1 h bei 0 °C und anschließend über Nacht bei Raumtemperatur gerührt und danach mit 25 % Kaliumcarbonatlösung alkalisch gestellt. Die Phasen wurden getrennt und die wässrige Phase mit Dichlormethan (2 × 30 ml) extrahiert. Die vereinigten organischen Phasen wurden mit Natriumsulfat getrocknet und i. Vak. eingeengt. Der Rückstand (7.01 g) wurde in Tetrahydrofuran (120 ml) aufgenommen und filtriert. Das Filtrat wurde i. Vak. eingeengt.

### Ausbeute: 6.60 g (95 %), weißer Feststoff

*Schmelzpunkt:* 142-144 °C

*¹H-NMR (DMSO-d₆):* 2.03 (s, 3H); 7.25 (dt, 1 H, J = 8.6, 2.9 Hz); 7.37 (dd, 1 H, J = 8.8. 5.7 Hz); 7.75 (dd, 1H, J = 8.2, 2.9 Hz); 9.46 (s, 1 H).

### Iodanilinbaustein 9:

### N-(4-Fluor-2-iodphenyl)methanesulfonamid (Ian-09)

Eine Lösung von 4-Fluor-2-iodanilin **(lan-04)** (2.38 g, 10 mmol) und 4-(Dimethylamino)-pyridin (121 mg, 1 mmol) in wasserfreiem Pyridin (25 ml) wurde bei Raumtemperatur tropfenweise mit Methansulfonylchlorid (1.37 g, 0.94 ml, 12 mmol) versetzt und anschließend 12 h unter Rückfluss gerührt. Das auf Raumtemperatur abgekühlte Gemisch wurde mit Dichlormethan (100 ml) verdünnt, mit 5 % Salzsäure (3 × 20 ml) und Wasser (3 × 20 ml) gewaschen. Die organische Phase wurde mit Natriumsulfat getrocknet und i. Vak. eingeengt. Der Rückstand wurde wiederholt mit Toluol versetzt und jeweils wieder i. Vak. eingeengt. Das Rohprodukt (2.91 g) wurde durch Flashchromatographie (100 g, 3.7 × 20 cm) mit Cyclohexan / Ethylacetat (4:1) gereinigt.

### Ausbeute: 2.25 g (71 %), beigefarbener Feststoff

*Schmelzpunkt:* 90-92 °C

*¹H-NMR (DMSO-d₆):* 3.03 (s, 3H); 7.29 (m, 1 H); 7.41 (dd, 1H, J = 5.5, 8.8 Hz); 7.80 (dd, 1H, J = 2.9, 8.2 Hz); 9.33 (s, 1H).

### Iodanilinbaustein 11:

### 4-Fluor-2-iod-N-methylanilin (Ian-11)

Eine Lösung von 4-Fluor-2-iodanilin **(Ian-04)** (5.93 g, 25 mmol) in wasserfreiem Tetrahydrofuran (50 ml) wurde bei -78 °C mit einer 1.6 M Lösung von Methyllithium in Tetrahydrofuran (15 ml, 24 mmol) versetzt und 30 min bei Raumtemperatur gerührt. Das Gemisch wurde bei -78 °C mit Dimethylsulfat (4.75 g, 37.6 mmol) versetzt, 10 min bei dieser Temperatur gerührt und anschließend 2 h bei Raumtemperatur gerührt. Das Reaktionsgemisch wurde danach tropfenweise mit Wasser (15 ml) versetzt und das Tetrahydrofuran i. Vak. abdestilliert. Der Rückstand wurde mit 1 M Salzsäure sauer gestellt und mit Diethylether (2 × 30 ml) extrahiert. Anschließend wurde die wässrige Phase mit 25 % Kaliumcarbonatlösung alkalisch gestellt und mit Diethylether (3 × 40 ml) extrahiert. Die organischen Phasen aus beiden Extraktionen wurden jeweils mit Natriumsulfat getrocknet und i. Vak. eingeengt. Das aus dem basischen Extrakt erhaltene Rohprodukt (1.37 g) wurde durch Flashchromatographie (100 g, 4.1 × 20 cm) mit Cyclohexan und anschließend Cyclohexan / Ethylacetat (95:5) gereinigt.

### Ausbeute: 943 mg, gelbes Öl

Es handelt sich um ein Produktgemisch, das auch die Zielverbindung enthielt.

Das aus dem sauren Extrakt erhaltene Rohprodukt (2.78 g) wurde ebenfalls durch Flashchromatographie (200 g, 5.6 × 20 cm) mit Cyclohexan gereinigt.

### Ausbeute: 1.10 g (17 %), gelbes Öl, das zu 88 % aus der Titelverbindung und zu 12 % aus dem entsprechenden Dimethylderivat besteht.

*¹H-NMR (DMSO-d₆):* 2.62 (s, 0.84H); 2.71 (d, 3H, J = 4.9 Hz); 4.87 (q, 1 H, J = 4.9 Hz); 6.48 (dd, 1 H, J = 5.0, 9.0 Hz); 7.10 (m, 1 H); 7.22 (m, 0.24 Hz); 7.51 (dd, 1H, J = 2.9, 8.1 Hz); 7.68 (m, 0.12H).

### Iodanilinbaustein 12:

### 4-Trifluormethoxy-2-iodanilin (lan-12)

Cas Nr.: 845866-79-7; zur Zeit der Synthese kommerziell erhältlich bei z.B. ABCR

### Beispiel 1:

### [1-Phenyl-4-(2-triethylsilanyl-1H-indol-3-ylmethoxymethyl)cyclohexyl]dimethyl-amin (Eines von zwei möglichen Diastereomeren

Eine Mischung von **Ain-01** (560 mg, 1.45 mmol), 2-lodanilin **(lan-01)** (381 mg, 1.74 mmol), [1,3-Bis-(2,6-diisopropylphenyl)imidazol-2-yliden]-(3-chlorpyridyl)palladium(II)-chlorid (PEPPSI, 197 mg, 0.29 mmol) und Natriumcarbonat (768 mg, 7.25 mmol) in wasserfreiem *N,N*-Dimethylformamid (10 ml) wurde 18 h bei 100 °C gerührt. Das Lösungsmittel wurde i. Vak. entfernt und der Rückstand in Wasser und Diethylether (je 50 ml) verteilt. Die wässrige Phase wurde mit Diethylether extrahiert (30 ml), die vereinigten organischen Phasen wurden mit Wasser und Natriumthiosulfatlösung (je 30 ml) gewaschen, mit Natriumsulfat getrocknet und i. Vak. eingeengt. Das Rohprodukt (1.00 g) wurde durch Flashchromatographie (100 g, 20 × 4.0 cm) mit Ethylacetat / Cyclohexan (1:3) gereinigt.

### Ausbeute: 259 mg (37 %), braunes Öl

*¹H-NMR (DMSO-d₆):* 0.86-0.99 (m, 15H); 1.27-1.57 (m, 7H); 1.88 (s, 6H); 2.53-2.60 (m, 2H); 3.33-3.36 (m, 2H); 4.62 (s, 2H); 6.99 (ddd, 1H, J = 7.9, 1.1, 1.0 Hz); 7.08 (ddd, 1 H, J = 8.1, 1,2, 1.0 Hz); 7.18-7.25 (m, 1H); 7.28-7.34 (m, 4H); 7.40 (dt, 1H, J = 8.1, 0.9 Hz); 7.58 (d, 1H, J = 7.3 Hz); 10.67 (s, 1 H).

*¹³C-NMR (DMSO-d₆):* 3.2; 7.3; 24.5; 32.1; 37.1; 37.4; 58.5; 64.5; 74.8; 111.3; 118.5; 121.3; 121.4; 126.1; 126.5; 127.2; 128.5; 133.3; 138.6; 139.4.

### Beispiel 2:

### [1-Phenyl-4-(1H-indol-3-ylmethoxymethyl)cyclohexyl]dimethylamin (Eines von zwei möglichen Diastereomeren)

Eine Aufschlämmung von Benzyltrimethylammoniumfluorid-Monohydrat (152 mg, 0.9 mmol) in wasserfreiem Tetrahydrofuran (20 ml) wurde mit aktiviertem Molsieb 4 Å (2.00 g) versetzt und 1 h bei Raumtemperatur gerührt. Anschließend wurde zu dieser Suspension eine Lösung von von **Beispiel 1** (140 mg, 0.3 mmol) in wasserfreiem Tetrahydrofuran (7 ml) gegeben und die Mischung 1 h unter Rückfluss gerührt. Das Reaktionsgemisch wurde danach filtriert, das Filtrat i. Vak. eingeengt und der Rückstand durch Flashchromatographie (10 g, 20 × 1.5 cm) mit Ethylacetat 1 Methanol (95:5) gereinigt.

### Ausbeute: 34 mg (31 %), Öl

*¹H-NMR (DMSO-d₆):* 1.48-1.78 (m, 7H); 2.03 (s, 6H); 2.47 (br s, 2H); 3.45 (d, 2H, J = 6.7 Hz); 4.74 (s, 2H); 7.10-.Z.40 (m, 9H); 7.74 (d, 1H, J = 7.7 Hz); 8.17 (br s, 1H).

*¹³C-NMR (DMSO-d₆):* 24.2; 30.1; 34.8; 37.5; 55.1; 65.0; 72.6; 111.2; 113.3; 113.9; 119.3; 119.6; 122.1; 124.2; 126.9; 127.2; 127.7; 128.1; 136.5.

### Beispiel 3:

### 1-Phenyl-4-(((2-(tert-butyldimethylsilyl)-5-cyano-1H-indol-3-yl)methoxy)methyl)-N,N-dimethylcyclohexanamin (Eines von zwei möglichen Diastereomeren)

Eine Mischung von **Ain-02** (350 mg, 0.9 mmol), 4-Amino-3-iodbenzonitril **(lan-02)** (268 mg, 1.1 mmol), [1,3-Bis-(2,6-diisopropylphenyl)imidazol-2-yliden]-(3-chlorpyridyl)palladium(II)-chlorid (PEPPSI, 122 mg, 0.18 mmol) und Natriumcarbonat (477 mg, 4.5 mmol) in wasserfreiem *N,N*-Dimethylformamid (10 ml) wurde 24 h bei 100 °C gerührt. Anschließend wurde das Reaktionsgemisch i. Vak. eingeengt, der Rückstand wiederholt mit Toluol versetzt und jeweils wieder eingeengt. Der Rückstand wurde zwischen Wasser und Diethylether (je 10 ml) verteilt. Die wässrige Phase wurde mit Diethylether extrahiert (3 × 10 ml) und die vereinigten organischen Phasen wurden mit Wasser und Natriumthiosulfatlösung (je 20 ml) gewaschen. Die organische Phase wurde mit Natriumsulfat getrocknet und i. Vak. eingeengt. Das Rohprodukt (400 mg) wurde durch Flashchromatographie (38 g, 20 × 2.5 cm) mit Ethylacetat / Cyclohexan (1:2) gereinigt.

### Ausbeute: 147 mg (32 %), gelblicher Feststoff

*Schmelzpunkt:* 72-75 °C

*¹H-NMR (DMSO-d₆):* 0.41 (s, 6H); 0.89 (s, 9H); 1.28-1.38 (m, 2H); 1.41-1.65 (m, 5H); 1.90 (s, 6H); 2.56 (d, 2H, J = 13.3 Hz); 3.38 (d, 2H, J = 6.4 Hz); 4.65 (s, 2H); 7.19-7.33 (m, 5H); 7.44 (d, 1 H, J = 8.4 Hz); 7.56 (d, 1H, J = 8.4 Hz); 8.10 (s, 1H); 11.24 (s, 1 H).

*¹H-NMR (CDCl₃):* 0.43 (s, 6H); 0.95 (s, 9H); 150-1.80 (m, 7H); 2.03 (s, 6H); 2.50-2.56 (m, 2H); 3.48 (d, 2H, J = 6.6 Hz); 4.72 (s, 2H); 7.22-7.38 (m, 5H); 7.41 (d, 2H, J = 1.0 Hz); 8.13 (s, 1 H); 8.29 (s, 1 H).

*¹³C-NMR (CDCl₃):* -5.2; 17.4; 24.9; 26.4; 26.9; 32.3; 37.1; 37.8; 59.4; 65.3; 76.0; 102.7; 111.6; 120.9; 123.6; 125.3; 125.6; 126.3; 126.9; 127.3; 128.7; 136.8; 140.0.

### Beispiel 4:

### 1-Phenyl-4-(((5-cyano-2-(triethylsilyl)-1H-indol-3-yl)methoxy)methyl)-N,N-dimethyl-cyclohexanamin (Eines von zwei möglichen Diastereomeren)

Eine Mischung von **Ain-01** (385 mg, 1 mmol), 4-Amino-3-iodbenzonitril **(lan-02)** (293 mg, 1.2 mmol), 1,3-Bis-(2,6-diisopropylphenyl)imidazol-2-yliden]-(3-chlorpyridyl)palladium(II)-chlorid (PEPPSI, 136 mg, 0.2 mmol) und Natriumcarbonat (530 mg, 5 mmol) in wasserfreiem *N,N*-Dimethylformamid (10 ml) wurde 18 h bei 100 °C gerührt. Das Lösungsmittel wurde danach i. Vak. entfernt und der Rückstand in Wasser und Diethylether (je 30 ml) verteilt. Die wässrige Phase wurde mit Diethylether (10 ml) extrahiert, die vereinigten organischen Phasen wurden mit Wasser und Natriumthiosulfatlösung (je 30 ml) gewaschen, mit Natriumsulfat getrocknet und i. Vak. eingeengt. Das Rohprodukt (500 mg) wurde durch Flashchromatographie (18 g, 20 × 2.0 cm) mit Ethylacetat / Cyclohexan (1:3) gereinigt.

### Ausbeute: 328 mg (65 %), farbloser Feststoff

*Schmelzpunkt: 48-52 °C*

*¹H-NMR (CDCl₃):* 0.89-0.97 (m, 6H); 1.00-1.06 (m, 9H); 1.47-1.80 (m, 7H); 2.02 (s, 6H); 2.49-2.56 (m, 2H); 3.47 (d, 2H, J = 6.7 Hz); 4.72 (s, 2H); 7.22-7.38 (m, 5H); 7.41 (d, 2H, J = 1.0 Hz); 8.11 (s, 1 H); 8.31 (s, 1 H).

*¹³C-NMR (CDCl₃):* 3.7; 7.3; 24.9; 26.9; 32.3; 37.1; 37.8; 59.4; 65.1; 75.8; 102.6; 111.6; 120.9; 123.4; 125.2; 125.4; 126.3; 126.9; 127.3; 128.7; 136.6; 139.5; 140.0.

### Beispiel 5:

### 1-Phenyl-4-(((5-cyano-1H-indol-3-yl)methoxy)methyl)-N,N-dimethylcyctohexanamin (Eines von zwei möglichen Diastereomeren)

Eine Aufschlämmung von Benzyltrimethylammoniumfluorid-Monohydrat (218 mg, 1.29 mmol) in wasserfreiem Tetrahydrofuran (20 ml) wurde mit aktiviertem Molsieb 4 Å (2.00 g) versetzt und 1 h bei Raumtemperatur gerührt. Anschließend wurde zu dieser Suspension eine Lösung von **Beispiel 4** (218 mg, 0.43 mmol) in wasserfreiem Tetrahydrofuran (10 ml) gegeben und die Mischung 1 h unter Rückfluss gerührt. Das Reaktionsgemisch wurde danach filtriert, das Filtrat i. Vak. eingeengt und der Rückstand durch Flashchromatographie (5 g, 15 × 0.9 cm) mit Ethylacetat / Methanol (95:5) gereinigt.

### Ausbeute: 98 mg (59 %), Öl

*¹H-NMR (CDCl₃):* 1.50-1.67 (m, 6H); 1.70-1.80 (br s, 1H); 2.02 (s, 6H); 2.42-2.51 (m, 2H); 3.45 (d, 2H, J = 6.7 Hz); 4.70 (s, 2H); 7.20-7.43 (m, 8H); 8.08 (s, 1 H); 9.01 (s, 1 H).

*¹³C-NMR (CDCl₃):* 24.7; 32.0; 36.8; 37.7; 59.6; 64.8; 75.2; 102.7; 112.0; 114.5; 120.8; 124.8; 125.1; 125.2; 125.5; 126.4; 126.9; 127.4; 130.2; 138.2; 139.0.

### Beispiel 6:

### 1-Phenyl-4-(((2-(tert-butyldimethytsilyl)-5-trifluormethyl-1H-indol-3-yl)methoxy)methyl)-N,N-dimethylcyclohexanamin (Eines von zwei möglichen Diastereomeren)

Eine Mischung von **Ain-02** (385 mg, 1 mmol), 4-Amino-3-iodbenzotrifluorid **(lan-03)** (344 mg, 1.2 mmol), [1,3-Bis-(2,6-diisopropylphenyl)imidazol-2-yliden]-(3-chlorpyridyl)palladium(II)-chlorid (PEPPSI, 136 mg, 0.2 mmol) und Natriumcarbonat (530 mg, 5 mmol) in wasserfreiem *N,N*-Dimethylformamid (10 ml) wurde 24 h bei 100 °C gerührt. Anschließend wurde das Lösungsmittel i. Vak. entfernt und der Rückstand zwischen Wasser und Diethylether (je 10 ml) verteilt. Die wässrige Phase wurde mit Diethylether extrahiert (3 × 10 ml) und die vereinigten organischen Phasen wurden mit Wasser und Natriumthiosulfatlösung (je 20 ml) gewaschen. Die organische Phase wurde mit Natriumsulfat getrocknet, i. Vak. eingeengt und das Rohprodukt durch Flashchromatographie (38 g, 20 × 2.5 cm) mit Ethylacetat / Cyclohexan (1:4) gereinigt.

### Ausbeute: 326 mg (60 %)

*Schmelzpunkt: 48-50 °C*

*¹H-NMR (DMSO-d₆):* 0.41 (s, 6H); 0.90 (s, 9H); 1.28-1.66 (m, 7H); 1.89 (s, 6H); 2.55 (d, 2H, J = 13.0 Hz); 3.37 (d, 2H, J = 6.2 Hz); 4.68 (s, 2H); 7.18-7.24 (m, 1H); 7.28-7.35 (m, 4H); 7.39 (dd, 1 H, J = 8.5, 1.3 Hz); 7.58 (d, 1 H, J = 8.5 Hz); 7.97 (s, 1H); 11.10 (s, 1 H).

*¹³C-NMR (CDCl₃):* -5-2; 17.7; 24.9; 26.4; 32.3; 37.1; 37.8; 59.4; 65.5; 75.7; 110.9; 117.7 (q, J = 4.6 Hz); 119.3 (q, J = 3.6 Hz); 121.9; 122.2; 123.8; 126.3; 126.9; 127.3; 128.3; 136.0; 119.5, 139.7.

### Beispiel 7:

### 1-Phenyl-4-(((5-trifluormethyl-2-(triethylsilyl)-1H-indol-3-yl)methoxy)methyl)-N,N-dimethylcyclohexanamin (Eines von zwei möglichen Diastereomeren)

Eine Mischung von **Ain-01** (385 mg, 1 mmol), 4-Amino-2-iodbenzotrifluorid **(lan-03)** (344 mg, 1.2 mmol), [1,3-Bis-(2,6-diisopropylphenyl)imidazol-2-yliden]-(3-chlorpyridyl)palladium(II)-chlorid (PEPPSI, 136 mg, 0.2 mmol) und Natriumcarbonat (530 mg, 5 mmol) in wasserfreiem *N,N*-Dimethylformamid (10 ml) wurde bei 100 °C 18 h gerührt. Das Lösungsmittel wurde i. Vak. entfernt und der Rückstand in Wasser und Diethylether (je 30 ml) verteilt. Die wässrige Phase wurde mit Diethylether extrahiert (10 ml), die vereinigten organischen Phasen wurden mit Wasser und Natriumthiosulfatlösung (je 30 ml) gewaschen, mit Natriumsulfat getrocknet und i. Vak. eingeengt. Das Rohprodukt (600 mg) wurde durch Flashchromatographie (10 g, 20 × 1.5 cm) mit Ethylacetat / Cyclohexan (1:9) gereinigt.

### Ausbeute: 329 mg (60 %), Öl

*¹H-NMR (DMSO-d₆):* 0.85-0.98 (m, 15H); 1.25-1.67 (m, 7H); 1.88 (s, 6H); 2.57 (br s, 2H); 3.34 (br d, 2H, J = 6.2 Hz); 4.68 (s, 2H); 7.19-7.41 (m, 6H); 7.58 (d, 1 H, J = 8.5 Hz); 7.97 (s, 1H); 11.10 (s, 1H).

*¹³C-NMR (DMSO-d₆):* 3.1; 7.2; 24.5; 26.3; 28.9; 32.0; 37.0; 37.4; 58.5; 64.4; 74.5; 112.0; 116.4; 117.7; 119.2; 119.6; 122.6; 124.3; 126.1; 126.5; 127.2: 127.6; 136.1; 139.3; 140.1.

### Beispiel 8:

### 1-Phenyl-4-(((5-trifluormethyl-1H-indol-3-yl)methoxy)methyl)-N,N-dimethylcyclohexanamin (Eines von zwei möglichen Diastereomeren)

Eine Aufschlämmung von Benzyltrimethylammoniumfluorid-Monohydrat (237 mg, 1.4 mmol) in wasserfreiem Tetrahydrofuran (20 ml) wurde mit aktiviertem Molsieb 4 Å (2.00 g) versetzt und 1 h bei Raumtemperatur gerührt. Anschließend wurde zu dieser Suspension eine Lösung von **Beispiel 7** (250 mg, 0.46 mmol) in wasserfreiem Tetrahydrofuran (15 ml) gegeben und die Mischung 1 h unter Rückfluss gerührt. Das Reaktionsgemisch wurde danach filtriert, das Filtrat i. Vak. eingeengt und der Rückstand durch Flashchromatographie (10 g, 20 × 1.5 cm) mit Ethylacetat / Methanol (95:5) gereinigt.

### Ausbeute: 70 mg (35 %), Öl

*¹H-NMR (CDCl₃):* 1.50-1.80 (m, 7H); 2.02 (s, 6H); 2.40-2.50 (m, 2H); 3.47 (d, 2H, J = 6.8 Hz); 4.74 (s, 2H); 7.13-7.45 (m, 8H); 8.05 (s, 1H); 8.69 (s, 1H).

*¹³C-NMR (CDCl₃):* 24.7; 32.0; 36.8; 37.7; 59.7; 64.8; 65.0; 74.9; 111.4; 114.6; 117.3; 117.4; 118.9; 122.1 (q, J = 32 Hz); 124.0; 125.0; 126.4; 126.5; 126.7; 127.0; 127.4; 129.7; 137.8; 139.0.

### Beispiel 9:

### 1-Phenyl-4-(((5-fluor-2-(triethylsilyl)-1H-indol-3-yl)methoxy)methyl)-N,N-dimethylcyclohexanamin (Eines von zwei möglichen Diastereomeren)

Eine Mischung von **Ain-01** (485 mg, 1.26 mmol), 4-Fluor-2-iodanilin **(lan-04)** (357 mg, 1.51 mmol), [1,3-Bis-(2,6-diisopropylphenyl)imidazol-2-yliden]-(3-chlorpyridyl)palladium(II)-chlorid (PEPPSI, 171 mg, 0.25 mmol) und Natriumcarbonat (668 mg, 6.3 mmol) in wasserfreiem *N,N*-Dimethylformamid (10 ml) wurde 18.h bei 100 °C gerührt. Das Lösungsmittel wurde i. Vak. entfernt und der Rückstand in Wasser und Diethylether (je 50 ml) verteilt. Die wässrige Phase wurde mit Diethylether extrahiert (30 ml), die vereinigten organischen Phasen wurden mit Wasser und Natriumthiosulfatlösung (je 30 ml) gewaschen, mit Natriumsulfat getrocknet und i. Vak. eingeengt. Das Rohprodukt (600 mg) wurde durch Flashchromatographie (10 g, 20 × 1.5 cm) mit Ethylacetat / Cyclohexan (1:9) gereinigt.

### Ausbeute: 285 mg (46 %), Öl

*¹H-NMR (DMSO-d₆):* 0.84-0.99 (m, 15H); 1.27-1.65 (m, 7H); 1.89 (s, 6H); 2.56 (d, 2H, J = 12.6 Hz); 3.30-3.35 (2H, unter dem HDO-Signal); 4.59 (s, 2H); 6.93 (dt, 1H, J = 9.2, 2.5 Hz); 7.18-7.41 (m, 7H); 10.77 (s, 1H).

*¹³C-NMR (DMSO-d₆):* 3.1; 7.2; 24.5; 26.3; 28.9; 32.0; 37.0; 37.4; 58.5; 64.4; 74.8; 102.9 (d, J = 23 Hz); 109.6 (d, J = 26 Hz); 112.2 (d, J = 11 Hz); 121.5 (d, J = 5 Hz); 126.1; 126.5; 127.2; 128.7 (d, J = 10 Hz); 135.3; 135.8; 139.4; 156.7 (d, J = 232 Hz).

### Beispiel 10:

### 1-Phenyl-4-(((5-fluor-1H-indol-3-yl)methoxy)methyl)-N,N-dimethyl-cyclohexanamin (Eines von zwei möglichen Diastereomeren)

Eine Aufschlämmung von Benzyltrimethylammoniumfluorid-Monohydrat (237 mg, 1.4 mmol) in wasserfreiem Tetrahydrofuran (20 ml) wurde mit aktiviertem Molsieb 4 Å (2.00 g) versetzt und 1 h bei Raumtemperatur gerührt. Anschließend wurde zu dieser Suspension eine Lösung von **Beispiel 9** (230 mg, 0.46 mmol) in wasserfreiem Tetrahydrofuran (15 ml) gegeben und die Mischung 1 h unter Rückfluss gerührt. Das Reaktionsgemisch wurde danach filtriert, das Filtrat i. Vak. eingeengt und der Rückstand durch Flashchromatographie (10 g, 20 × 1.5 cm) mit Ethylacetat / Methanol (95:5) gereinigt.

### Ausbeute: 91 mg (66 %), Öl

*¹H-NMR (CDCl₃):* 1.42-1.80 (m, 7H); 2.03 (s, 6H); 2.47 (br d, 2H, J = 6.9 Hz); 3.44 (d, 2H, J = 6.7 Hz); 4.67 (s, 2H); 6.95 (dt, 1 H, J = 9.0, 2.4 Hz); 7.21-7.40 (m, 8H); 8.27 (s, 1 H).

*¹³C-NMR (CDCl₃):* 24.7; 32.0; 36.9; 37.8; 65.1; 74.9; 104.4 (d, J = 23 Hz); 110.6 (d, J = 26 Hz); 111.6 (d, J = 10 Hz); 114.0; 125.3; 126.5; 127.0; 127.5; 127.7 (d, J = 10 Hz); 133.0; 157.9 (d, J = 235 Hz).

### Beispiel 11:

### 1-Phenyl-N,N-dimethyl-4-(((2-(triethylsilyl)-1H-pyrrolo[2,3-b]pyridin-3-yl)methoxy)-methyl)cyclohexanamin (Eines von zwei möglichen Diastereomeren)

Eine Mischung von **Ain-01** (385 mg, 1 mmol), 2-Amino-3-iodpyridin **(lan-05)** (264 mg, 1.2 mmol), 1,3-Bis-(2,6-diisopropylphenyl)imidazol-2-yliden]-(3-chlorpyridyl)palladium(II)-chlorid (PEPPSI, 136 mg, 0.2 mmol) und Natriumcarbonat (530 mg, 5 mmol) in wasserfreiem *N,N*-Dimethylformamid (10 ml) wurde 18 h bei 100 °C gerührt. Das Lösungsmittel wurde anschließend i. Vak. entfernt und der Rückstand in Wasser und Diethylether (je 30 ml) verteilt. Die wässrige Phase wurde mit Diethylether extrahiert (10 ml), die vereinigten organischen Phasen wurden mit Wasser und Natriumthiosulfatlösung (je 30 ml) gewaschen, mit Natriumsulfat getrocknet und i. Vak. eingeengt. Das Rohprodukt (560 mg) wurde durch Flashchromatographie (18 g, 20 × 2.0 cm) mit Ethylacetat / Cyclohexan (1:2) gereinigt.

### Ausbeute: 225 mg (47 %), Öl

*¹H-NMR (CDCl₃):* 0.88-0.96 (m, 15H); 1.45-1.78 (m, 7H); 2.02 (s, 6H); 2.49-2.56 (m, 2H); 3.45 (d, 2H, J = 6.5 Hz); 4.72 (s, 2H); 7.05-7.09 (m, 1 H); 7.22-7.37 (m, 5H); 8.03-8.06 (m, 1H); 8.27-8.31 (m, 1 H); 8.68 (br s, 1H).

*¹³C-NMR (CDCl₃):* 3.8; 7.4; 24.9; 26.9; 32.3; 37.2; 37.8; 59.4; 65.6; 75.5; 115.8; 121.0; 121.3; 126.3; 126.9; 127.3; 127.8; 134.3; 139.6; 143.8; 150.6.

### Beispiel 12:

### 4-(((1H-Pyrrolo[2,3-b]pyridin-3-yl)methoxy)methyl)-1-phenyl-N,N-dimethylcyclohexan-amin (Eines von zwei möglichen Diastereomeren)

Eine Aufschlämmung von Benzyltrimethylammoniumfluorid-Monohydrat (191 mg, 1.13 mmol) in wasserfreiem Tetrahydrofuran (20 ml) wurde mit aktiviertem Molsieb 4 Å (2.00 g) versetzt und 1 h bei Raumtemperatur gerührt. Anschließend wurde zu dieser Suspension eine Lösung von **Beispiel 11** (180 mg, 0.37 mmol) in wasserfreiem Tetrahydrofuran (15 ml) gegeben und 1 h unter Rückfluss gerührt. Das Reaktionsgemisch wurde danach filtriert, das Filtrat i. Vak. eingeengt und der Rückstand durch Flashchromatographie (5 g, 15 × 0.9 cm) mit Ethylacetat / Methanol (9:1) gereinigt.

### Ausbeute: 36 mg (27 %), Öl

*¹H-NMR (CDCl₃):* 1.45-1.70 (m, 8H); 2.02 (s, 6H); 2.45-2.60 (m, 1H); 3.44 (d, 2H, J = 6.6 Hz); 4.72 (s, 2H); 7.12 (dd, 1H, J = 7.8, 4.8 Hz); 7.21-7.39 (m, 6H); 8.07 (dd, 1H, J = 7.8, 1.5 Hz); 8.34 (dd, 1H, J = 4.8, 1.5 Hz); 10.49 (s, 1 H).

*¹³C-NMR (CDCl₃):* 24.8; 32.1; 37.0; 37.8; 45.3; 59.6; 65.3; 75.0; 112.2; 115.5; 115.8; 119.9; 124.0; 126.4; 126.9; 127.4; 127.7; 128.0; 128.2; 129.1; 139.4; 143.0; 143.1; 149.2.

### Beispiel 13:

### 1-Phenyl-4-(((2-(tert-butyldimethylsilyl)-1 H-pyrrolo[3,2-c]pyridin-3-yl)methoxy)methyl)-N,N-dimethylcyclohexanamin (Eines von zwei möglichen Diastereomeren)

Eine Mischung von **Ain-02** (385 mg, 1 mmol), 4-Amino-3-iodpyridin **(lan-06)** (264 mg, 1.2 mmol), [1,3-Bis-(2,6-diisopropylphenyl)imidazol-2-yliden]-(3-chlorpyridyl)palladium(II)-chlorid (PEPPSI, 136 mg, 0.2 mmol) und Natriumcarbonat (530 mg, 5 mmol) in wasserfreiem *N,N*-Dimethylformamid (10 ml) wurde 24 h bei 120 °C gerührt. Anschließend wurde das Lösungsmittel i. Vak. entfernt und der Rückstand zwischen Wasser und Diethylether (je 10 ml) verteilt. Die wässrige Phase wurde mit Diethylether extrahiert (3 × 10 ml) und die vereinigten organischen Phasen wurden mit Wasser und Natriumthiosulfatlösung (je 20 ml) gewaschen. Die organische Phase wurde mit Natriumsulfat getrocknet und i. Vak. eingeengt. Das Rohprodukt (600 mg) wurde durch Flashchromatographie (38 g, 20 × 2.5 cm) mit Ethylacetat / Methanol (1:4) gereinigt.

### Ausbeute: 185 mg (39 %), farbloser Feststoff

*Schmelzpunkt:* 95-100 °C

*¹H-NMR (DMSO-d₆):* 0.41 (s, 6H); 1.17 (s, 9H); 1.30-1.57 (m, 6H); 1.57-1.66 (br s, 1 H); 1.92 (s, 6H); 2.56 (d, 2H, J = 12.9 Hz); 3.38 (d, 2H, J = 6.3 Hz); 4.68 (s, 2H); 7.20-7.26 (br s, 1H); 7.31-7.38 (m, 5H); 8.15 (d, 1 H, J = 5.7 Hz); 8.90 (s, 1 H), 11.08 (s, 1 H).

*¹³C-NMR (DMSO-d₆):* -5.2; 16.9; 24.4; 26.3; 31.8; 36.8; 37.4; 64.6; 75.0; 106.5; 11.8; 125.3; 126.2; 126.6; 127.2; 135.1; 140.0; 141.7; 142.2.

### Beispiel 14:

### 1-Phenyl-N,N-dimethyl-4-(((2-(triethylsilyl)-1H-pyrrolo[3,2-c]pyridin-3-yl)methoxy)-methyl)cyclohexanamin (Eines von zwei möglichen Diastereomeren)

Eine Mischung von **Ain-01** (385 mg, 1 mmol), 4-Amino-2-iodpyridin **(lan-06)** (264 mg, 1.2 mmol), 1,3-Bis-(2,6-diisopropylphenyl)imidazol-2-yliden]-(3-chlorpyridyl)palladium(II)-chlorid (PEPPSI, 136 mg, 0.2 mmol) und Natriumcarbonat (530 mg, 5 mmol) in wasserfreiem *N,N*-Dimethylformamid (10 ml) wurde 18 h bei 120 °C gerührt. Das Lösungsmittel wurde anschließend i. Vak. entfernt und der Rückstand in Wasser und Diethylether (je 30 ml) verteilt. Die wässrige Phase wurde mit Diethylether extrahiert (10 ml), die vereinigten organischen Phasen wurden mit Wasser und Natriumthiosulfatlösung (je 30 ml) gewaschen, mit Natriumsulfat getrocknet und i. Vak. eingeengt. Das Rohprodukt (560 mg) wurde durch Flashchromatographie (10 g, 20 × 1.5 cm) mit Ethylacetat / Methanol (4:1) gereinigt.

### Ausbeute: 262 mg (55 %), Öl

*¹H-NMR (CDCl₃):* 0.87-1.06 (m, 15H); 1.50-1.80 (m, 8H); 2.04 (s, 6H); 2.46-2.57 (m, 2H); 3.48 (d, 2H, J = 6.7 Hz); 4.77 (s, 2H); 7.21-7.38 (m, 5H); 8.28 (d, 1 H, J = 5.8 Hz); 8.46 (s, 1 H); 9.07 (s, 1 H).

*¹³C-NMR (CDCl₃):* 3.7; 7.3; 14.2; 24.8; 31.9; 36.8; 37.8; 65.3; 75.4; 106.1; 122.9; 125.8; 126.5; 127.0; 127.4; 135.1; 141.0; 142.1; 143.1.

### Beispiel 15:

### 4-(((1H-pyrrolo[3,2-c]pyridin-3-yl)methoxy)methyl)-1-phenyl-N,N-dimethylcyclohexan-amin (Eines von zwei möglichen Diastereomeren)

Eine Aufschlämmung von Benzyltrimethylammoniumfluorid-Monohydrat (157 mg, 0.93 mmol) in wasserfreiem Tetrahydrofuran (20 ml) wurde mit aktiviertem Molsieb 4 Å (1.20 g) versetzt und 1 h bei Raumtemperatur gerührt. Anschließend wurde zu dieser Suspension eine Lösung von **Beispiel 14** (150 mg, 0.31 mmol) in wasserfreiem Tetrahydrofuran (15 ml) gegeben und die Mischung 1 h unter Rückfluss gerührt. Das Reaktionsgemisch wurde danach filtriert, das Filtrat i. Vak. eingeengt und der Rückstand durch Flashchromatographie (5 g, 15 × 0.9 cm) mit Ethylacetat / Methanol (4:1) gereinigt.

### Ausbeute: 78 mg (70 %), Öl

*¹H-NMR (CDCl₃):* 1.50-1.68 (m, 6H); 1.73 (br s, 1 H); 1.99 (s, 6H); 2.40-2.50 (m, 2H); 3.47 (d, 2H, J = 7.0 Hz); 4.75 (s, 2H); 7.17-7.37 (m, 7H); 8.3 (d, 1 H, J = 5.9 *Hz); 9.07 (d, 1H, J* = *1.2 Hz); 10.0 (br s, 1H).*

*¹³C-NMR (CDCl₃):* 24.7; 32.0; 36.9; 37.7; 59.6; 64.9; 75.1; 106.7; 113.6; 124.1; 124.5; 126.4; 126.9; 127.4; 139.2; 140.5; 140.7; 142.6.

### Beispiel 16:

### 1-(3-(((4-(Dimethylamino)-4-phenylcyclohexyl)methoxy)methyl)-5-fluor-2-(triethylsilyl)-1H-indol-1-yl)ethanon (Eines von zwei möglichen Diastereomeren)

Eine Mischung von **Ain-01** (578 mg, 1.5 mmol), [1,3-Bis-(2,6-diisopropylphenyl)imidazol-2-yliden]-(3-chlorpyridyl)palladium(II)-chlorid (PEPPSI, 204 mg, 0.3 mmol), N-(4-fluor-2-iodphenyl)acetamide **(lan-8)** (521 mg, 1.9 mmol) und Natriumcarbonat (793 mg, 7.5 mmol) wurde 30 min an der Ölpumpe evakuiert. Anschließend wurde über einen Schlenk-Aufsatz wasserfreies *N,N*-Dimethylformamid (8 ml), dass zuvor 1 h mit Argon gespült wurde, zugesetzt. Das Reaktionsgemisch wurde danach 18 h bei 100 °C gerührt und anschließend i. Vak. eingeengt. Der Rückstand wurde wiederholt mit Toluol versetzt, jeweils wieder i. Vak. eingeengt und dann zwischen Wasser und Ethylacetat (jeweils 20 ml) verteilt. Die Phasen wurden getrennt und die wässrige Phase wurde mit Ethylacetat (3 × 30 ml) extrahiert. Die vereinigten organischen Phasen wurden mit 1 *M* Natriumthiosulfatlösung (30 ml) und gesättigter Natriumchloridlösung (50 ml) gewaschen. Die vereinigten organischen Phasen wurden mit Natriumsulfat getrocknet und i. Vak. eingeengt. Das Rohprodukt (1.17 g) wurde durch Flashchromatographie (100 g, 20 × 3.7 cm) mit Ethylacetat / Methanol (9:1) gereinigt. Das gewonnene Substanzgemisch wurde erneut durch Flashchromatographie (85 g, 20 × 3.4 cm) mit Cyclohexan 1 Ethylacetat (4:1) gereinigt.

### Ausbeute: 365 mg (45 %) gelblicher Feststoff

*Schmelzpunkt:* 90-92 °C

*¹H-NMR (DMSO-d₆):* 0.84-0.99 (m, 15H); 1.20-1.70 (m, 7H); 1.89 (s, 6H); 2.57 (br d, 2H, J = 13.0 Hz); 2.84 (s, 3H); 3.37 (d, 2H, J = 6.1 Hz); 4.60 (s, 2H); 7.16-7.40 (m, 6H); 7.48 (dd,1 H, J = 2.6, 9.1 Hz); 7.83 (dd, 1 H, J = 4.2, 9.2 Hz).

*¹³C-NMR (DMSO-d₆):* 5.1; 7.9; 24.4; 26.1; 31.9; 36.9; 37.4; 58.5; 63.4; 75.2; 105.1 (d, J = 23 Hz); 112.3 (d, J = 25 Hz); 115.6 (d, J = 9 Hz); 126.1; 126.5; 127.2; 130.9; 133.1; 133.4 (d, J = 9 Hz); 138.4; 139.3; 158.3 (d, J = 238 Hz); 170.2.

### Beispiel 17:

### 4-(((5-Fluor-1-(methylsulfonyl)-2-(triethylsilyl)-1H-indol-3-yl)methoxy)methyl)-N,N-dimethyl-1-phenylcyclohexanamin (Eines von zwei möglichen Diastereomeren)

Eine Mischung von **Ain-01** (578 mg, 1.5 mmol), [1,3-Bis-(2,6-diisopropylphenyl)imidazol-2-yliden]-(3-chlorpyridyl)palladium(II)-chlorid (PEPPSI, 204 mg, 0.3 mmol), *N*-(4-Fluor-2-iodphenyl)methanesulfonamide **(lan-9)** (590 mg, 1.9 mmol) und Natriumcarbonat (793 mg, 7.5 mmol) wurde 30 min an der Ölpumpe evakuiert. Anschließend wurde über einen Schlenk-Aufsatz wasserfreies *N,N*-Dimethylformamid (8 ml), das zuvor 1 h mit Argon gespült wurde, zugesetzt. Das Reaktionsgemisch wurde danach 18 h bei 100 °C gerührt und anschließend i. Vak. eingeengt. Der Rückstand wurde wiederholt mit Toluol versetzt, jeweils wieder i. Vak. eingeengt und dann zwischen Wasser und Ethylacetat (jeweils 20 ml) verteilt. Die Phasen wurden getrennt und die wässrige Phase wurde mit Ethylacetat (3 × 30 ml) extrahiert. Die vereinigten organischen Phasen wurden mit 1 *M* Natriumthiosulfatlösung (30 ml) und gesättigter Natriumchloridlösung (50 ml) gewaschen, mit Natriumsulfat getrocknet und i. Vak. eingeengt. Das Rohprodukt (1.06 g) wurde durch Flashchromatographie (110 g, 20 × 3.7 cm) mit Cyclohexan / Ethylacetat (4:1) und anschließend mit Cyclohexan / Ethylacetat (2:1) gereinigt.

### Ausbeute: 504 mg (58 %), braunes Öl

*¹H-NMR (DMSO-d₆):* 0.86-1.06 (m, 15H); 1.10-1.70 (m, 7H); 1.89 (s, 6H); 2.56 (br d, 2H, J = 12.4 Hz); 3.09 (s, 3H); 3.40 (d, 2H, J = 6.1 Hz); 4.61 (s, 2H); 7.20-7.34 (m, 6H); 7.53 (dd, 1 H, J = 2.5, 9.1 Hz); 7.90 (dd, 1H, J = 4.4, 9.1 Hz).

*¹³C-NMR (DMSO-d₆):* 4.4; 7.5; 24.4; 26.2; 31.9; 36.8; 37.4; 58.5; 63.5; 75.3; 105.5 (d, J = 24 Hz); 113.4 (d, J = 26 Hz); 115.7 (d, J = 10 Hz); 122.5; 126.1; 126.5; 127.1; 132.9 (d, J = 10 Hz); 133.6; 135.1; 139.1; 139.3; 159.1 (d, J = 239 Hz).

### Beispiel 18:

### 1-Phenyl-4-[2-(tert-butyldimethylsilanyl)-1H-indol-3-ylmethoxymethyljcyclohexyl-dimethylamin (Eines von zwei möglichen Diastereomeren)

Eine Mischung von **Ain-02** (965 mg, 2.5 mmol), 2-lodanilin **(lan-01)** (655 mg, 3 mmol), Palladium(II)-acetat (110 mg, 0.5 mmol), Tetra-*n*-butylammoniumchlorid (655 mg, 2.5 mmol), Triphenylphosphin (260 mg, 1 mmol) und Natriumcarbonat (1.32 g, 12.5 mmol) in wasserfreiem *N,N*-Dimethylformamid (15 ml) wurde 18 h bei 100 °C gerührt. Anschließend wurde das Lösungsmittel i. Vak. entfernt und der Rückstand in Wasser und Diethylether (je 50 ml) verteilt. Die wässrige Phase wurde mit Diethylether (30 ml) und die vereinigten organischen Phasen wurden mit Wasser und Natriumthiosulfatlösung (je 30 ml) gewaschen. Die organische Phase wurde mit Natriumsulfat getrocknet und i. Vak. eingeengt. Das Rohprodukt (247 mg) wurde durch Flashchromatographie (100 g, 20 × 4.0 cm) mit Ethylacetat / Cyclohexan (1:2) gereinigt.

### Ausbeute: 930 mg (78 %), braunes Öl

*¹H-NMR (DMSO-d₆):* 0.39 (s, 6H); 0.89 (s, 9H); 1.25-1.60 (m, 7H); 1.89 (s, 6H); 2.56 (d, 2H, J = 12.2 Hz); 3.34 (s, 2H); 4.62 (s, 2H); 6.96-7.02 (m,1H); 7.09 (dt, 1H, J = 6.9, 1.0 Hz); 7.17-7.26 (m, 2H); 7.27-7.38 (m, 3H); 7.41 (d, 1H, J = 8.1 Hz); 7.59 (d, 1H, J = 7.8 Hz); 10.7 (s, 1H).

*¹³C-NMR (DMSO-d₆):* -5.0; 17.0; 24.5; 26.3; 26.4; 32.1; 37.4; 58.5; 64.8; 75.0; 111.2; 118.5; 118.7; 121.5; 121.6; 126.1; 126.5; 127.2; 128.5; 133.6; 138.6.

### Beispiel 19:

### 1-Phenyl-4-(((2-(tert-butyldimethylsilyl)-5-fluor-1H-indol-3-yl)methoxy)methyl)-N,N-dimethylcyclohexanamin (Eines von zwei möglichen Diastereomeren)

Eine Mischung von **Ain-02** (385 mg, 1 mmol), 4-Fluor-2-iodanilin **(lan-04)** (284 mg, 1.2 mmol), [1,3-Bis-(2,6-diisopropylphenyl)imidazol-2-yliden]-(3-chlorpyridyl)palladium(II)-chlorid (PEPPSI, 136 mg, 0.2 mmol) und Natriumcarbonat (530 mg, 5 mmol) in wasserfreiem *N,N*-Dimethylformamid (10 ml) wurde 24 h bei 100 °C gerührt. Anschließend wurde das Lösungsmittel i. Vak. entfernt und der Rückstand zwischen Wasser und Diethylether (je 10 ml) verteilt. Die wässrige Phase wurde mit Diethylether extrahiert (10 ml) und die vereinigten organischen Phasen wurden mit Wasser und Natriumthiosulfatlösung (je 20 ml) gewaschen. Die organische Phase wurde mit Natriumsulfat getrocknet und i. Vak. eingeengt. Das Rohprodukt (500 mg) wurde durch Flashchromatographie (38 g, 20 × 2.5 cm) mit Ethylacetat / Cyclohexan (1:2) gereinigt.

### Ausbeute: 370 mg (75 %), braunes Öl

*¹H-NMR (DMSO-d₆):* 0.39 (s, 6H); 0.89 (s, 9H); 1.30-1.60 (m, 7H); 1.92 (s, 6H); 2.57 (d, 2H, J = 12.8 Hz); 3.35 (d, 2H, J = 6.1 Hz); 4.59 (s, 2H); 6.94 (dt, 1H, J = 9.1, 2.5 Hz); 7.19-7.40 (m, 7H); 10.77 (s, 1H).

*¹³C-NMR (DMSO-d₆):* -5.1; 16.9; 24.5; 26.3; 32.0; 37.1; 37.4; 58.5; 64.7; 75.0; 103.0 (d, 1C, J = 23 Hz); 109.7 (d, J = 26 Hz); 112.1 (d, J = 10 Hz); 121.7 (d, J = 5 Hz); 126.1; 126.5; 127.2; 128.7 (d, J = 10 Hz); 135.3; 136.1; 139.4; 156.7 (d, J = 232 Hz).

### Beispiel 20:

### 1-Phenyl-4-(((2-(tert-butyldimethylsilyl)-1H-pyrrolo[2,3-b]pyridin-3-yl)methoxy)methyl)-N,N-dimethylcyclohexanamin (Eines von zwei möglichen Diastereomeren)

Eine Mischung von **Ain-02** (385 mg, 1 mmol), 2-Amino-3-iodpyridin **(lan-05)** (264 mg, 1.2 mmol), [1,3-Bis-(2,6-diisopropylphenyl)imidazol-2-yliden]-(3-chlorpyri¬dyl)palla¬dium(II)-chlorid (PEPPSI, 136 mg, 0.2 mmol) und Natriumcarbonat (530 mg, 5 mmol) in wasserfreiem N,N-Dimethylformamid (10 ml) wurde 24 h bei 100 °C gerührt. Anschließend wurde das Lösungsmittel i. Vak. entfernt und der Rückstand zwischen Wasser und Diethylether (je 10 ml) verteilt. Die wässrige Phase wurde mit Diethylether extrahiert (3 × 10 ml) und die vereinigten organischen Phasen wurden mit Wasser und Natriumthiosulfatlösung (je 20 ml) gewaschen. Die organische Phase wurde mit Natriumsulfat getrocknet und i. Vak. eingeengt. Das Rohprodukt (535 mg) wurde durch Flashchromatographie (38 g, 20 × 2.5 cm) mit Ethylacetat / Cyclohexan (1:2), Ethylacetat und Ethylacetat / Methanol (9:1) gereinigt.

### Ausbeute: 190 mg (40 %), bräunlicher Feststoff

*Schmelzpunkt:* 73-78 °C

*1H-NMR (DMSO-d6):* 0.41 (s, 6H); 0.89 (s, 9H); 1.28-1.63 (m, 7H); 1.90 (s, 6H); 2.56 (d, 2H, J = 12.6 Hz); 3.35 (d, 2H, J = 6.2 Hz); 4.62 (s, 2H); 7.05 (dd, 1H, J = 7.8, 4.6 Hz); 7.19-7.24 (m, 1 H); 7.28-7.35 (m, 4H); 7.99 (dd, 1H, J = 7.8, 1.0 Hz); 8.24 (dd, 1H, J = 4.6, 1.5 Hz); 11.31 (s, 1 H).

*¹³C-NMR (CDCl3):* -5.1; 17.4; 24.8; 26.4; 32.2; 37.1; 37.8; 65.8; 75.5; 115.8; 116.2; 121.1; 121.4; 126.9; 127.4; 127.9; 134.5; 143.9; 150.6.

### Beispiel 21: (nicht erfindungsgemäß)

### 4-(((5-Fluor-3-methyl-1H-indol-2-yl)methoxy)methyl)-N,N-dimethyl-1-phenylcyclohexan-amin (Eines von zwei möglichen Diastereomeren)

Eine Mischung von **Ain-12** (570 mg, 2 mmol), 4-Fluor-2-iodanilin **(lan-04)** (592 mg, 2.5 mmol), Palladium(II)-acetat (90 mg, 0.38 mmol), Tetra-n-butylammoniumchlorid (564 mg, 2 mmol), Triphenylphosphin (203 mg, 0.80 mmol) und Natriumcarbonat (1.05 g, 10 mmol) wurde 30 min an der Ölpumpe evakuiert. Anschließend wurde über einen Schlenk-Aufsatz wasserfreies *N,N*-Dimethylformamid (15 ml), das zuvor 1 h mit Argon gespült wurde, zugesetzt. Das Gemisch wurde anschließend 18 h bei 100 °C gerührt. Danach wurde das Reaktionsgemisch i. Vak. eingeengt. Der Rückstand wurde wiederholt mit Toluol versetzt, jeweils wieder i. Vak. eingeengt und anschließend zwischen Wasser und Ethylacetat (jeweils 20 ml) verteilt. Die Phasen wurden getrennt und die wässrige Phase wurde mit Ethylacetat (3 × 30 ml) extrahiert. Die vereinigten organischen Phasen wurden mit 1 *M* Natriumthiosulfatlösung (30 ml) und gesättigter Natriumchloridlösung (50 ml) gewaschen. Die vereinigten organischen Phasen wurden mit Natriumsulfat getrocknet und i. Vak. eingeengt. Das Rohprodukt (1.06 g) wurde durch MPLC [230 g, 46 × 3.6 cm, LiChroprep Si60 (15-25 µm)] mit Ethylacetat / Methanol (9:1) gereinigt.

### Ausbeute: 319 mg (40 %), braunes Öl

*¹H-NMR (DMSO-d₆):* 1.25-1.70 (m, 7H); 1.90 (s, 6H); 2.21 (s, 3H); 2.56 (br d, 2H, J = 11.9 Hz); 3.30 (d, 2H, J = 6.3 Hz); 4.55 (s, 2H); 6.88 (ddd, 1H, J = 9.5, 8.9, 2.6 Hz); 7.16-7.36 (m, 3H); 7.51-7.68 (m, 4H); 10.93 (s, 1 H).

*¹³C-NMR (DMSO-d₆):* 8.2; 24.4; 32.0; 36.9; 37.4; 58.5; 63.3; 74.8; 102.8 (d, J = 23 Hz); 108.1; 109.0 (d, J = 26 Hz); 111.8 (d, J = 10 Hz); 126.1; 126.5; 127.2; 128.6 (d, J = 12 Hz); 131.4; 131.9; 132.1; 132.2; 133.2; 133.9; 139.3; 156.5 (d, J = 231 Hz).

### Beispiel 22:

### {1-Benzyl-4-[2-(tert-butyldimethylsilanyl)-1H-indol-3-ylmethoxymethyl]cyclohexyl}-dimethylamin (Unpolareres Diastereomer)

In einem ausgeheizten Kolben mit Schlenk-Aufsatz wurde ein Gemisch von **Ain-04** (400 mg, 1 mmol), 2-Iodanilin **(lan-01)** (263 mg, 1.2 mmol), Natriumcarbonat (530 mg, 5 mmol) und [1,3-Bis-(2,6-diisopropylphenyl)imidazol-2-yliden]-(3-chlorpyridyl)palladium(II)-dichlorid (PEPPSI, 136 mg, 0.2 mmol) evakuiert, mit Argon gespült und anschließend mit *N,N-*Dimethylformamid (6 ml) versetzt. Das Reaktionsgemisch wurde 20 h bei 100°C gerührt und anschließend i. Vak. eingeengt. Der Rückstand wurde zweimal mit Toluol versetzt, jeweils wieder zur Trockne eingeengt und danach mit Diethylether (30 ml) versetzt. Das Gemisch wurde mit 1 *M* Natriumthiosulfatlösung (10 ml) und Wasser (10 ml) gewaschen. Der ausgefallene Niederschlag wurde abfiltriert. Die organische Phase wurde mit Natriumchloridlösung (10 ml) gewaschen, mit Natriumsulfat getrocknet und i. Vak. eingeengt. Das Rohprodukt (540 mg) wurde durch Flashchromatographie (30 g, 22 × 2.5 cm) mit Chloroform / Methanol (9:1) und danach erneute Flashchromatographie (39 g, 22 × 2.5 cm) mit Methanol gereinigt.

### Ausbeute: 240 mg (49 %), gelblicher Feststoff

*Schmelzpunkt:* 53-56 °C

*¹H-NMR (DMSO-d₆):* 0.35 (s, 6H); 0.86 (s, 9H); 0.88-0.99 (m, 1H); 1.09-1.40 (m, 6H); 1.77 (d, J = 13.5 Hz, 2H); 2.20 (s, 6H); 2.55 (s, 2H); 3.17-3.22 (m, 2H); 4.54 (s, 2H); 6.95 (t, J = 7.4 Hz, 1H); 7.02-7.29 (m, 6H); 7.39 (d, J = 8.1 Hz, 1H); 7.53 (d, J = 7.9 Hz, 1H); 10.64 (s, 1 H).

*¹³C-NMR (DMSO-d₆):* -5.1; 17.0; 23.9; 26.4; 31.5; 36.2; 36.5; 37.5; 57.1; 64.7; 75.3; 111.2; 118.5; 118.7; 121.5; 125.4; 127.6; 128.4; 130.5; 133.6; 138.6; 139.1.

### Beispiel 23:

### [1-Benzyl-4-(2-triethylsilanyl-1H-indol-3-ylmethoxymethyl)cyclohexyl]dimethylamin (Unpolareres Diastereomer)

In einem Kolben mit Schlenk-Aufsatz wurde ein Gemisch von 1-Benzyl-N,N-dimethyl-4-((3-(triethylsilyl)prop-2-ynyloxy)methyl)cyclohexanamin (**Ain-05,** unpolareres Diastereomer) (520 mg, 1.3 mmol), 2-Iodanilin (342 mg, 1.56 mmol), Natriumcarbonat (689 mg, 6.5 mmol) und [1,3-Bis-(2,6-diisopropylphenyl)imidazol-2-yliden]-(3-chlorpyridyl)palladium(II)-dichlorid (PEPPSI, 133 mg, 0.2 mmol) evakuiert, mit Argon gespült und anschließend mit *N,N-*Dimethylformamid (7 ml) versetzt. Das Reaktionsgemisch wurde 20 h bei 120°C gerührt und dann i. Vak. eingeengt. Der Rückstand wurde zweimal mit Toluol versetzt, jeweils wieder zur Trockne eingeengt und danach mit Ethylacetat (30 ml) versetzt. Das Gemisch wurde mit 1 *M* Natriumthiosulfatlösung (10 ml) gewaschen. Der Niederschlag wurde abfiltriert. Die organische Phase wurde mit Natriumchloridlösung und Wasser (je 10 ml) gewaschen, mit Natriumsulfat getrocknet und i. Vak. eingeengt. Das Rohprodukt (617 mg) wurde durch Flashchromatographie (40 g, 17 × 3.6 cm) mit Ethylacetat / Methanol (9:1→4:1) gereinigt.

### Ausbeute: 239 mg (38 %), braunes Öl

*¹H-NMR (DMSO-dₑ): 0.80-0.95* (m, 17H); 1.13-1.37 (m, 5H); 1.71 (d, J = 13.1 Hz, 2H); 2.19 (s, 6H); 2.55 (s, 2H); 3.18 (d, J = 5.0 Hz, 2H); 4.55 (s, 2H); 6.92-7.26 (m, 7H); 7.38 (d, J = 8.1 Hz, 1 H); 7.53 (d, J = 7.9 Hz, 1 H); 10.62 (s, 1 H).

*¹³C-NMR (DMSO-d₆):* 3.1; 7.2; 23.9; 31.5; 36.2; 36.5; 37.4; 57.1; 64.4; 75.1; 11.3; 118.5; 121.3; 121.4; 125.4; 127.6; 128.5; 130.6; 133.3; 138.6; 139.1.

### Beispiel 24:

### [1-Benzyl-4-(1H-indol-3-ylmethoxymethyl)cyclohexyl]dimethylamin (Unpolareres Diastereomer)

Ein Gemisch von Benzyltrimethylammoniumfluorid-Monohydrat (202 mg, 1.08 mmol) und aktiviertem Molsieb 4 Å (700 mg) in wasserfreiem Tetrahydrofuran (5 ml) wurde 1 h bei Raumtemperatur gerührt. Anschließend wurde die Mischung mit einer Lösung von **Beispiel 23** (175 mg, 0.36 mmol) in wasserfreiem Tetrahydrofuran (3 ml) versetzt und 2.5 h unter Rückfluss erhitzt. Das Reaktionsgemisch wurde filtriert, der Filterrückstand mit Tetrahydrofuran gewaschen, das Filtrat i. Vak. eingeengt und der Rückstand (172 mg) durch Flashchromatographie (40 g, 17 × 3.6 cm) mit Ethylacetat / Methanol (4:1) gereinigt.

### Ausbeute: 82 mg (61 %), braunes Öl

*¹H-NMR (DMSO-d₆):* 0.86-1.03 (m, 2H); 1.11-1.38 (m, 5H); 1.77 (d, J = 12.8 Hz, 2H); 2.20 (s, 6H); 2.55 (s, 2H); 3.15 (s, 2H); 4.53 (s, 2H); 6.97 (ddd, J = 8.0, 7.0 und 1.1 Hz, 1 H); 7.34 (td, J = 8.1, 0.9 Hz, 1 H); 7.02-7.29 (m, 7H); 7.50-7.55 (m, 1H); 10.95 (s, 1 H).

*¹³C-NMR (DMSO-d₆):* 23.8; 31.5; 36.2; 36.5; 37.2; 48.5; 57.1; 64.3; 74.7; 111.3; 112.0; 118.5; 118.7; 121.0; 124.5, 125.4; 127.0; 127.6 (2C); 130.6 (2C); 136.3; 139.1.

### Beispiel 25:

### 1-Benzyl-4-(((5-fluor-2-(triethylsilyl)-1H-indol-3-yl)methoxy)methyl)-N,N-dimethylcyclo-hexanamin (Unpolareres Diastereomer)

In einem Kolben mit Schlenk-Aufsatz wurde ein Gemisch von Ain-05 (486 mg, 1.2 mmol), 4-Fluor-2-iodanilin (**lan-04**, 284 mg, 1.2 mmol), Natriumcarbonat (636 mg, 6.0 mmol) und [1,3-Bis-(2,6-diisopropylphenyl)imidazol-2-ylidenl-(3-chlorpyridyl)palladium(II)-dichlorid (PEPPSI, 122 mg, 0.18 mmol) evakuiert, mit Argon gespült und anschließend mit *N,N-*Dimethylformamid (7 ml) versetzt. Das Reaktionsgemisch wurde 20 h bei 100 °C gerührt und danach i. Vak. eingeengt. Der Rückstand wurde mit Toluol versetzt, wieder zur Trockne eingeengt und danach mit Diethylether (30 ml) versetzt. Die Suspension wurde mit 1 *M* Natriumthiosulfatlösung, Natriumchloridlösung und Wasser (je 10 ml) gewaschen, mit Natriumsulfat getrocknet und i. Vak. eingeengt. Das Rohprodukt (660 mg) wurde durch Flashchromatographie (50 g, 12 × 4 cm) mit Methanol und danach erneute Flashchromatographie (80 g, 12 × 4 cm) mit Chloroform / Methanol (9:1) gereinigt.

### Ausbeute: 388 mg (63 %), beigefarbener Feststoff

*Schmelzpunkt:* nicht bestimmbar

*¹H-NMR (DMSO-d₆):* 0.77-1.00 (m, 15H); 1.12-1.39 (m, 7H); 1.77 (d, J = 12.7 Hz, 2H); 2.19 (s, 6H); 2.54 (s, 2H); 3.17 (d, J = 5.2 Hz, 2H); 4.51 (s, 2H); 6.91 (dt, J = 9.1 und 2.5 Hz, 1H); 7.06-7.40 (m, 7H); 10.75 (s, 1 H). Enthält noch Signale für ca. 15 % einer Verunreinigung, wobei es sich um das entsprechende Monomethylaminoderivat oder das Hydrochlorid des Mono- oder Dimethylaminoderivates handelt.

*¹³C-NMR (DMSO-d₆):* 3.1; 7.2; 22.2; 23.8; 29.1; 31.5; 36.2; 36.5; 37.3; 57.1; 64.3; 73.6; 75.0; 102.8 (d, J = 22 Hz); 109.6 (d, J = 27 Hz); 112.2 (d, J = 10 Hz); 121.4; 125.4; 127.6; 128.2; 128.6; 128.7; 130.5; 135.3; 135.8; 139.1; 156.7 (d, J = 231 Hz).

### Beispiel 26:

### 1-Benzyl-4-(((2-(tert-butyldimethylsilyl)-5-fluor-1H-indol-3-yl)methoxy)methyl)-N,N-dimethylcyclohexanamin (Unpolareres Diastereomer)

In einem ausgeheizten Kolben mit Schlenk-Aufsatz wurde ein Gemisch von **Ain-04** (400 mg, 1 mmol), 4-Fluor-2-iodanilin (**lan-04,** 284 mg, 1.2 mmol), Natriumcarbonat (530 mg, 5 mmol) und [1,3-Bis-(2,6-diisopropylphenyl)imidazol-2-yliden]-(3-chlorpyridyl)palladium(II)-dichlorid (PEPPSI, 136 mg, 0.2 mmol) evakuiert, mit Argon gespült und anschließend mit *N,N-*Dimethylformamid (6 ml) versetzt. Das Reaktionsgemisch wurde 20 h bei 100 °C gerührt und anschließend i. Vak. eingeengt. Der Rückstand wurde dreimal mit Toluol versetzt, jeweils wieder zur Trockne eingeengt und danach mit Diethylether (30 ml) versetzt. Das Gemisch wurde mit Wasser (10 ml) gewaschen und der ausgefallene Niederschlag wurde abfiltriert. Die organische Phase wurde mit 1 *M* Natriumthiosulfatlösung (10 ml), Natriumchloridlösung (10 ml) und mit Wasser (10 ml) gewaschen, mit Natriumsulfat getrocknet und i. Vak. eingeengt. Das Rohprodukt (590 mg) wurde durch Flashchromatographie (40 g, 23 × 2.5 cm) mit Methanol, erneute Flashchromatographie (18 g, 18 × 2 cm) mit Chloroform / Methanol (95:5) und nochmalige Flashchromatographie (20 g, 13 × 2.5 cm) mit Ethylacetat / Cyclohexan (1:2) gereinigt.

### Ausbeute: 289 mg (57 %), gelblicher Feststoff

*Schmelzpunkt:* 58-62 °C

*¹H-NMR (DMSO-d₆):* 0.35 (s, 6H); 0.85 (s, 9H); 0.87-1.00 (m, 2H); 1.13-1.35(m, 5H); 1.77 (d, J = 13.1 Hz, 2H); 2.20 (s, 6H); 2.55 (s, 2H); 3.19 (d, J = 5.4 Hz, 2H); 4.51 (s, 2H); 6.92 (dt, J = 9.1 Hz, 1H); 7.07-7.27 (m, 6H); 7.36 (dd, J = 8.8 Hz, 1H); 10.74 (s, 1 H).

*¹³C-NMR (DMSO-d₆):* -5.2; 16.9; 23.9; 26.3; 31.5; 36.2; 36.5; 37.4; 57.1; 64.6; 75.2; 103.0 (d, J = 23 Hz); 109.7 (d, J = 27 Hz); 112.1 (d, J = 11 Hz); 121.6 (d, J = 5 Hz); 125.4; 127.6; 128.6; 130.5; 135.3; 136.0; 139.1; 156.7 (d, J = 232 Hz).

### Beispiel 27:

### 1-Benzyl-4-(((5-fluor-1H-indol-3-yl)methoxy)methyl)-N,N-dimethylcyclohexanamin (Unpolareres Diastereomer)

Ein Gemisch von Benzyltrimethylammoniumfluorid-Monohydrat (292 mg, 1.56 mmol) und aktiviertem Molsieb 4 Å (ca. 1 g) in wasserfreiem Tetrahydrofuran (7 ml) wurde 1 h bei Raumtemperatur gerührt. Anschließend wurde die Mischung mit einer Lösung von **Beispiel 25** (263 mg, 0.52 mmol) in wasserfreiem Tetrahydrofuran (3 ml) versetzt und 2.5 h unter Rückfluss erhitzt. Das Reaktionsgemisch wurde filtriert, der Filterrückstand mit Tetrahydrofuran gewaschen, das Filtrat i. Vak. eingeengt und der Rückstand (257 mg) durch Flashchromatographie (20 g, 16 × 2.5 cm) mit Ethylacetat / Methanol (4:1) gereinigt.

### Ausbeute: 112 mg (54 %), beigefarbener Feststoff

*Schmelzpunkt:* 42-45°C

*¹H-NMR (DMSO-d₆):* 0.87-1.02 (m, 2H); 1.12-1.38 (m, 5H); 1.77 (d, J = 13.1 Hz, 2H); 2.20 (s, 6H); 2.55 (s, 2H); 3.14 (d, J = 4.2 Hz, 2H); 4.50 (s, 2H); 6.86-6.96 (m, 2H); 7.08-7.37 (m, 7H); 11.06 (s, 1 H).

*¹³C-NMR (DMSO-d₆):* 23.8; 31.5; 36.2; 36.5; 37.2; 57.1; 64.1; 74.7; 103.2 (d, J = 24 Hz); 109.1(d, J = 27 Hz); 112.3 (d, J = 11 Hz); 125.4; 126.5; 127.3; 127.6; 130.6; 132.9; 139.1; 156.7 (d, J = 231 Hz).

### Beispiel 28:

### 1-Benzyl-N,N-dimethyl-4-(((2-(triethylsityl)-1H-pyrrolo[2,3-b]pyridin-3-yl)methoxy)-methyl)cyclohexanamin (Unpolareres Diastereomer)

In einem Kolben mit Schlenk-Aufsatz wurde ein Gemisch von **Ain-05** (570 mg, 1.43 mmol), 2-Amino-3-iodpyridin (346 mg, 1.57 mmol), Natriumcarbonat (758 mg, 7.15 mmol) und [1,3-Bis-(2,6-diisopropylphenyl)imidazol-2-yliden]-(3-chlorpyridyl)palladium(II)-dichlorid (PEPPSI, 146 mg, 0.21 mmol) evakuiert, mit Argon gespült und anschließend mit *N,N*-Dimethylformamid (8 ml) versetzt. Das Reaktionsgemisch wurde 20 h bei 110 °C gerührt und dann i. Vak. eingeengt. Der Rückstand wurde mit Toluol versetzt, wieder zur Trockne eingeengt und danach mit Diethylether (40 ml) versetzt. Die Lösung wurde mit 1 *M* Natriumthiosulfatlösung, Natriumchloridlösung und Wasser (je 20 ml) gewaschen, mit Natriumsulfat getrocknet und i. Vak. eingeengt. Das Rohprodukt (508 mg) wurde durch Flashchromatographie (25 g, 20 × 2.2 cm) mit Chloroform / Methanol (95:5) gereinigt.

### Ausbeute: 374 mg (53 %), cremefarbener Feststoff

*Schmelzpunkt:* 124-127°C

*¹H-NMR (DMSO-d₆):* 0.82-1.00 (m, 17H); 1.15-1.35 (m, 5H); 1.76 (d, J = 13.2 Hz, 2H); 2.19 (s, 6H); 2.54 (s, 2H); 3.17 (d, J = 4.5 Hz, 2H); 4.54 (s, 2H); 7.01 (dd, J = 7.85 und 4.6 Hz, 1 H); 7.07-7.27 (m, 5H); 7.93 (dd, J = 7.9 und 1.4 Hz, 1H); 8.21 (dd, J = 4.6 und 1.6 Hz, 1 H); 11.27 (s, 1 H).

*¹³C-NMR (DMSO-d₆):* 2.9 (3C); 7.1 (3C); 23.8 (2C); 31.4 (2C), 36.2; 36.5 (2C); 37.3; 57.1; 64.2; 75.0; 115.0; 120.4; 125.4; 126.7; 127.6 (2C); 130.6 (2C); 134.4; 139.1; 143.0; 150.7.

### Beispiel 29:

### 1-Benzyl-4-(((2-(tert-butyldimethylsilyl)-1 H-pyrrolo[2,3-b]pyridin-3-yl)methoxy)methyl)-N,N-dimethylcyclohexanamin (Unpolareres Diastereomer)

In einem ausgeheizten Kolben mit Schlenk-Aufsatz wurde ein Gemisch von **Ain-04** (175 mg, 0.44 mmol), 2-Amino-3-iodpyridin (116 mg, 0.53 mmol), Natriumcarbonat (232 mg, 2.2 mmol) und [1,3-Bis-(2,6-diisopropylphenyl)imidazol-2-yliden]-(3-chlorpyridyl)palladium(II)-chlorid (PEPPSI, (45 mg, 0.066 mmol) evakuiert, mit Argon gespült und anschließend mit *N,N*-Dimethylformamid (4 ml) versetzt. Das Reaktionsgemisch wurde 20 h bei 100 °C gerührt und anschließend i. Vak. eingeengt. Der Rückstand wurde mit Toluol versetzt und erneut zur Trockne eingeengt und danach mit Diethylether (30 ml) versetzt. Das Gemisch wurde mit 1 *M* Natriumthiosulfatlösung (10 ml) gewaschen, der ausgefallene Niederschlag abfiltriert, und die organische Phase mit Natriumchloridlösung und mit Wasser (je10 ml) gewaschen, mit Natriumsulfat getrocknet und i. Vak. eingeengt. Das Rohprodukt (430 mg) wurde durch Flashchromatographie (90 g, 20 × 4 cm) mit Methanol und erneute Flashchromatographie (30 g, 18 × 2.5 cm) mit Chloroform / Methanol (9:1) gereinigt.

### Ausbeute: 202 mg (93 %), gelblicher Feststoff

*Schmelzpunkt*: 51-56°C

*¹H-NMR (DMSO-d₆):* 0.37 (s, 6H); 0.85 (s, 9H); 0.90-1.02 (m, 2H); 1.11-1.43 (m, 5H); 1.76 (br d, J = 13.4 Hz, 2H); 2.19 (s, 6H); 2.54 (s, 2H); 3.20 (m, 2H); 4.54 (s, 2H); 7.01 (dd, J =4.63 und 7.85 Hz, 1H); 7.06-7.28 (m, 5H); 7.92 (dd, J =1.50 und 7.85 Hz, 1H); 8.21 (dd, J =1.59 und 4.63 Hz, 1 H); 11.30 (s, 1 H). Die durch eine Verunreinigung verursachten Signale liegen bei 0.05; 0.87; 2.20 und 4.06 ppm.

*¹³C-NMR (DMSO-d₆):* -5.0; 17.0; 23.7; 25.7; 26.4; 29.1; 31.5; 36.2; 36.5;37.4; 57.7; 58.0; 64.6; 75.0; 75.3; 1145.0; 120.3; 125.5; 126.9; 127.6; 130.5; 134.5; 139.1; 143.1; 150.7.

### Beispiel 30:

### 4-(((1H-Pyrrolo[2,3-b]pyridin-3-yl)methoxy)methyl)-1-benzyl-N,N-dimethylcyclohexan-amin (Unpolareres Diastereomer)

Ein Gemisch von Benzyltrimethylammoniumfluorid-Monohydrat (236 mg, 1.26 mmol) und aktiviertem Molsieb 4 Å (ca. 1 g) in wasserfreiem Tetrahydrofuran (7 ml) wurde 1 h bei Raumtemperatur gerührt. Anschließend wurde die Mischung mit einer Lösung von **Beispiel 28** (205 mg, 0.42 mmol) in wasserfreiem Tetrahydrofuran (3 ml) versetzt und 2 h unter Rückfluss erhitzt. Das Reaktionsgemisch wurde filtriert, der Filterrückstand mit Tetrahydrofuran gewaschen, das Filtrat i. Vak. eingeengt und der Rückstand (229 mg) durch Flashchromatographie (20 g, 21 × 2.0 cm) mit Ethylacetat / Methanol (9:1) gereinigt.

### Ausbeute: 70 mg (45 %), weißer Feststoff

*Schmelzpunkt:* 39-43 °C

*¹H-NMR (DMSO-d₆):* 0.86-1.01 (m, 2H); 1.14-1.38 (m, 5H); 1.77 (d, J = 13.1 Hz, 2H); 2.20 (s, 6H); 2.55 (s, 2H); 3.15 (d, J = 4.6 Hz, 2H); 4.52 (s, 2H); 7.03 (dd, J = 7.8 und 4.6 Hz, 1 H); 7.08-7.26 (m, 5H); 7.39 (d, J = 2.4 Hz, 1 H); 7.91 (dd, J = 7.8 und 1.3 Hz, 1 H); 8.19 (dd, J = 4.6 und 1.5 Hz, 1 H); 11.47 (s, 1H).

*¹³C-NMR (DMSO-d₆):* 23.8 (2C); 31.4 (2C); 36.2; 36.5 (2C); 37.1; 57.1; 64.3; 74.7; 110.9; 115.2; 119.1; 124.8; 125.4; 126.9; 127.6 (2C); 130.5 (2C); 139.1, 142.6; 148.7.

### Beispiel 31:

### 1-Benzyl-N,N-dimethyl-4-(((2-(triethylsilyl)-1H-pyrrolo[3,2-c]pyridin-3-yl)methoxy)-methyl)cyclohexanamin (Unpolareres Diastereomer)

In einem Kolben mit Schlenk-Aufsatz wurde ein Gemisch von Ain-05 (430 mg, 1.08 mmol), 4-Amino-3-iodpyridin (285 mg, 1.3 mmol), Natriumcarbonat (572 mg, 5.4 mmol) und [1,3-Bis-(2,6-diisopropylphenyl)imidazol-2-yliden]-(3-chlorpyridyl)palladium(II)-dichlorid (PEPPSI, 110 mg, 0.16 mmol) evakuiert, mit Argon gespült und anschließend mit *N,N*-Dimethylformamid (7 ml) versetzt. Das Reaktionsgemisch wurde 20 h bei 120 °C gerührt und dann i. Vak. eingeengt. Der Rückstand wurde mit Toluol versetzt, wieder zur Trockne eingeengt und danach mit Diethylether (15 ml) und Ethylacetat (15 ml) versetzt. Die Lösung wurde mit 1 *M* Natriumthiosulfatlösung, Natriumchloridlösung (20 ml) und Wasser (20 ml) gewaschen, mit Natriumsulfat getrocknet und i. Vak. eingeengt. Das Rohprodukt (581 mg) wurde durch Flashchromatographie (40 g, 23 × 2.2 cm) mit Chloroform / Methanol (9:1) gereinigt.

### Ausbeute: 310 mg (58 %), braunes Öl

*¹H-NMR (DMSO-d₆):* 0.83-0.96 (m, 15H); 1.13-1.40 (m, 7H); 1.80 (d, J = 13.3 Hz, 2H); 2.25 (s, 6H); 2.58 (s, 2H); 3.21 (d, J = 4.0 Hz, 2H); 4.61 (s, 2H); 7.09-7.28 (m, 5H); 7.34-7.37 (m, 1H); 8.13 (d, J = 5.7 Hz, 1 H); 8.84 (s, 1H); 11.08 (s, 1 H).

*¹³C-NMR (DMSO-d₆):* 3.0 (3C); 7.2 (3C); 23.7 (2C); 31.2 (2C), 36.2; 36.5 (2C); 37.2; 64.1; 75.1; 106.6; 121.7; 125.3; 125.6; 127.6 (2C); 130.6 (2C); 135.0; 138.7; 139.7; 141.8.

### Beispiel 32:

### 1-Benzyl-4-(((2-(tert-butyldimethylsilyl)-1 H-pyrrolo[3,2-c]pyridin-3-yl)methoxy)methyl)-N,N-dimethylcyclohexanamin (Unpolareres Diastereomer)

In einem Kolben mit Schlenk-Aufsatz wurde ein Gemisch von **Ain-04** (unpolares Diastereoisomer) (400 mg, 1 mmol), 4-Amino-3-lodpyridin (100 mg, 0.4 mmol), Natriumcarbonat (530 mg, 5 mmol) und [1,3-Bis-(2,6-diisopropylphenyl)imidazol-2-yliden]-(3-chlorpyridyl)palladium(II)-chlorid (PEPPSI, 68 mg, 0.1 mmol) evakuiert, mit Argon gespült und anschließend mit *N,N*-Dimethylformamid (6 ml) versetzt. Das Reaktionsgemisch wurde 20 h bei 120 °C gerührt und danach i. Vak. eingeengt. Der Rückstand wurde zweimal mit Toluol versetzt, jeweils wieder zur Trockne eingeengt und danach mit Diethylether (20 ml) versetzt. Das Gemisch wurde mit 1 *M* Natriumthiosulfat-Lösung (10 ml) gewaschen und der ausgefallene Niederschlag abfiltriert. Die organische Phase wurde mit Natriumchlorid-Lösung und Wasser (je 10 ml) gewaschen, mit Natriumsulfat getrocknet und i. Vak. eingeengt. Das Rohprodukt (520 mg) wurde durch Flashchromatographie (30 g, 20 × 2.5 cm) mit Chloroform / Methanol (9:1→0:1) gereinigt.

### Ausbeute: 179 mg (80 %), gelblicher Feststoff

*Schmelzpunkt:* 139-144 °C

*¹H-NMR (DMSO-d₆):* 0.37 (s, 6H); 0.85 (s, 9H); 0.93-1.13 (m, 2H); 1.15-1.48 (m, 5H); 1.81-1.94 (m, 2H), 2.37 (br s, 6H), 2.67 (s, 2H), 3.25 (d, J =4.8 Hz, 2H), 4.61 (s, 2H), 7.12-7.31 (m, 5H), 7.41 (dd, J = 5.8, 1.0 Hz, 1H), 8.15 (d, J = 5.8 Hz, 1H), 8.87 (s, 1H), 11.28 (s, 1 H).

*¹³C-NMR (DMSO-d₆):* -5.1; 16.9; 23.3; 26.3 (3C); 30,7; 36.5; 36.7; 38.9; 64.4; 75.0; 106.9; 122.2; 125.1; 125.9; 127.7 (2C);130.7 (2C); 136.0; 138.7; 141.2; 142.0.

### Beispiel 33:

### 4-(((1H-Pyrrolo[3,2-c]pyridin-3-yl)methoxy)methyl)-1-benzyl-N,N-dimethylcyclohexan-amin (Unpolareres Diastereomer)

Ein Gemisch von Benzyltrimethylammoniumfluorid-Monohydrat (275mg, 1.47 mmol) und aktiviertem Molsieb 4 Å (ca. 1 g) in wasserfreiem Tetrahydrofuran (8 ml) wurde 1 h bei Raumtemperatur gerührt. Anschließend wurde die Mischung mit einer Lösung von Beispiel 31 (240 mg, 0.49 mmol) in wasserfreiem Tetrahydrofuran (3 ml) versetzt und 2 h unter Rückfluss erhitzt. Das Reaktionsgemisch wurde filtriert, der Filterrückstand mit Tetrahydrofuran gewaschen, das Filtrat i. Vak. eingeengt und der Rückstand (200 mg) durch Flashchromatographie (10 g, 19 × 1.8 cm) mit Methanol gereinigt.

### Ausbeute: 25 mg (14 %)

*Schmelzpunkt:* nicht bestimmbar

*¹H-NMR (CDCl₃):* 1.03-1.19 (m, 2H); 1.25-1.50 (m, 5H); 1.79 (d, J = 12.7 Hz, 2H); 2.29 (s, 6H); 2.60 (s, 2H); 3.29-3.32 (m, 2H); 4.65 (s, 2H); 7.06-7.28 (m, 8H); 8.25 (d, J = 5.8 Hz, 1 H); 8.97 (s, 1 H).

*¹³C-NMR (CDCl₃)*:24.2; 31.8; 37.0; 37.5; 58.4; 64.7; 76.0; 106.7; 113.9; 124.0; 124.4; 125.7; 127.7; 130.8; 139.1; 140.4; 140.5; 142.4.

### Beispiel 34:

### 1-Benzyl-N,N-dimethyl-4-(((2-(triethylsilyl)-1H-indol-3-yl)methoxy)methyl)cyclohexan-amin (Polareres Diastereomer)

In einem Kolben mit Schlenk-Aufsatz wurde ein Gemisch von **Ain-06** (650 mg, 1.6 mmol), 2-lodanilin (385 mg, 1.76 mmol), Natriumcarbonat (848 mg, 8.0 mmol) und [1,3-Bis-(2,6-diisopropylphenyl)imidazol-2-yliden]-(3-chlorpyridyl)palladium(II)-dichlorid (PEPPSI, 163 mg, 0.24 mmol) evakuiert, mit Argon gespült und anschließend mit *N,N*-Dimethylformamid (10 ml) versetzt. Das Reaktionsgemisch wurde 18 h bei 100 °C gerührt und dann i. Vak. eingeengt. Der Rückstand wurde mit Toluol versetzt, wieder zur Trockne eingeengt und danach mit Diethylether (40 ml) versetzt. Die Lösung wurde mit 1 *M* Natriumthiosulfatlösung, danach mit Natriumchloridlösung und Wasser (je 20 ml) gewaschen, mit Natriumsulfat getrocknet und i. Vak. eingeengt. Das Rohprodukt (960 mg) wurde durch Flashchromatographie (90 g, 20 × 4 cm) mit Chloroform / Methanol (95:5) gereinigt.

### Ausbeute: 380 mg (48 %), brauner Feststoff

*Schmelzpunkt:* nicht bestimmbar

*¹H-NMR (DMSO-d₆):* 0.77-1.00 (m, 15H); 1.21-1.77 (m, 9H); 2.40 (br s, 6H); 3.23 (d, J = 5.9 Hz, 2H); 4.56 (s, 2H); 6.97 (ddd, J = 7.9; 7.9 und 1.0 Hz, 1H); 7.08 (ddd, J = 8.1; 8.1 und 1.2 Hz, 1 H); 7.13-7.36 (m, 5H); 7.40 (d, J = 8.1 Hz, 1H); 7.53 (d, J = 7.8 Hz, 1H); 10.7 (s, 1 H). Eine Methylen-Gruppe ist vom HDO-Signal überlagert.

*¹³C-NMR (DMSO-d₆):* 3.1; 7.2; 24.3 (br); 25.8; 29.8; 34.8 (br); 36.1; 36.3; 37.2; 64.5; 72.6 (br); 73.5; 11.3; 118.4; 118.5; 121.23; 121.34; 127.0 (br); 128.4; 130.4; 130.5; 133.3; 138.6.

### Beispiel 35:

### 1-Benzyl-4-(((2-(tert-butyldimethylsilyl)-1H-indol-3-yl)methoxy)methyl)-N,N-dimethyl-cyclohexanamin (Polareres Diastereomer)

In einem Kolben mit Schlenk-Aufsatz wurde ein Gemisch von **Ain-07** (540 mg, 1.35 mmol), 2-lodanilin (355 mg, 1.62 mmol), Natriumcarbonat (715 mg, 6.75 mmol) und [1,3-Bis-(2,6-diisopropylphenyl)imidazol-2-yliden]-(3-chlorpyridyl)palladium(II)-dichlorid (PEPPSI, 136 mg, 0.2 mmol) evakuiert, mit Argon gespült und anschließend mit *N,N*-Dimethylformamid (7 ml) versetzt. Das Reaktionsgemisch wurde 22 h bei 100 °C gerührt und dann i. Vak. eingeengt. Der Rückstand wurde mit Toluol versetzt, jeweils wieder zur Trockne eingeengt und danach mit Diethylether (20 ml) versetzt. Das Gemisch wurde mit 1 *M* Natriumthiosulfatlösung (10 ml) gewaschen. Der ausgefallene Niederschlag wurde abfiltriert. Die organische Phase wurde mit Natriumchloridlösung (10 ml) und mit Wasser (10 ml) gewaschen, mit Natriumsulfat getrocknet und i. Vak. eingeengt. Das Rohprodukt (590 mg) wurde durch Flashchromatographie (30 g, 20 × 2.5 cm) mit Methanol und danach erneute Flashchromatographie (40 g, 14 × 4 cm) mit Ethylacetat / Methanol (9:1) gereinigt.

### Ausbeute: 240 mg (36 %), cremefarbener Feststoff

*Schmelzpunkt:* 46-48 °C

*¹H-NMR (DMSO-d₆):* 0.35 (s, 6H); 0.87 (s, 9H); 1.11-1.75 (m, 9H); 2.24 (br s, 6H); 2.67 (s, 2H); 3.23 (d, *J* = 5.9 Hz, 2H); 4.53 (s, 2H); 6.91-7.02 (m, 1 H); 7.03-7.12 (m, 1H); 7.31-7.12 (m, 5H); 7.40 (d, J = 8.1 Hz, 1 H); 7.52 (d, J = 7.8 Hz, 1H); 10.67 (s, 1 H).

### Beispiel 36:

### 4-(((1H-Indol-3-yl)methoxy)methyl)-1-benzyl-N,N-dimethylcyclohexanamin (Polareres Diastereomer)

Ein Gemisch von Benzyltrimethylammoniumfluorid-Monohydrat (315 mg, 1.68 mmol) und aktiviertem Molsieb 4Å (ca. 1.2 g) in wasserfreiem Tetrahydrofuran (8 ml) wurde 1 h bei Raumtemperatur gerührt. Anschließend wurde die Mischung mit einer Lösung von **Beispiel 34** (275 mg, 0.56 mmol) in wasserfreiem Tetrahydrofuran (4 ml) versetzt und 2 h unter Rückfluss erhitzt. Zum Reaktionsgemisch wurde noch weiteres aktiviertes Molsieb 4 Å (300 mg) gegeben und weitere 1.5 h unter Rückfluss erhitzt. Das Reaktionsgemisch wurde filtriert, der Filterrückstand mit Tetrahydrofuran gewaschen, das Filtrat i. Vak. eingeengt und der Rückstand (260 mg) durch Flashchromatographie (20 g, 22 × 2.0 cm) mit Ethylacetat / Methanol (4: 1) gereinigt.

### Ausbeute: 81 mg (38 %), gelblicher Feststoff

*Schmelzpunkt:* 44-50°C

*¹H-NMR (DMSO-d₆):* 1.18-1.79 (m, 9H); 2.28 (s, 6H); 2.74 (br s, 2H); 3.21 (d, J = 6.4 Hz, 2H); 4.54 (s, 2H); 6.95-7.13 (m, 2H); 7.13-7.40 (m, 7H); 7.53 (d, J = 7.8 Hz, 1 H); 10.99 (s, 1 H). Es wird teilweise ein doppelter Signalsatz beobachtet, wobei es sich bei der Verunreinigung entweder um das entsprechende Monomethylaminoderivat oder das Hydrochlorid des Mono- oder Dimethylaminoderivates handeln könnte.

*¹³C-NMR (DMSO-d₆):24.0* (br); 28.4 (br); 30.0; 34.8; 356.; 36.3; 37.2; 64.4; 72.4; 73.2; 11.4; 11.9; 118.6.; 121.1; 124.5, 125.9 (br); 126.9; 127.7 (br); 130.5; 130.6; 136.3.

### Beispiel 37:

### 1-Benzyl-4-(((5-fluor-2-(triethylsilyl)-1H-indol-3-yl)methoxy)methyl)-N,N-dimethylcyclo-hexanamin (Polareres Diastereomer)

In einem Kolben mit Schlenk-Aufsatz wurde ein Gemisch von **Ain-06** (550 mg, 1.38 mmol), 4-Fluor-2-iodanilin (360 mg, 1.52 mmol), Natriumcarbonat (731 mg, 6.9 mmol) und [1,3-Bis-(2,6-diisopropylphenyl)imidazol-2-yliden]-(3-chlorpyridyl)palladium(II)-dichlorid (PEPPSI, 141 mg, 0.21 mmol) evakuiert, mit Argon gespült und anschließend mit *N,N-*Dimethylformamid (8 ml) versetzt. Das Reaktionsgemisch wurde 18 h bei 110 °C gerührt und dann i. Vak. eingeengt. Der Rückstand wurde mit Toluol versetzt, wieder zur Trockne eingeengt und danach mit Diethylether (50 ml) versetzt. Die Lösung wurde mit 1 *M* Natriumthiosulfatlösung (50 ml), Natriumchloridlösung (50 ml) und Wasser (50 ml) gewaschen, mit Natriumsulfat getrocknet und i. Vak. eingeengt. Das Rohprodukt (682 mg) wurde durch Flashchromatographie (84 g, 19 × 4 cm) mit Chloroform / Methanol (95:5) gereinigt.

### Ausbeute: 432 mg (61 %), Öl

*¹H-NMR (DMSO-d₆):* 0.78-0.99 (m, 15H);1.18-1.53 (m, 5H); 1-54-1.75 (m, 4H); 2.24 (s, 6H); 2.67 (s, 2H); 3.19 (d, J = 6.3 Hz, 2H); 4.50 (s, 2H); 6.88-6.98 (m, 1H); 7.10-7.35 (m, 5H); 7.35-7.42 (m, 2H); 10.75 (s, 1 H). Es wird teilweise ein doppelter Signalsatz beobachtet, wobei es sich bei der Verunreinigung entweder um das entsprechende Monomethylaminoderivat oder das Hydrochlorid des Mono- oder Dimethylaminoderivates handeln könnte.

*¹³C-NMR (DMSO-d₆):* 3.1,7.2; 23.6; 24.4; 28.0; 34.4; 35.7; 36.3; 37.1; 64.3; 72.3; 73.6; 102.8 (d, J = 23 Hz); 109.6 (d, J = 26 Hz); 112.1 (d, J = 10 Hz); 121.3 (d, J = 5 Hz); 125.5; 127.5; 128.4; 128.5; 130.4; 135.3; 135.8; 156.7 (d, J = 232 Hz).

### Beispiel 38:

### 1-Benzyl-4-(((2-(tert-butyldimethylsilyl)-5-fluor-1H-indol-3-yl)methoxy)methyl)-N,N-dimethylcyclohexanamin (Polareres Diastereomer)

**In** einem ausgeheizten Kolben mit Schlenk-Aufsatz wurde ein Gemisch von **Ain-07** (400 mg, 1 mmol), 4-Fluor-2-iodanilin (284 mg, 1.2 mmol), Natriumcarbonat (530 mg, 5 mmol) und [1,3-Bis-(2,6-diisopropylphenyl)imidazol-2-yliden]-(3-chlorpyridyl)palladium(II)-dichlorid (PEPPSI, 136 mg, 0.2 mmol) evakuiert, mit Argon gespült und anschließend mit *N,N-*Dimethylformamid (6 ml) versetzt. Das Reaktionsgemisch wurde 20 h bei 100°C gerührt und anschließend i. Vak. eingeengt. Der Rückstand wurde mit Toluol versetzt, erneut zur Trockne eingeengt und danach mit Diethylether (40 ml) versetzt. Das Gemisch wurde mit 1 M Natriumthiosulfatlösung (10 ml) gewaschen. Der ausgefallene Niederschlag wurde abfiltriert. Die organische Phase wurde mit Natriumchloridlösung (10 ml) und mit Wasser (10 ml) gewaschen, mit Natriumsulfat getrocknet und i. Vak. eingeengt. Das Rohprodukt (460 mg) wurde durch Flashchromatographie (39 g, 22 × 2.5 cm) und danach erneute Flashchromatographie (30 g, 18 × 2.5 cm) jeweils mit Methanol gereinigt.

### Ausbeute: 289 mg (53 %), gelblicher Feststoff

*Schmelzpunkt:* 50-55 °C

*¹H-NMR (DMSO-d₆):* 0.34 (s, 6H); 0.86 (s, 9H); 1.16-1.52 (m, 6H); 1.56-1.75 (m, 3H); 2.22 (s, 6H); 2.65 (s, 2H); 3.14-3.27 (m, 2H); 4.49 (s, 2H); 6.93 (dt, J = 9.2 Hz, 1 H); 7.10-7.26 (m, 6H); 7.39 (dd, J = 8.8, 4.2 Hz, 1H); 10.77 (s, 1H).

*¹³C-NMR (DMSO-d₆):* -5.2; 16.9; 23.5; 26.3; 28.0; 34.3; 35.8; 37.1; 57.7; 64.6; 72.5; 102.9 (d, J = 23 Hz); 109.7 (d, J = 26 Hz); 112.1 (d, J = 10 Hz); 121.5 (d, J = 5 Hz); 125.5; 127.5; 128.6; 130.4; 135.3; 136.0; 139.0; 156.7 (d, J = 232 Hz).

### Beispiel 39:

### 1-Benzyl-4-(((5-fluor-1 H-indol-3-yl)methoxy)methyl)-N,N-dimethylcyclohexanamin (Polareres Diastereomer)

Ein Gemisch von Benzyltrimethylammoniumfluorid-Monohydrat (276 mg, 1.47 mmol) und aktiviertem Molsieb 4 Å (ca. 1 g) in wasserfreiem Tetrahydrofuran (7 ml) wurde 1 h bei Raumtemperatur gerührt. Anschließend wurde die Mischung mit einer Lösung von **Beispiel 37** (250 mg, 0.49 mmol) in wasserfreiem Tetrahydrofuran (3 ml) versetzt und 2 h unter Rückfluss erhitzt. Das Reaktionsgemisch wurde filtriert, der Filterrückstand mit Tetrahydrofuran gewaschen, das Filtrat i. Vak. eingeengt und der Rückstand (200 mg) durch Flashchromatographie (20 g, 21 × 2.0 cm) mit Ethylacetat / Methanol (9:1) gereinigt.

### Ausbeute: 48 mg (25 %), gelbliches Öl

*¹H-NMR (DMSO-d₆):* 1.20-1-52 (m, 5H); 1.52-1.73(m, 4H); 2.23 (s, 6H); 2.68 (s, 2H); 3.19 (d, J = 6.3 Hz, 2H); 4.50 (s, 2H); 6.89-6.97 (m, 1 H); 7.11-7.40 (m, 8H); 11.09 (s, 1 H).

*¹³C-NMR (DMSO-d₆):* 23.8; 28.2; 34.6; 35.8; 37.2; 57.7; 64.2; 72.2; 103.2 (d, J = 23 Hz); 109.2 (d, J = 26 Hz); 112.2 (d, J = 4 Hz); 112.2 (d, J = 10 Hz); 125.5; 126.5; 127.2; 127.5; 130.4; 133.0; 139.0; 156.7 (d, J = 231 Hz).

### Beispiel 40:

### 1-Benzyl-N,N-dimethyl-4-(((2-(triethylsilyl)-1H-pyrrolo[2,3-b]pyridin-3-yl)methoxy)-methyl)cyclohexanamin hydrochlorid (Polareres Diastereomer)

In einem Kolben mit Schlenk-Aufsatz wurde ein Gemisch von **Ain-06** (500 mg, 1.25 mmol), 2-Amino-3-iodpyridin (302 mg, 1.38 mmol), Natriumcarbonat (662 mg, 6.25 mmol) und [1,3-Bis-(2,6-diisopropylphenyl)imidazol-2-yliden]-(3-chlorpyridyl)palladium(II)-dichlorid (PEPPSI, 127 mg, 0.19 mmol) evakuiert, mit Argon gespült und anschließend mit *N,N-*Dimethylformamid (7 ml) versetzt. Das Reaktionsgemisch wurde 20 h bei 110 °C gerührt und dann i. Vak. eingeengt. Der Rückstand wurde mit Toluol versetzt, wieder zur Trockne eingeengt und danach mit Ethylacetat (40 ml) versetzt. Das Gemisch wurde mit 1 *M* Natriumthiosulfatlösung, Natriumchloridlösung und Wasser (je 20 ml) gewaschen, mit Natriumsulfat getrocknet und i. Vak. eingeengt. Das Rohprodukt (665 mg) wurde durch Flashchromatographie (100 g, 23 × 4 cm) mit Chloroform / Methanol (9:1) gereinigt.

### Ausbeute: 590 mg (88 %), beigefarbener Feststoff

*Schmelzpunkt:* 62-67°C

*¹H-NMR (DMSO-d₆):* 0.82-1.01 (m, 15H); 1.18-1.44 (m, 4H); 1.49-1.84 (m, 5H); 2.56 (s, 6H); 3.08 (s, 2H); 3.24 (s, 2H); 4.58 (s, 2H); 7.02-7.08 (m, 1H); 7.17- 7.41 (m, 5H); 7.90-8.00 (m, 1H); 8.22-8.26 (m, 1H); 9.83 (br s, 1H); 11.31 (s, 1H).

*¹³C-NMR (DMSO-d₆):* 3.0 (3C); 7.2 (3C); 24.5 (2C); 29.9 (2C); 43.5; 35.4; 37.3 (2C); 64.3; 66.6; 73.2; 115.0; 120.2; 120.3; 126.6; 127.1; 128.5; 130.5; 134.5; 135.5; 143.1; 150.7.

### Beispiel 41:

### 4-(((1H-Pyrrolo[2,3-b]pyridin-3-yl)methoxy)methyl)-1-benzyl-N,N-dimethylcyclohexan-amin (Polareres Diastereomer)

Ein Gemisch von Benzyltrimethylammoniumfluorid-Monohydrat (466 mg, 2.49 mmol) und aktiviertem Molsieb 4 Å (ca. 1 g) in wasserfreiem Tetrahydrofuran (10 ml) wurde 1 h bei Raumtemperatur gerührt. Anschließend wurde die Mischung mit einer Lösung von Beispiel 40 (440 mg, 0.83 mmol) in wasserfreiem Tetrahydrofuran (5 ml) versetzt und 2.5 h unter Rückfluss erhitzt. Das Reaktionsgemisch wurde filtriert, der Filterrückstand mit Tetrahydrofuran gewaschen, das Filtrat i. Vak. eingeengt und der Rückstand (270 mg) durch Flashchromatographie (24 g, 18 × 2.5 cm) mit Ethylacetat / Methanol (4:1) gereinigt.

### Ausbeute: 76 mg (24 %), gelbes Öl

*¹H-NMR (DMSO-d₆):* 1.19-1.74 (m, 9H); 2.23 (br s, 6H); 2.66 (s, 2H); 3.19 (d, J = 6.23 Hz, 2H); 4.54 (s, 2H); 7.05 (dd, J = 7.8 und 4.6 Hz, 1H); 7.08-7.31 (m, 5H); 7.38-7.40 (m, 1H); 7.91 (d, J = 7.6 Hz, 1 H); 8.22 (dd, J = 4.6 und 1.5 Hz, 1 H); 11.49 (s, 1 H).

### Beispiel 42:

### 1-Benzyl-4-(((2-(tert-butyldimethylsilyl)-1H-pyrrolo[3,2-c]pyridin-3-yl)methoxy)methyl)-N,N-dimethylcyclohexanamin (Polareres Diastereomer)

In einem Kolben mit Schlenk-Aufsatz wurde ein Gemisch von Ain-07 (480 mg, 1.2 mmol), 4-Amino-3-lodpyridin (317 mg, 1.44 mmol), Natriumcarbonat (636 mg, 6 mmol) und [1,3-Bis-(2,6-diisopropylphenyl)imidazol-2-yliden]-(3-chlorpyridyl)palladium(II)-dichlorid (PEPPSI, 122 mg, 0.18 mmol) evakuiert, mit Argon gespült und anschließend mit *N,N-*Dimethylformamid (6 ml) versetzt. Das Reaktionsgemisch wurde 3 h bei 120 °C und 22 h bei 130 °C gerührt und anschließend i. Vak. eingeengt. Der Rückstand wurde zweimal mit Toluol versetzt, jeweils wieder zur Trockne eingeengt und danach mit Diethylether (20 ml) versetzt. Das Gemisch wurde mit 1 *M* Natriumthiosulfatlösung (10 ml) gewaschen. Der ausgefallene Niederschlag wurde abfiltriert. Die organische Phase wurde mit Natriumchloridlösung (10 ml) und mit Wasser (10 ml) gewaschen, mit Natriumsulfat getrocknet und i. Vak. eingeengt. Das Rohprodukt (530 mg) wurde durch Flashchromatographie (45 g, 15 × 4 cm) mit Ethylacetat / Methanol (9:1→4:1) gereinigt.

### Ausbeute: 253 mg (43 %)

*Schmelzpunkt:* nicht bestimmbar

*¹H-NMR (DMSO-d₆):* 0.37 (s, 6H); 0.86 (s, 9H); 1.20-1.75 (m, 9H); 2.28 (s, 6H); 2.74 (s, 2H); 3.25 (d, J = 6.3 Hz, 2H); 4.60 (s, 2H); 7.11-7.30 (m, 5H); 7.37 (d, J = 5.7 Hz, 1H), 8.15 (d, J = 5.8 Hz, 1 H), 8.85 (s, 1 H); 11.11 (s, 1 H).

*¹³C-NMR (DMSO-d₆):* -5.1; 17.0; 23.7; 26.3; 28.4; 34.5; 35.5; 37.1; 59.7; 64.5; 72.8; 106.5; 121.7; 125.2; 125.8; 127.7; 130.4; 135,2; 138.2; 140.0; 141.8; 142.0.

### Beispiel 43:

### 1-Butyl-N,N-dimethyl-4-(((2-(triethylsilyl)-1H-indol-3-yl)methoxy)methyl)cyclohexanamin (Unpolareres Diastereomer)

In einem Kolben mit Schlenk-Aufsatz wurde ein Gemisch von **Ain-08** (365 mg, 1.0 mmol), 2-lodanilin (261 mg, 1.2 mmol), [1,3-Bis-(2,6-diisopropylphenyl)imidazol-2-yliden]-3-(chlorpyridyl)palladium(II)-chlorid (PEPPSI^{™}, 136 mg, 0.2 mmol) und Natriumcarbonat (529 mg, 5 mmol) 30 min evakuiert. Danach wurde unter einem Argonstrom *N,N-*Dimethylformamid (8 ml), dass vorher 1 h mit Argon gesättigt wurde, zugesetzt. Die Suspension wurde über Nacht bei 100°C gerührt und anschließend i. Vak. eingeengt. Der Rückstand wurde wiederholt mit Toluol (3 × 30 ml) versetzt, jeweils wieder i. Vak. eingeengt und in Wasser (20 ml) und Ethylacetat (20 ml) aufgenommen. Die Phasen wurden getrennt und die wässrige Phase mit Ethylacetat (2 × 20 ml) extrahiert. Die vereinigten organischen Phasen wurden mit 1 M Natriumthiosulfat-Lösung (30 ml) und gesättigter Natriumchloridlösung (50 ml) gewaschen und i. Vak. eingeengt. Das Rohprodukt (617 mg) wurde durch Flashchromatographie (70 g, 20 × 3.6 cm) mit Ethylacetat / Methanol (9:1) gereinigt.

### Ausbeute: 256 mg (56 %), braunes Öl

*¹H-NMR (DMSO-d₆):* 0.81-1.00 (m, 18H); 1.08-1.90 (m, 15H), 2.10 (s, 4H); 2.62 (s, 2H); 3.25 (s, 2H); 4.57 und 4.62 (s, 2H); 6.98 (t, 1H, J = 7.4 Hz); 7.08 (t, 1H, J = 7.4 Hz); 7.40 (d, 1H, J = 8.1 Hz); 7.56 (d, 1 H, J = 7.7 Hz); 8.76 (s, 0.3H); 10.69 (s, 1 H).

*¹³C-NMR (DMSO-d₆):* 3.1; 3.7; 7.2; 22.7; 23.3; 24.1; 24.9; 25.4; 26.2; 28.1; 30.6; 31.5; 35.0; 36.6; 37.5; 55.6; 64.4; 66.5; 73.3; 75.1; 111.2; 118.4; 121.3; 128.5; 133.3; 138.6

### Beispiel 44:

### 4-(((1H-Indol-3-yl)methoxy)methyl)-1-butyl-N,N-dimethylcyclohexanamin (Unpolareres Diastereomer)

Eine Suspension von Benzyltrimethylammoniumfluorid-Monohydrat (323 mg, 1.72 mmol) und aktiviertem Molsieb 4 Å (2 g) in wasserfreiem Tetrahydrofuran (10 ml) wurde 1 h bei Raumtemperatur gerührt. Anschließend wurde dem Gemisch eine Lösung von **Beispiel 43** (273 mg, 0.57 mmol) in wasserfreiem Tetrahydrofuran (15 ml) zugesetzt und das Gemisch 1.5 h unter Rückftuss gerührt. Das Gemisch wurde danach filtriert und der Filterrückstand mit Tetrahydrofuran (2 × 35 ml) gewaschen. Das Filtrat wurde i. Vak. eingeengt und das Rohprodukt (246 mg) durch Flashchromatographie (30 g, 20 x 2.7 cm) mit Ethylacetat / Cyclohexan (9:1) gereinigt.

### Ausbeute: 137 mg (67 %), gelbliches Öl

*¹H-NMR (DMSO-d₆):* 0.87 (t, 3H, J = 7.0 Hz); 1.10-1.75 (m, 15H); 2.10 (br s, 6H); 3.22 (d, 2H, J = 5.9 Hz); 4.58 (s, 2H); 6.99 (dt, 1H, J = 7.0, 1.1 Hz); 7.08 (dt, 1H, J = 7.0, 1.2 Hz); 7.30 (d, 1 H, J = 2.4 Hz); 7.36 (td, J = 8.0, 0.9 Hz); 7.56 (br d, 1 H, J = 7.2 Hz); 11.01 (s, 1 H).

*¹³C-NMR (DMSO-d₆):* 14.0; 23.3; 24.1; 26.3; 30.6; 31.5; 36.7; 37.5; 55.7; 64.4; 74.1; 111.4; 112.0; 118.6; 118.7; 121.1; 124.6; 127.0; 136.3.

### Beispiel 45:

### 1-Butyl-4-(((5-fluor-2-(triethylsilyl)-1H-indol-3-yl)methoxy)methyl)-N,N-dimethylcyclo-hexanamin (Unpolareres Diastereomer)

In einem Kolben mit Schlenk-Aufsatz wurde ein Gemisch von **Ain-08** (548 mg, 1.5 mmol), 4-Fluor-2-iodanilin (426 mg, 1.8 mmol), [1,3-Bis-(2,6-diisopropylphenyl)imidazol-2-yliden]-3-(chlorpyridyl)palladium(II)-chlorid) (PEPPSI^{™}, 204 mg, 0.3 mmol) und Natriumcarbonat (793 mg, 7.48 mmol) 30 min evakuiert. Anschließend wurde unter einem Argonstrom *N,N-*Dimethylformamid (8 ml), dass vorher 1 h mit Argon gesättigt wurde, zugesetzt. Die Suspension wurde über Nacht bei 120 °C gerührt und danach i. Vak. eingeengt. Der Rückstand wurde wiederholt mit Toluol (3 × 30 ml) versetzt, jeweils wieder i. Vak. eingeengt und in Wasser (20 ml) und Ethylacetat (20 ml) aufgenommen. Die Phasen wurden getrennt und die wässrige Phase mit Ethylacetat (2 × 20 ml) extrahiert. Die vereinigten organischen Phasen wurden mit 1 *M* Natriumthiosulfat-Lösung (30 ml) und gesättigter Natriumchloridlösung (50 ml) gewaschen und i. Vak. eingeengt. Das Rohprodukt (932 mg) wurde durch Flashchromatographie (100 g, 3.6 × 25 cm) mit Ethylacetat / Methanol (9:1) gereinigt. Da kein reines Produkt isoliert werden konnte, wurden die entsprechenden Mischfraktionen (472 mg) durch MPLC [130 g, LiChroprep Si60 (15-25 µm)] 46 × 2.6 cm] mit Ethylacetat / Methanol (9:1) gereinigt.

### Ausbeute: 343 mg (48 %), braunes Öl

*¹H-NMR (DMSO-d₆):* 0.82-0.98 (m, 18 H); 1.05-1.55 (m, 13H); 1.65 (d, 2H, J = 8.6 Hz); 2.10 (s, 6H); 3.24 (d, 2H, J = 6.2 Hz); 4.55 (s, 2H); 6.92 (dt, 1H, J = 9.1, 2.5 Hz); 7.26 (dd, 1H, J = 2.5 und 10.0 Hz); 7.37 (dd, 1H, J = 4.6 und 8.8 Hz); 10.76 (s, 1H).

*¹³C-NMR (DMSO-d₆):* 3.0; 7.2; 13.9; 23.3; 24.0; 26.3; 30.6; 31.5; 36.6; 37.5; 55.6; 64.3; 75.1; 102.8 (d, J = 22 Hz); 110 (d, J = 26 Hz); 112.2; 121.5; 128.6 (d, J = 10 Hz); 135.3; 135.8; 156.7 (d, J = 232 Hz).

### Beispiel 46:

### 1-Butyl-4-(((5-fluor-1H-indol-3-yl)methoxy)methyl)-N,N-dimethylcyclohexanamin (Unpolareres Diastereomer)

Eine Suspension von Benzyltrimethylammoniumfluorid-Monohydrat (334 mg, 1.78 mmol) und aktiviertem Molsieb 4 Å (2 g) in wasserfreiem Tetrahydrofuran (10 ml) wurde 1 h bei Raumtemperatur gerührt. Anschließend wurde dem Gemisch eine Lösung von **Beispiel 45** (283 mg, 0.59 mmol) in wasserfreiem Tetrahydrofuran (20 ml) zugesetzt und die Suspension 2 h unter Rückfluss gerührt. Das Gemisch wurde danach filtriert und der Filterrückstand mit Tetrahydrofuran (2 × 35 ml) gewaschen. Das Filtrat wurde i. Vak. eingeengt und das Rohprodukt (246 mg) durch Flashchromatographie (30 g, 20 × 2.5 cm) mit Ethylacetat / Methanol (9:1) gereinigt.

### Ausbeute: 148 mg (68 %), gelbliches Öl

*¹H-NMR (DMSO-d₆):* 0.87 (t, 3H, J = 6.8 Hz); 1.00-1.60 (m, 15H); 2.09 (br s, 5H); 2.62 (br s, 1H); 3.21 (d, 2H, J = 5.7 Hz); 4.55 (s, 2H); 6.92 (dt, 1H, J = 9.3 und 2.6 Hz); 7.26 (dd, 1H, J = 10.0 und 2.6 Hz); 7.32-7.40 (m, 2H); 7.90 (br s, 0.2H, Me₂N⁺H-Signal); 11.09 und 11.17 (2 s, 1H).

*¹³C-NMR (DMSO-d₆):* 14.0; 23.3; 24.1; 26.3; 30.7; 31.5; 36.7; 37.4; 55.7; 64.2; 74.7; 103.2 (d, J = 24 Hz); 109.2 (d, J = 27 Hz); 112.3 (d, J = 9 Hz); 127.2 (d, J = 10 Hz); 133.0; 156.7 (d, J = 231 Hz).

### Beispiel 47:

### 1-Butyl-N,N-dimethyl-4-(((2-(triethylsilyl)-1H-pyrrolo[2,3-b]pyridin-3-yl)methoxy)-methyl)cyclohexanamin (Unpolareres Diastereomer)

In einem Kolben mit Schlenk-Aufsatz wurde ein Gemisch von **Ain-08** (548 mg, 1.5 mmol), 2-Amino-3-iodpyridin (393 mg, 1.78 mmol), [1,3-Bis-(2,6-diisopropylphenyl)imidazol-2-yliden]-3-(chlorpyridyl)palladium(II)-chlorid) (PEPPSI^{™}, 204 mg, 0.3 mmol) und Natriumcarbonat (794 mg, 7.50 mmol) 30 min evakuiert. Anschließend wurde unter einem Argonstrom *N,N-*Dimethylformamid (8 ml), dass vorher 1 h mit Argon gesättigt wurde, zugesetzt. Die Suspension wurde über Nacht bei 120 °C gerührt und danach i. Vak. eingeengt. Der Rückstand wurde wiederholt mit Toluol (3 × 30 ml) versetzt, jeweils wieder i. Vak. eingeengt und in Wasser (20 ml) und Ethylacetat (20 ml) aufgenommen. Die Phasen wurden getrennt und die wässrige Phase mit Ethylacetat (2 × 20 ml) extrahiert. Die vereinigten organischen Phasen wurden mit 1 M Natriumthiosulfat-Lösung (30 ml) und gesättigter Natriumchloridlösung (50 ml) gewaschen und i. Vak. eingeengt. Das Rohprodukt (966 mg) wurde durch Flashchromatographie (100 g, 20 × 3.6 cm) mit Ethylacetat / Methanol (9:1) gereinigt.

### Ausbeute: 475 mg (70 %), gelbes Öl

*¹H-NMR (DMSO-d₆):* 0.82-0.94 (m, 18H); 1.20-1.56 (m, 13H); 1.65 (br d, 2H, J = 8.5 Hz); 2.09 (s, 6 H); 3.24 (d, 2H, J = 6.0 Hz); 4.58 (s, 2H); 7.04 (dd, 1 H, J = 4.6 und 7.8 Hz); 7.96 (dd, 1 H, J = 1.4 und 7.8 Hz); 8.22 (dd, 1 H, J = 1.5 und 4.6 Hz); 11.28 (s, 1H).

*¹³C-NMR (DMSO-d₆):* 3.0; 7.2; 14.0; 23.3; 24.1; 26.3; 26.5; 30.7; 31.5; 36.6; 37.5; 55.6; 60.0; 64.3; 75.1; 115.0; 120.4; 126.7; 134.4; 143.0; 150.7.

### Beispiel 48:

### 4-(((1H-Pyrrolo[2,3-b]pyridin-3-yl)methoxy)methyl)-1-butyl-N,N-dimethylcyclohexan-amin (Unpolareres Diastereomer)

Eine Suspension von Benzyltrimethylammoniumfluorid-Monohydrat (468 mg, 2.49 mmol) und aktiviertem Molsieb 4 Å (2 g) in wasserfreiem Tetrahydrofuran (20 ml) wurde 1 h bei Raumtemperatur gerührt. Anschließend wurde dem Gemisch eine Lösung von Beispiel 47 (396 mg, 0.86 mmol) in wasserfreiem Tetrahydrofuran (10 ml) zugesetzt und die Suspension 2 h unter Rückfluss gerührt. Das Gemisch wurde danach filtriert und der Filterrückstand mit Tetrahydrofuran (2 × 35 ml) gewaschen. Das Filtrat wurde i. Vak. eingeengt und das Rohprodukt (398 mg) durch Flashchromatographie (40 g, 20 × 2.5 cm) mit Methanol gereinigt.

### Ausbeute: 178 mg (60 %), farbloses Öl

*¹H-NMR (DMSO-d₆):* 0.86 (t, 3H, J = 7.1 Hz); 1.06-1.54 (m, 13H); 1.64 (br d, 2H, J = 11.6 Hz); 2.09 (s, 6H); 3.21 (d, 2H, J = 6.3 Hz); 4.57 (s, 2H); 7.05 (dd, 1H, J = 4.7 und 7.8 Hz); 7.40 (d, 1H, 2.4 Hz); 7.95 (dd, 1H, 1.4 und 7.8 Hz); 8.20 (dd, 1 H, J = 1.4 und 4.7 Hz); 11.49 (s, 1 H).

*¹³C-NMR (DMSO-d₆):* 13.9; 23.3; 23.8; 24.0; 26.3; 30.7; 31.5; 36.6;37.4; 54.8; 55.7; 64.4; 74.8; 111.0; 115.2; 119.1; 124.9; 126.9; 142.6; 148.7.

### Beispiel 49:

### 1-butyl-N,N-dimethyl-4-(((-2-(triethylsilyl)-1H-pyrrolo[3,2-c]pyridin-3-yl)methoxy)-methyl)cyclohexanamin (Unpolareres Diastereomer)

In einem Kolben mit Schlenk-Aufsatz wurde ein Gemisch von Ain-08 (548 mg, 1.50 mmol), 4-Amino-3-iodpyridin (391 mg, 1.78 mmol), [1,3-Bis-(2,6-diisopropylphenyl)imidazol-2-yliden]-3-(chlorpyridyl)palladium(II)-chlorid) (PEPPSI^{™}, 204 mg, 0.30 mmol) und Natriumcarbonat (794 mg, 7.50 mmol) 30 min evakuiert. Anschließend wurde unter einem Argonstrom *N,N*-Dimethylformamid (8 ml), dass vorher 1 h mit Argon gesättigt wurde, zugesetzt. Die Suspension wurde über Nacht bei 120 °C gerührt und danach i. Vak. eingeengt. Der Rückstand wurde wiederholt mit Toluol (3 × 30 ml) versetzt, jeweils wieder i. Vak. eingeengt und dann in Wasser (20 ml) und Ethylacetat (20 ml) aufgenommen. Die Phasen wurden getrennt und die wässrige Phase mit Ethylacetat (2 × 20 ml) extrahiert. Die vereinigten organischen Phasen wurden mit 1 *M* Natriumthiosulfat-Lösung (30 ml) und gesättigter Natriumchloridlösung (50 ml) gewaschen und i. Vak. eingeengt. Das Rohprodukt (940 mg) wurde durch Flashchromatographie (100 g, 20 × 3.6 cm) mit Ethylacetat / Methanol (1:1) gereinigt.

### Ausbeute: 428 mg (62 %), gelbliches Öl

*¹H-NMR (DMSO-d₆):* 0.84-0.95 (m, 18H); 1.10-1.60 (m, 13H); 1.69 (br d, 2H, J = 5.6 Hz); 2.20 (s, 6H); 3.29 (d, 2H, J = 6.2 Hz); 4.65 (s, 2H); 7.35 (d, 1 H, J = 5.6 Hz); 8.14 (d, 1 H, J = 5.6 Hz); 8.86 (s, 1 H); 11.07 (s, 1 H).

*¹³C-NMR (DMSO-d₆):* 3.1; 7.2; 13.9; 23.2; 23.7; 26.1; 30.6; 36.6; 37.0; 64.2; 74.9; 106.6; 121.6; 125.3; 134.8; 140.0; 141.8; 142.0.

### Beispiel 50:

### 4-(((1H-Pyrrolo[3,2-c]pyridin-3-yl)methoxy)methyl)-1-butyl-N,N-dimethylcyclo-hexanamin (Unpolareres Diastereomer)

Eine Suspension von Benzyltrimethylammoniumfluorid-Monohydrat (431 mg, 2.30 mmol) und aktiviertem Molsieb 4 A (2 g) in wasserfreiem Tetrahydrofuran (15 ml) wurde 1 h bei Raumtemperatur gerührt. Anschließend wurde dem Gemisch eine Lösung von Beispiel 49 (349 mg, 0.76 mmol) in wasserfreiem Tetrahydrofuran (20 ml) zugesetzt und die Suspension 2 h unter Rückfluss gerührt. Das Gemisch wurde danach filtriert und der Filterrückstand mit Tetrahydrofuran (2 × 35 ml) gewaschen. Das Filtrat wurde i. Vak. eingeengt und das Rohprodukt (313 mg) durch Flashchromatographie (32 g, 20 × 2.5 cm) mit Methanol gereinigt.

### Ausbeute: 160 mg (61 %), farbloses Öl

*¹H-NMR (DMSO-d₆):* 0.87 (t, 3H, J = 7.1 Hz); 1.20-1.60 (m, 13 Hz); 1.66 (br d, 2H, J = 10.5 Hz); 2.14 (s, 6H); 3.24 (d, 2H, J = 6.3 Hz); 4.63 (s, 2H); 7.34 (d, 1 H, J = 5.6 Hz); 7.38 (s, 1H); 8.16 (d, 1 H, J = 5.6 Hz); 8.84 (s, 1 H) 11.37 (s, 1 H).

*¹³C-NMR (DMSO-d₆)*: 14.0; 23.3; 23.9; 26.2; 30.6; 31.2; 36.7; 37.2; 64.1; 74.7; 106.8; 112.1; 123.8; 125.3; 127.1; 128.6; 139.7; 140.1; 142.0.

### Beispiel 51:

### 1-Butyl-4-(((5-fluor-2-(triethylsilyl)-1 H-indol-3-yl)methoxy)methyl)-N-methylcyclo-hexanamin (Unpolareres Diastereomer)

In einem Kolben mit Schlenk-Aufsatz wurde ein Gemisch von **Ain-08** (548 mg, 1.5 mmol), 4-Fluor-2-iodanilin (426 mg, 1.8 mmol), [1,3-Bis-(2,6-diisopropylphenyl)imidazol-2-yliden]-3-(chlorpyridyl)palladium(II)-chlorid) (PEPPSI^{™}, 204 mg, 0.3 mmol) und Natriumcarbonat (793 mg, 7.48 mmol) 30 min evakuiert. Anschließend wurde unter einem Argonstrom *N,N-*Dimethylformamid (8 ml), dass vorher 1 h mit Argon gesättigt wurde, zugesetzt. Die Suspension wurde über Nacht bei 120°C gerührt und danach i. Vak. eingeengt. Der Rückstand wurde wiederholt mit Toluol (3 × 30 ml) versetzt, jeweils wieder i. Vak. eingeengt und in Wasser (20 ml) und Ethylacetat (20 ml) aufgenommen. Die Phasen wurden getrennt und die wässrige Phase mit Ethylacetat (2 × 20 ml) extrahiert. Die vereinigten organischen Phasen wurden mit 1 *M* Natriumthiosulfat-Lösung (30 ml) und gesättigter Natriumchloridlösung (50 ml) gewaschen und i. Vak. eingeengt. Das Rohprodukt (932 mg) wurde durch Flashchromatographie (100 g, 3.6 × 25 cm) mit Ethylacetat / Methanol (9:1) gereinigt. Da kein reines Produkt isoliert werden konnte, wurden die entsprechenden Mischfraktionen (472 mg) durch MPLC [130 g, LiChroprep Si60 (15-25 µm)] 46 × 2.6 cm] mit Ethylacetat / Methanol (9:1) gereinigt.

### Ausbeute: 70 mg (10 %), braunes Öl

*¹H-NMR (DMSO-d₆):* 0.83-0.97 (m, 18H); 1.03-1.29 (m,14H); 1.56 (d, 2H,J = 12.5 Hz); 2.06 (s, 3H); 3.24 (d, 2H, J = 6.2 Hz); 4.55 (s, 2H); 6.92 (dt, 1H, J = 2.5 und 9.1 Hz); 7.25 (dd, 1 H, J = 2.4 und 10.0 Hz); 7.38 (dd, 1H, J = 4.6 und 8.8 Hz); 10.74 (s, 1H).

*¹³C-NMR (DMSO-d₆):* 3.1; 7.2; 14.0; 22.8; 24.1; 24.2; 26.8; 33.1; 37.4; 53.0; 64.4; 74.9; 102.8 (d, J = 23 Hz); 109.6 (d, J = 26 Hz) 112.1 (d, J = 10 Hz); 121.4; 128.7; 135.5; 135.8; 156.7 (d, J = 231 Hz).

### Beispiel 52:

### 1-Butyl-N-methyl-4-(((2-(triethylsilyl)-1H-indol-3-yl)methoxy)methyl)cyclohexanamin (Unpolareres Diastereomer)

In einem Kolben mit Schlenk-Aufsatz wurde ein Gemisch von **Ain-08** (WW318A-unpolares Diastereoisomer) (365 mg, 1.0 mmol), 2-lodanilin (261 mg, 1.2 mmol), [1,3-Bis-(2,6-diisopropylphenyl)imidazol-2-yliden]-3-(chlorpyridyl)palladium(II)-chlorid (PEPPSITM, 136 mg, 0.2 mmol) und Natriumcarbonat (529 mg, 5 mmol) 30 min evakuiert. Danach wurde unter einem Argonstrom N,N-Dimethylformamid (8 ml), dass vorher 1 h mit Argon gesättigt wurde, zugesetzt. Die Suspension wurde über Nacht bei 120 °C gerührt und anschließend i. Vak. eingeengt. Der Rückstand wurde wiederholt mit Toluol (3 × 30 ml) versetzt, jeweils wieder i. Vak. eingeengt und in Wasser (20 ml) und Ethylacetat (20 ml) aufgenommen. Die Phasen wurden getrennt und die wässrige Phase mit Ethylacetat (2 × 20 ml) extrahiert. Die vereinigten organischen Phasen wurden mit 1 M Natriumthiosulfat-Lösung (30 ml) und gesättigter Natriumchloridlösung (50 ml) gewaschen und i. Vak. eingeengt. Das Rohprodukt (378 mg) durch MPLC [130 g, LiChroprep Si60 (15-25 µm), 46 × 2.6 cm,] mit Ethylacetat / Methanol (9:1) gereinigt wurde.

### Ausbeute: 52 mg (7 %)

*¹H-NMR (DMSO-d₆):* 0.82-0.99 (m, 18H); 1.00-1.50 (m, 14H); 1.56 (br d, 2H, J = 12.5 Hz); 2.06 (s, 3H); 3.25 (d, 2H, J = 6.1 Hz); 4.59 (s, 2H); 6.97 (t, 1 H, J = 7.4 Hz); 7.07 (t, 1H, J = 7.4 Hz); 7.40 (d, 1H, J = 8.1 Hz); 7.55 (d, 1H, J = 7.9 Hz); 10.63 (s, 1 H).

*¹³C-NMR (DMSO-d₆):* 3.2; 7.3; 14.0; 22.8; 24.1; 24.2; 26.8; 28.9; 33.1; 37.4; 37.6; 53.0; 64.4; 74.9; 111.3; 118.5; 118.4; 121.3; 121.4; 128.5; 133.3; 138.7.

### Beispiel 53:

### 1-Butyl-N-methyl-4-(((2-(triethylsilyl)-5-(trifluormethyl)-1H-indol-3-yl)methoxy)methyl)-cyclohexanamin (Unpolareres Diastereomer)

In einem Kolben mit Schlenk-Aufsatz wurde ein Gemisch von **Ain-08** (400 mg, 1.09 mmol), 4-Amino-3-iodbenzotrifluorid (372 mg, 1.29 mmol), [1,3-Bis-(2,6-diisopropylphenyl)imidazol-2-yliden]-3-(chlorpyridyl)palladium(II)-chlorid (PEPPSI^{™}, 148 mg, 0.22 mmol) und Natriumcarbonat (579 mg, 5.46 mmol) 30 min evakuiert. Anschließend wurde unter einem Argonstrom *N,N*-Dimethylformamid (10 ml), dass vorher 1 h mit Argon gesättigt wurde, zugesetzt. Die Suspension wurde über Nacht bei 120 °C gerührt und danach i. Vak. eingeengt. Der Rückstand wurde wiederholt mit Toluol (3 × 30 ml) versetzt, jeweils wieder i. Vak. eingeengt und in Wasser (20 ml) und Ethylacetat (20 ml) aufgenommen. Die Phasen wurden getrennt und die wässrige Phase mit Ethylacetat (2 × 20 ml) extrahiert. Die vereinigten organischen Phasen wurden mit 1 *M* Natriumthiosulfatlösung (30 ml) und gesättigter Natriumchloridlösung (50 ml) gewaschen, i. Vak. eingeengt und das Rohprodukt (709 mg) durch Flashchromatographie (100 g, 25 × 3.6 cm) mit Ethylacetat / Methanol (9:1) gereinigt.

### Ausbeute: 177 mg (30 %), gelbliches Öl

*¹H-NMR (DMSO-d₆):* 0.82-1.00 (m, 18H); 1.12-1.52 (m, 14H); 1.71 (br d, 2H, J = 11.5 Hz); 2.27 (s, 3H); 3.30 (d, 2H, J = 6.5 Hz, überlagert vom HDO-Signal); 4.66 (s, 2H); 7.37 (dd, 1 H, J = 1.6 und 8.6 Hz); 7.58 (d, 1 H, J = 8.6 Hz); 7.92 (s, 1 H); 11.12 (s, 1 H).

### Beispiel 54:

### 1-Butyl-N-methyl-4-(((5-(trifluormethyl)-1 H-indol-3-yl)methoxy)methyl)cyclohexanamin (Unpolareres Diastereomer)

Eine Suspension von Benzyltrimethylammoniumfluorid-Monohydrat (176 mg, 0.93 mmol) und aktiviertem Molsieb 4 Å (2 g) in wasserfreiem Tetrahydrofuran (20 ml) wurde 1 h Stunde bei Raumtemperatur gerührt. Anschließend wurde dem Gemisch eine Lösung von Beispiel 53 (163 mg, 0.32 mmol) in wasserfreiem Tetrahydrofuran (20 ml) zugesetzt und die Suspension 2 h unter Rückfluss gerührt. Das Gemisch wurde filtriert und der Filterrückstand mit Tetrahydrofuran (2 × 35 ml) gewaschen. Das Filtrat wurde i. Vak. eingeengt und das Rohprodukt (86 mg) durch Flashchromatographie (10 g, 20 × 1.1 cm) mit Methanol gereinigt.

### Ausbeute: 76 mg (60 %), gelbes Öl

*¹H-NMR (DMSO-d₆):* 0.83 (t, 3H, J = 7.0 Hz); 1.00 (dt, 2H, J = 13.1 und 3.5 Hz); 1:08-1.45 (m, 11 H); 1.50 (br d, 2H, J = 12.6 Hz); 1.99 (s, 3H); 3.20 (d, 2H, J = 6.2 Hz); 4.60 (s, 2H); 7.35 (dd, 1 H, J = 1.6 und 8.6 Hz); 7.47 (d, 1 H, J = 1.7 Hz); 7.53 (d, 1 H, J = 8.6 Hz); 7.89 (s, 1 H); 11.39 (s, 1 H). 1 H konnte nicht identifiziert werden.

*¹³C-NMR (DMSO-d₆):* 14.0; 22.9; 23.3; 24.24; 24.28; 26.3; 27.0; 31.5; 33.6; 36.5; 37.5; 38.0; 37.7 (d, J = 46.8 Hz); 52.2; 64.1; 74.7; 112.2; 113.3; 116.4; 117.5; 119.5 (q, J = 31 Hz); 124.2; 126.2; 126.7; 126.9; 137.9.

### Beispiel 55:

### 4-(((5-Fluor-2-(triethylsilyl)-1H-indol-3-yl)methoxy)methyl)-N,N-dimethyl-1-(thiophen-2-yl)cyclohexanamin (Eines von zwei möglichen Diastereomeren

Eine entgaste Mischung von **Ain-09** (795 mg, 2 mmol), 4-Fluor-2-iodanilin (567 mg, 2.4 mmol), [1,3-Bis-(2,6-diisopropylphenyl)imidazol-2-yliden]-(3-chlorpyridyl)palladium(II)-chlorid (PEPPSI, 273 mg, 0.4 mmol) und Natriumcarbonat (1.06 g, 10 mmol) in wasser- und sauerstofffreiem *N,N*-Dimethylformamid (10 ml) wurde 18 h bei 100 °C gerührt. Das Lösungsmittel wurde anschließend i. Vak. entfernt und der Rückstand zwischen Wasser und Diethylether (je 20 ml) verteilt. Die Phasen wurden getrennt und die wässrige Phase wurde mit Diethylether (3 × 10 ml) extrahiert. Die vereinigten organischen Phasen wurden mit Wasser und 1 *M* Natriumthiosulfatlösung (je 20 ml) gewaschen, mit Natriumsulfat getrocknet und i. Vak. eingeengt. Das Rohprodukt (1.30 g) wurde durch Flashchromatographie (100 g, 20 × 4.0 cm) mit Ethylacetat / Cyclohexan (1:9) gereinigt.

### Ausbeute: 552 mg (55 %), bräunliches Öl

*¹H-NMR (DMSO-d₆):* 0.82-0.98 (m, 15H); 1.30-1.70 (m, 7H); 1.96 (s, 6H); 2.37 (br d, 2H); 3.25-3.33 (m, 2H); 4.58 (s, 2H); 6.88-7.04 (m, 3H); 7.23-7.31 (m, 1H); 7.35-7.41 (m, 2H); 10.77 (s, 1 H).

*¹³C-NMR (DMSO-d₆):* 3.1; 7.2; 24.4; 34.6; 37.1; 37.4; 58.2; 64.4; 74.7; 102.8 (d, J = 23 Hz); 109.6 (d, J = 27 Hz); 112.1 (d, J = 10 Hz); 121.5; 123.0; 123.8; 126.0; 128.7 (d, J = 10 Hz); 135.3; 135.9; 145.0; 156.7 (d, J = 231 Hz).

### Beispiel 56:

### 4-(((5-Fluor-1H-indol-3-yl)methoxy)methyl)-N,N-dimethyl-1-(thiophen-2-yl)cyclohexan-amin (Eines von zwei möglichen Diastereomeren)

Eine Aufschlämmung von Benzyltrimethylammoniumfluorid-Monohydrat (338 mg, 2 mmol) in wasserfreiem Tetrahydrofuran (30 ml) wurde mit aktiviertem Molsieb 4 Å (2.00 g) versetzt und 1 h bei Raumtemperatur gerührt. Anschließend wurde zu dieser Suspension eine Lösung von **Beispiel 55** (320 mg, 0.64 mmol) in wasserfreiem Tetrahydrofuran (20 ml) gegeben und 2 h unter Rückfluss gerührt. Das Reaktionsgemisch wurde filtriert, der Filterrückstand mit Tetrahydrofuran gewaschen, das Filtrat i. Vak. eingeengt und der Rückstand (320 mg) durch Flashchromatographie (18 g, 20 × 2.0 cm) mit Ethylacetat / Cyclohexan (2:1) gereinigt.

### Ausbeute: 126 mg (51 %), gelbliches Öl

*¹H-NMR (DMSO-d₆):* 1.30-1.70 (m, 7H); 1.97 (s, 6H); 2.38 (br d, 2H, J = 11.1 Hz); 3.26 (d, 2H, J = 6.3 Hz); 4.57 (s, 2H); 6.88-6.97 (m, 2H); 7.02 (dd, 1H, J = 5.1, 3.5 Hz); 7.28 (dd, 1H, J = 9.9, 2.6 Hz); 7.33-7.41 (m, 3H); 11.09 (s, 1H).

*¹³C-NMR (DMSO-d₆):* 24.4; 34.6; 37.0; 37.4; 58.3; 64.2; 74.3; 103.3 (d, J = 23 Hz); 109.2 (d, J = 26 Hz); 112.3 (d, J = 5 Hz); 112.4, 123,0; 123.8; 126.0; 126.6; 127.3 (d, J = 10 Hz); 132.9; 145.1; 156.7 (d, J = 231 Hz).

### Beispiel 57:

### 3-(((4-(Azetidin-1-yl)-4-phenylcyclohexyl)methoxy)methyl)-5-fluor-2-(triethylsilyl)-1H-indol (Eines von zwei möglichen Diastereomeren)

Eine Mischung von **Ain-10** (795 mg, 2 mmol), 4-Fluor-2-iodanilin (567 mg, 2.4 mmol), [1,3-Bis-(2,6-diisopropylphenyl)imidazol-2-yliden]-(3-chlorpyridyl)palladium(II)-chlorid (PEPPSI, 273 mg, 0.4 mmol) und Natriumcarbonat (1.06 g, 10 mmol) in wasserfreiem *N,N-*Dimethylformamid (10 ml) wurde 18 h bei 100 °C gerührt. Das Lösungsmittel wurde anschließend i. Vak. entfernt und der Rückstand zwischen Wasser und Diethylether (je 20 ml) verteilt. Die wässrige Phase wurde mit Diethylether (3 × 10 ml) und die organische Phase mit Wasser und 1 *M* Natriumthiosulfatlösung (je 20 ml) gewaschen. Die vereinigten organischen Phasen wurden mit Natriumsulfat getrocknet und i. Vak. eingeengt. Das Rohprodukt (1.30 g) wurde durch Flashchromatographie (100 g, 20 × 4.0 cm) mit Ethylacetat / Cyclohexan (1:9) gereinigt.

### Ausbeute: 707 mg (70 %), braunes Öl

*¹H-NMR (DMSO-d₆):* 0.85-0.99 (m, 15H); 1.20-1.34 (m, 2H); 1.43-1.54 (m, 5H); 1.55-1.64 (m, 2H); 2.18 (d, 2H, J = 13.0 Hz); 2.82 (t, 4H, J = 6.8 Hz); 3.32-3.35 (m, 2H); 4.59 (s, 2H); 6.93 (dt, 1H, J = 9.2, 2.5 Hz); 7.23-7.41 (m, 7H); 10.77 (s, 1 H).

*¹³C-NMR (DMSO-d₆):* 3.1; 7.2; 15.5; 24.9; 26.3; 28.9; 30.4; 36.8; 45.8; 57.2; 64.4; 74.7; 102.8 (d, J = 22 Hz); 109.6 (d, J = 22 Hz); 112.2 (d, J = 10 Hz); 121.5 (d, J = 5 Hz); 126.3; 126.6; 127.4; 128.7 (d, J = 10 Hz); 135.3; 135.9; 140.2; 156.8 (d, J = 232 Hz).

### Beispiel 58:

### 3-(((4-(Azetidin-1-yl)-4-phenylcyclohexyl)methoxy)methyl)-5-fluor-1H-indol (Eines von zwei möglichen Diastereomeren)

Eine Aufschlämmung von Benzyltrimethylammoniumfluorid-Monohydrat (148 mg, 0.84 mmol) in wasserfreiem Tetrahydrofuran (20 ml) wurde mit aktiviertem Molsieb (2.00 g) versetzt und 1 h bei Raumtemperatur gerührt. Anschließend wurde zu dieser Suspension eine Lösung von **Beispiel 57** (142 mg, 0.28 mmol) in wasserfreiem Tetrahydrofuran (20 ml) gegeben und 1 h unter Rückfluss gerührt. Das Reaktionsgemisch wurde anschließend filtriert, der Filterrückstand mit Tetrahydrofuran gewaschen und das Filtrat i. Vak. eingeengt. Der Rückstand (73 mg) wurde durch Flashchromatographie (10 g, 20 × 1.5 cm) mit Ethylacetat / Methanol (9:1) gereinigt.

### Ausbeute: 38 mg (34 %), gelbliches Öl

*¹H-NMR (CDCl₃):* 1.48-1.78 (m, 9H); 2.09-2.16 (m, 2H); 3.01 (br s, 4H); 3.46 (d, 2H, J = 6.4 Hz); 4.67 (s, 2H); 6.94 (dt, 1H, J = 9.1, 2.5 Hz); 7.19 (d, 1H, J = 2.5 Hz); 7.23-7.43 (m, 7H); 8.42 (s, 1 H).

*¹³C-NMR (CDCl₃):* 16.1; 24.8; 30.0; 36.3; 46.6; 58.9; 65.1; 74.5; 104.2; 104.3 (d, J = 24 Hz); 110.5 (d, J = 27 Hz); 111.7 (d, J = 10 Hz); 113.8 (d, J = 5 Hz); 125.4; 126.8; 127.2; 127.7; 133.0; 139.4; 157.9 (d, J = 235 Hz).

### Beispiel 60:

### 4-(((5-Fluor-1-methyl-2-(triethylsilyl)-1H-indol-3-yl)methoxy)methyl)-N,N-dimethyl-1-phenylcyclohexanamin (Eines von zwei möglichen Diastereomeren)

Eine Mischung von **Ain-01** (578 mg, 1.5 mmol), [1,3-Bis-(2,6-diisopropylphenyl)imidazol-2-yliden]-(3-chlorpyridyl)palladium(II)-chlorid (PEPPSI, 204 mg, 0.3 mmol), 4-Fluor-2-iod-N-methylaniline **(lan-11)** (594 mg, 1.9 mmol) und Natriumcarbonat (793 mg, 7.5 mmol) wurde 30 min an der Ölpumpe evakuiert. Anschließend wurde über einen Schlenk-Aufsatz wasserfreies *N,N*-Dimethylformamid (8 ml), dass zuvor 1 h mit Argon gespült wurde, zugesetzt. Das Reaktionsgemisch wurde danach 18 h bei 100 °C gerührt und anschließend i. Vak. eingeengt. Der Rückstand wurde wiederholt mit Toluol versetzt, jeweils wieder i. Vak. eingeengt und dann zwischen Wasser und Ethylacetat (jeweils 20 ml) verteilt. Die Phasen wurden getrennt und die wässrige Phase wurde mit Ethylacetat (3 × 30 ml) extrahiert. Die vereinigten organischen Phasen wurden mit 1 *M* Natriumthiosulfatlösung (30 ml) und gesättigter Natriumchloridlösung (50 ml) gewaschen. Die vereinigten organischen Phasen wurden mit Natriumsulfat getrocknet und i. Vak. eingeengt. Das Rohprodukt (965 mg) wurde durch Flashchromatographie (100 g, 20 × 3.7 cm) mit Dichlormethan / Methanol (95:5) gereinigt.

### Ausbeute: 156 mg (21 %), braunes Öl

*¹H-NMR (DMSO-d₆):* 0.86-1.04 (m, 14H); 1.20-1.70 (m, 9H); 1.88 (br s, 6H); 2.55 (br d, 1H, J = 12.0 Hz); 3.34 (d, 2H, überlagert vom HDO-Signal); 3.82 (s, 3H); 4.57 (s, 2H); 7.02 (dt, 1 H, J = 2.5, 9.2 Hz); 7.23 (br d, 1 H, J = 4.4 Hz); 7.26-7.40 (m, 5H); 7.45 (dd, 1 H, J = 4.4, 8.9 Hz).

*¹³C-NMR (DMSO-d₆):* 3.6; 7.2; 24.4; 32.0; 33.2; 37.1; 37.3; 54.7; 58.4; 63.4; 74.5; 102.9 (d, J = 23 Hz); 109.9 (d, J = 25 Hz); 110.6 (d, J = 10 Hz); 122.6; 126.0; 126.4; 127.2; 128.6 (d, J = 10 Hz); 136.0; 137.8; 139.4; 157.0 (d, J = 231 Hz).

### Beispiel 61:

### 4-(((5-fluor-1-methyl-1H-indol-3-yl)methoxy)methyl)-N,N-dimethyl-1-phenylcyclohexanamin (Eines von zwei möglichen Diastereomeren)

Eine Suspension von Benzyltrimethylammoniumfluorid-Monohydrat (251 mg, 1.35 mmol) und Molsieb 4 Å (2.0 g) in wasserfreiem Tetrahydrofuran (50 ml) wurde 1 h bei Raumtemperatur gerührt. Anschließend wurde zu dieser Suspension eine Lösung von Beispiel 60 (220 mg, 0.432 mmol) in wasserfreiem Tetrahydrofuran (60 ml) zugesetzt. Das Gemisch wurde 6 h bei 75 °C und anschließend über Nacht bei Raumtemperatur gerührt. Die Suspension wurde filtriert, der Filterrückstand mit Tetrahydrofuran (2 × 20 ml) gewaschen und das Filtrat i. Vak. eingeengt. Das Rohprodukt (236 mg) wurde durch Flashchromatographie (18 g, 20 × 1.6 cm) mit Dichlormethan / Methanol (95:5) gereinigt.

### Ausbeute: 104 mg (61 %), gelbliches Öl

*¹H-NMR (DMSO-d₆):* 1.26-1.70 (m, 7H); 1.90 (s, 6H); 2.55 (brd, 2H, J = 11.6 Hz); 3.29 (d, 2H, J = 6.4 Hz); 3.76 (s, 3H); 4.56 (s, 2H); 7.00 (dt, 1H, J = 2.6, 9.2 Hz); 7.22 (m, 1H); 7.28-7.36 (m, 5H); 7.38 (s, 1 H); 7.42 (dd, 1 H, J = 4.4, 8.9 Hz).

*¹³C-NMR (DMSO-d₆):* 24.5; 32.0; 32.5; 36.8; 37.3; 58.6; 64.0; 74.2; 103.6 (d, J = 23 Hz); 109.3 (d, J = 26 Hz); 110.7 (d, J = 10 Hz); 111.2; 111.4; 126.0; 126.4; 127.1; 127.5; 127.6; 130.6; 133.4; 139.4; 156.8 (d, J = 231 Hz).

### Beispiel 62:

### 4-(((5-Fluor-1-(methylsulfonyl)-1H-indol-3-yl)methoxy)methyl)-N,N-dimethyl-1-phenylcyclohexanamin (Eines von zwei möglichen Diastereomeren)

Eine Suspension von Benzyltrimethylammoniumfluorid-Monohydrat (760 mg, 4.10 mmol) und Molsieb 4 Å (4.0 g) in wasserfreiem Tetrahydrofuran (60 ml) wurde 1 h bei Raumtemperatur gerührt. Anschließend wurde zu dieser Suspension eine Lösung von **Beispiel 17** (760 mg, 1.33 mmol) in wasserfreiem Tetrahydrofuran (60 ml) zugesetzt. Das Gemisch wurde 1 h bei 40 °C und anschließend über Nacht bei Raumtemperatur gerührt. Die Suspension wurde filtriert, der Filterrückstand mit Tetrahydrofuran (2 × 20 ml) gewaschen und das Filtrat i. Vak. eingeengt. Das Rohprodukt (780 mg) wurde durch Flashchromatographie (85 g, 3.6 × 20 cm) mit Cyclohexen / Ethylacetat (1:2) gereinigt.

### Ausbeute: 409 mg (67 %), gelbliches Öl

*¹H-NMR (DMSO-d₆):* 1.22-1.70 (m, 7H); 1.89 (s, 6H); 2.58 (d, 2H, J = 12.4 Hz); 3.35 (d, 2H, J = 6.3 Hz); 3.44 (s, 3H); 4.62 (s, 2H); 7.18-7.27 (m, 2H); 7.28-7.40 (m, 4H); 7.47 (dd, 1H. J = 2.5, 9.2 Hz); 7:65 (s, 1H); 7.84 (dd, 1H, J = 4.5, 9.1 Hz).

*¹³C-NMR (DMSO-d₆):* 24.4; 32.0; 36.8; 37.3; 58.4; 63.6; 75.0; 105.6 (d, J = 24 Hz); 112.5 (d, J = 26 Hz); 114.4 (d, J = 9 Hz); 118.6; 118.7; 126.0; 126.4; 126.5; 127.2; 130.5 (d, J = 10 Hz); 131.1; 139.4; 158.6 (d, J = 237 Hz).

### Beispiel 63:

### 1-(3-(((4-(Dimethylamino)-4-phenylcyclohexyl)methoxy)methyl)-5-fluor-1H-indol-1-yl)ethanon (Eines von zwei möglichen Diastereomeren)

Eine Suspension von Benzyltrimethylammoniumfluorid-Monohydrat (108 mg, 0.58 mmol) und Molsieb 4 Å (2.0 g) in wasserfreiem Tetrahydrofuran (30 ml) wurde 1 h bei Raumtemperatur gerührt. Anschließend wurde zu dieser Suspension eine Lösung von **Beispiel 16** (100 mg, 0.18 mmol) in wasserfreiem Tetrahydrofuran (30 ml) zugesetzt. Das Gemisch wurde 4 h bei 40 °C und anschließend über Nacht bei Raumtemperatur gerührt. Die Suspension wurde filtriert, der Filterrückstand mit Tetrahydrofuran (2 × 20 ml) gewaschen und das Filtrat i. Vak. eingeengt. Das Rohprodukt (110 mg) wurde durch Flashchromatographie (18 g, 18 × 1.6 cm) mit Dichlormethan / Methanol (95:5) gereinigt.

### Ausbeute: 37 mg (47 %), gelbliches Öl

*¹H-NMR (CDCl₃):* 1.45-1.82 (m, 7H); 2.04 (s, 6H); 2.45-2.58 (m, 2H); 2.62 (s, 3H); 3.47 (d, 2H, J = 6.7 Hz); (d, 2H, J = 0.8 Hz); 7.08 (dt, 1H, J = 2.6, 9.1 Hz), 7.22-7.40 (m, 6H); 7.43 (s, 1 H); 8.44 (dd, 1 H, J = 4.7, 9.1 Hz).

*¹³C-NMR (CDCl₃):* 23.5; 24.7; 29.7; 32.0; 36.8; 37.6; 58.3; 60.2; 64.7; 75.5; 105.4 (d, J = 24 Hz); 113.0 (d, J = 25 Hz); 117.7 (d, J = 9 Hz); 119.8; 124.9; 126.4; 126.9; 127.3; 130.7 (d, J = 10 Hz); 132.4; 159.7 (d, J = 241 Hz); 168.1.

### Beispiel 64:

### 4-(((2-tert-Butyl-5-fluor-1H-indol-3-yl)methoxy)methyl)-N,N-dimethyl-1-phenylcyclo-hexanamin (Eines von zwei möglichen Diastereomeren)

Eine Mischung von **Ain-13** (, (522 mg, 1.60 mmol), 4-Fluor-2-iodanilin **(lan-04)** (453 mg, 1.90 mmol), [1,3-Bis(2,6-diisopropylphenyl)imidazol-2-ylidene]-3-chlorpyridyl-palladium(II)-chlorid (PEPPSI, 109 mg, 0.16 mmol) und Natriumcarbonat (848 mg, 8.0 mmol) wurde 30 min evakuiert (Ölpumpe). Anschließend wurde mit Argon gespült und über einen Schlenk-Aufsatz absolutes *N,N*-Dimethylformamid (5 ml, vorher 1 h mit Argon gespült) per Spritze zugegeben. Das Reaktionsgemisch wurde 18 h bei 100 °C gerührt, wobei sich die Lösung dunkelbraun färbte. Die Reaktionslösung wurde anschließend i. Vak. eingeengt, der Rückstand mehrfach in Toluol aufgenommen (3 × 30 ml) und jeweils wieder eingeengt, dann zwischen Wasser und Ethylacetat (je 50 ml) verteilt und die Phasen getrennt. Die wässrige Phase wurde mit Ethylacetat (2 × 50 ml) extrahiert, die vereinigten organischen Phasen wurden mit 1 *M* Natriumthiosulfat-Lösung und gesättigter Natriumchlorid-Lösung (je 50 ml) gewaschen, mit Natriumsulfat getrocknet und i. Vak. eingeengt. Das Rohprodukt (948 mg) wurde mittels Flashchromatographie (38 g, 20 × 2.5 cm) mit Chloroform / Methanol (95:5) gereinigt.

### Ausbeute: 421 mg (60 %)

*Schmelzpunkt:* 51-53 °C

*¹H-NMR (DMSO-d₆):* 1.19-1.68 (m, 7H); 1.44 (s, 9H); 1.89 (s, 6H); 2.54-2.64 (m, 2H); 3.29-3.31 (m, 2H); 4.64 (s, 2H); 6.84 (dt, 1H, J = 2.5, 9.6 Hz); 7.23 (dd, 2H, J = 2.5, 10.2 Hz); 7.27 (d, 1H, J = 4.7 Hz); 7.29-7.37 (m, 4H); 10.79 (s, 1 H).

*¹³C-NMR (DMSO-d₆):* 24.5; 30.3; 32.1; 33.2; 37.0; 37.4; 58.5; 63.2; 74.5; 102.2 (d, 1 C, J = 23 Hz); 106.6; 108.1 (d, 1C, J = 26 Hz); 111.6 (d, 1C, J = 10 Hz); 126.1; 126.5; 127.2; 129.8 (d, 1C, J = 10 Hz); 130.7; 139.4; 147.5; 156.9 (d, 1C, J = 231 Hz). Weitere C-Signale konnten nicht identifiziert werden.

### Beispiel 65:

### 3-(((4-(Azetidin-1-yl)-4-phenylcyclohexyl)methoxy)methyl)-2-(triethylsilyl)-1H-pyrrolo[2,3-b]pyridin (Eines von zwei möglichen Diastereomeren)

Eine Mischung von **Ain-10** (473 mg, 1.19 mmol), 2-Amino-3-iodpyridin **(Ian-05)** (lan-0(308 mg, 1.4 mmol), [1,3-Bis-(2,6-diisopropylphenyl)imidazol-2-yliden]-(3-chlorpyridyl)palladium(II)-chlorid (PEPPSI, 162 mg, 0.24 mmol) und Natriumcarbonat (630 mg, 5.9 mmol) in sauerstoff- und wasserfreiem *N,N*-Dimethylformamid (10 ml) wurde 18 h bei 100 °C gerührt. Das Lösungsmittel wurde anschließend i. Vak. entfernt und der Rückstand zwischen Wasser und Diethylether (je 20 ml) verteilt. Die wässrige Phase wurde mit Diethylether (3 × 10 ml) und die organische Phase mit Wasser und 1 *M* Natriumthiosulfatlösung (je 20 ml) gewaschen. Die vereinigten organischen Phasen wurden mit Natriumsulfat getrocknet und i. Vak. eingeengt. Das Rohprodukt (700 mg) wurde durch Flashchromatographie (18 g, 20 × 2.0 cm) mit Ethylacetat / Cyclohexan (1:2) gereinigt.

### Ausbeute: 346 mg (59 %), gelbliches Öl

*¹H-NMR (DMSO-d₆):* 0.90-0.97 (m, 15H); 1.20-1.32 (m, 2H); 1.42-1.54 (m, 5H); 1.59 (quin, 2H, J = 6.8 Hz); 2.17 (d, 2H, J = 13.1 Hz); 2.81 (t, 4H, J = 6.8 Hz); 3.33 (d, 2H, J = 5.1 Hz); 4.62 (s, 2H); 7.05 (dd, 1 H; J = 7.8, 4.6 Hz); 7.22-7.31 (m, 3H); 7.34-7.40 (m, 2H); 7.99 (dd, 1H, J = 7.8, 1.3 Hz); 8.23 (dd, 1H, J = 4.6, 1.6 Hz); 11.28 (s, 1 H).

*¹³C-NMR (DMSO-d₆):* 3.0; 7.2; 15.5; 24.9; 26.3; 30.4; 36.8; 45.8; 57.2; 64.4; 74.8; 115.0; 120.4; 126.2; 126.6; 126.7; 127.4; 134.4; 140.2; 143.0; 150.7.

### Beispiel 66:

### 3-(((4-(Azetidin-1-yl)-d-phenylcyclohexyl)methoxy)methyl)-1H-pyrrolo[2,3-b]pyridin (Eines von zwei möglichen Diastereomeren)

Eine Aufschlämmung von Benzyltrimethylammoniumfluorid-Monohydrat (232 mg, 1.37 mmol) in wasserfreiem Tetrahydrofuran (20 ml) wurde mit aktiviertem Molsieb 4 Å (2.00 g) versetzt und 1 h bei Raumtemperatur gerührt. Anschließend wurde zu dieser Suspension eine Lösung von **Beispiel 65** (224 mg, 0.45 mmol) in wasserfreiem Tetrahydrofuran (20 ml) gegeben und 2 h unter Rückfluss gerührt. Das Reaktionsgemisch wurde anschließend filtriert, der Filterrückstand mit Tetrahydrofuran gewaschen und das Filtrat i. Vak. eingeengt. Der Rückstand (220 mg) wurde durch Flashchromatographie (10 g, 20 × 1.5 cm) mit Ethylacetat / Methanol (9:2) gereinigt.

### Ausbeute: 59 mg (35 %)

*¹H-NMR (CDCl₃):* 1.40-1.75 (m, 9H); 2.12-2.20 (m, 2H); 2.96 (br s, 4H); 3.44 (d, 2H, J = 6.3 Hz); 4.72 (s, 2H); 7.12 (dd, 1 H, J = 7.8, 4.8 Hz); 7.24-7.43 (m, 6H); 8.07 (dd, 1 H, J = 7.8, 1.5 Hz); 8.34 (dd, 1H, J = 4.7, 1.5 Hz); 10.32 (s, 1 H).

*¹³C-NMR (CDCl₃):* 16.1; 25.0; 30.4; 36.8; 46.5; 65.1; 65.3; 75.0; 112.3; 115.8; 119.9; 124.0; 126.5; 127.1; 127.6; 128.0; 128.2; 143.0; 149.2.

### Beispiel 67:

### 4-(((2-tert-Butyl-1H-pyrrolo[2,3-b]pyridin-3-yl)methoxy)methyl)-N,N-dimethyl-1-phenylcyclohexanamin (Eines von zwei möglichen Diastereomeren)

Eine Mischung von **Ain-13** (347 mg, 1.06 mmol), 2-Amino-3-iodpyridin **(Ian-05)** (280 mg, 1.27 mmol), [1,3-Bis(2,6-diisopropylphenyl)imidazol-2-yliden]-(3-chlorpyridyl)palladium(II)-chlorid (PEPPSI, 72 mg, 0.11 mmol) und Natriumcarbonat (562 mg, 5.3 mmol) wurde 30 min evakuiert (Ölpumpe). Anschließend wurde mit Argon gespült und über einen Schlenk-Aufsatz absolutes *N,N*-Dimethylformamid (5 ml, vorher 1 h mit Argon gespült) per Spritze zugegeben. Das Reaktionsgemisch wurde 18 h bei 100 °C gerührt, wobei es sich dunkelbraun färbte. Die Reaktionslösung wurde anschließend i. Vak. eingeengt, der Rückstand mehrfach in Toluol aufgenommen (3 × 30 ml) und jeweils wieder eingeengt, dann zwischen Wasser und Ethylacetat (je 50 ml) verteilt und die Phasen getrennt. Die wässrige Phase wurde mit Ethylacetat (2 × 50 ml) extrahiert, die vereinigten organischen Phasen wurden mit 1 *M* Natriumthiosulfat-Lösung und gesättigter Natriumchlorid-Lösung (je 50 ml) gewaschen, mit Natriumsulfat getrocknet und i. Vak. eingeengt. Das Rohprodukt (581 mg) wurde mittels Flashchromatographie (38 g, 20 × 2.5 cm) mit Chloroform / Methanol (95:5) gereinigt.

### Ausbeute: 77 mg (17 %)

*Schmelzpunkt:* 67-70 °C

*¹H-NMR (DMSO-d₆):* 1.21-1.44 (m, 6H); 1.46 (s, 9H); 1.48-1.55 (m, 1 H); 1.56-1.65 (m, 1 H); 1.89 (br s, 6H); 2.52-2.60 (m, 1 H); 3.31-3.33 (m, 2H); 4.67 (s, 2H); 7.02 (dd, 1H, J = 4.7, 7.8 Hz); 7.22 (sehr br s, 1H), 7.31 (sehr br s, 4H); 7.90 (dd, 1H, J = 1.5, 7.8 Hz); 8.14 (dd, 1H, J = 1. 5, 4.7 Hz); 11.30 (s, 1 H).

*¹³C-NMR (DMSO-d₆):* 24.5; 30.2; 32.0; 33.5; 37.0; 37.4; 58.5; 62.9; 74.5; 79.1; 104.8; 115.1; 121.6; 125.4; 126.1; 126.5; 127.2; 141.6; 146.2; 147.2.

### Beispiel 68: (nicht erfindungsgemäß)

### N,N-Dimethyl-1-phenyl-4-(((1-(phenylsulfonyl)-1H-indol-5-yl)methoxy)methyl)cyclohexanamin (Eines von zwei möglichen Diastereomeren)

### Stufe 1: 1-Benzolsulfonyl-5-methyl-1H-indol

Zu einer Lösung von 5-Methylindol (5.0 g, 38 mmol) in wasserfreiem Tetrahydrofuran (60 mL) wurde bei 0 °C innerhalb von 30 min eine 60 % Dispersion von Natriumhydrid in Mineralöl (1.55 g, 38.9 mmol) gegeben. Die entstandene Suspension wurde 1 h bei Raumtemperatur gerührt, anschließend tropfenweise mit Benzolsulfonylchlorid (6.89 g, 5.0 mL, 38.9 mmol) versetzt und über Nacht bei Raumtemperatur gerührt. Das Reaktionsgemisch wurde danach mit 5 % Natriumhydrogencarbonat-Lösung (200 mL) versetzt und mit Diethylether (3 × 80 mL) extrahiert. Die vereinigten organischen Phasen wurden mit Natriumsulfat getrocknet und i. Vak. eingeengt. Das Rohprodukt (10.4 g) wurde mittels Flashchromatographie mit Ethylacetat / Cyclohexan (1:9) gereinigt.

### Ausbeute: 9.1 g (88 %), hellbraunes Öl

¹H-NMR (DMSO-d₆): 2.34 (s, 3H); 6.76 (dd, 1H, J =0.8, 3.7 Hz); 7.13-7.18 (m, 1H); 7.37 (m, 1H); 7.53-7.71 (m, 3H); 7.74 (d, 1H, J = 3.7 Hz); 7.81 (d, 1 H, J = 8.5 Hz); 7.91-7.96 (m, 2H).

### Stufe 2: 1-Benzolsulfonyl-5-brommethyl-1H-indol

Eine Lösung von 1-Benzolsutfonyl-5-methyl-1*H*-indol (7.77 g, 28.6 mmol) in Tetrachlormethan (230 mL) wurde unter Rückfluss mit Azoisobutyronitril (50 mg) und *N-*Bromsuccinimid (6.10 g, 34.3 mmol) versetzt und weitere 5 h unter Rückfluss erhitzt. Der ausgefallene Niederschlag wurde abfiltriert und das Filtrat i. Vak. eingeengt. Der Rückstand wurde mittels Flashchromatographie (570 g, 35 × 6.8 cm) mit Ethylacetat / Cyclohexan (1:9) gereinigt.

### Ausbeute: 3.29 g (33 %), weißer Feststoff

¹H-NMR (DMSO-d₆): 4.79 (s, 2H); 6.85 (dd, 1H, J = 0.7, 3.7 Hz); 7.42 (dd, 1H, J = 1.8, 8.6 Hz); 7.56-7.62 (m, 2H); 7.67-7.72 (m, 2H); 7.85 (d, 1H, J = 3.7 Hz); 7.89-7.95 (m, 1H); 7.97-8.00 (m, 2H).

### Stufe 3: 1-Benzolsulfonyl-5-(1,4-dioxaspiro[4.5]dec-8-ylmethoxymethyl)-1H-indol

Ein Gemisch von1-Benzolsulfonyl-5-brommethyl-1 H-indol 1.70 g, 4.85 mmol); (1,4-Dioxaspiro[4.5]dec-8-yl)methanol (1.25 g, 7.27 mmol), *N,N,N',N*'-Tetramethyt-1,8-naphthalindiamin (1.04 g, 4.85 mmol) und Kupfer(II)-acetylacetonat (64 mg, 0.24 mmol) in p-Xylol (20 mL) wurde 21 h bei 140 °C gerührt. Anschließend wurde das Lösungsmittel i. Vak. entfernt und der Rückstand durch Flaschchromatographie (230 g, 27 × 5.2 cm) mit Ethylacetat / Cyclohexan (1:4) gereinigt.

### Ausbeute: 430 mg (20 %), gelbes dickflüssiges Öl

¹H-NMR (DMSO-d₆): 1.09-1.22 (m, 2H); 1.36-1.46 (m, 2H); 1.52-1.71 (m, 5H); 3.25 (d, 2H, J = 6.40 Hz); 3.82 (s, 4H); 4.48 (s, 2H); 6.84 (dd, 1H, J = 0.8, 3.7 Hz); 7.29 (dd, 1H, J = 1.6, 8.6 Hz); 7.52-7.54 (m, 1H); 7.55-7.61 (m, 2H); 7.65-7.71 (m, 1H); 7.79 (d, 1H, J = 3.7 Hz); 7.89-7.93 (m, 1 H); 7.94-7.99 (m, 2H).

### Stufe 4: 4-[1-Benzolsulfonyl-1H-indol-5-ylmethoxymethyl)cyclohexanon

Eine Lösung von 1-Benzolsulfonyl-5-(1,4-dioxaspiro[4.5]dec-8-ylmethoxymethyl)-1*H*-indol (212 mg, 0.48 mmol) in Tetrahydrofuran (2.5 mL) wurde mit 2 *N* Salzsäure (500 µL) versetzt und 5 h bei 60 °C gerührt. Das Reaktionsgemisch wurde anschließend mit Natriumhydrogencarbonat-Lösung auf pH 8 eingestellt. Das Tetrahydrofuran wurde i. Vak. abdestilliert und die wässrige Lösung mit Dichlormethan (2 × 10 mL) extrahiert. Die vereinigten organischen Phasen wurden mit Natriumchloridlösung gewaschen, mit Natriumsulfat getrocknet und i. Vak. eingeengt. Das Rohprodukt (170 mg) wurde mittels Flashchromatographie (18 g, 20 × 2 cm) mit Ethylacetat / Cyclohexan (1:2) gereinigt.

### Ausbeute: 120 mg (63 %), bräunliches Öl

¹H-NMR (DMSO-d₆): 1.27-1.44 (m, 2H); 1.90-2.10 (m, 3H); 2.11-2.23 (m, 2H); 2.29-2.43 (m, 2H); 3.35 (d, 2H, J = 6.4 Hz); 4.52 (s, 2H); 6.84 (dd, 1H, J = 0.7, 3.7 Hz); 7.30 (dd, 1H, J = 8.6 Hz); 7.53-7.62 (m, 3H); 7.65-7.72 (m, 1 H); 7.81 (d, 1H, J = 1.5, 3.7 Hz); 7.88-8.00 (m, 3H).

### Stufe 5: 4-[1-Benzolsulfonyl-1H-indol-5-ylmethoxymethyl)-1-dimethylaminocyclohexancarbonitril

Zu einer auf 0 °C gekühlten Lösung von 4 *N* Salzsäure (210 µL, 0.84 mmol) und Methanol (0.5 mL) wurde 40 % wässrige Dimethylaminlösung (420 µL, 3.32 mmol) und anschließend eine Lösung von 4-[1-Benzolsulfonyl-1*H*-indol-5-ylmethoxymethyl)cyclohexanon (330 mg, 0.83 mmol) in Methanol (1 mL) und Tetrahydrofuran (2.5 mL) gegeben. Dieses Gemisch wurde mit Kaliumcyanid (130 mg, 2.0 mmol) versetzt und 5 h bei Raumtemperatur gerührt. Nach Zugabe von Wasser (5 mL) wurde mit Diethylether (3 × 10 mL) extrahiert. Die vereinigten organischen Phasen wurden mit Natriumsulfat getrocknet und i. Vak. eingeengt.

### Ausbeute: 315 mg (84 %), braunes Öl

¹H-NMR (DMSO-d₆): 1.00-2.11 (m, 9H); 2.19 und 2.23 (2s, 6H); 3.19-3.31 (m, 2H); 4.46-4.53 (m, 2H); 6.83-6.86 (m, 1H); 7.26-7.33 (m, 1 H); 7.51-7.78 (m, 4H); 7.79-7.82 (m, 1 H); 7.88-8.00 (m, 3H).

Das Diastereoisomerenverhältnis betrug ca. 2:3.

### Stufe 6: N,N-Dimethyl-1-phenyl-4-(((1-(phenylsulfonyl)-1H-indol-5-yl)methoxy)methyl)cyclo-hexanamin (Eines von zwei möglichen Diastereomeren)

Zu einer eisgekühlten 2 *M* Lösung von Phenylmagnesiumchlorid in Tetrahydrofuran (1.4 mL, 2.8 mmol) wurde unter Argon eine Lösung von 4-[1-Benzolsulfonyl-1*H*-indol-5-ylmethoxymethyl)-1-dimethylaminocyclohexancarbonitril 315 mg, 0.7 mmol) in wasserfreiem Tetrahydrofuran (3 mL) getropft und anschließend 3 d bei Raumtemperatur gerührt. Danach wurde das Reaktionsgemisch mit 20 % Ammoniumchloridlösung (5 mL) versetzt. Die Phasen wurden getrennt und die wässrige mit Diethylether (2 × 10 mL) extrahiert. Die vereinigten organischen Phasen wurden mit Natriumchloridlösung (20 mL) gewaschen, mit Natriumsulfat getrocknet und i. Vak. eingeengt. Das Rohprodukt (432 mg) wurde mittels Flashchromatographie (37 g, 21 × 2.5 cm) mit Ethylacetat / Methanol (9:1) gereinigt.

### Ausbeute: 89 mg (25 %), gelbes Öl

¹H-NMR (CDCl₃): 1.44-1.77 (m, 7H); 2.01 (s, 6H); 2.48 -2.57 (m, 2H); 3.41 (d, 2H, J = 6.6 Hz); 4.56 (s, 2H); 6.65 (dd, 1H, J = 0.7, 3.7 Hz); 7.21-7.58 (m, 11H); 7.85-7.89 (m, 2H); 7.97 (d, 1H, J = 8.5 Hz).

Es wurde nur ein Diastereoisomer isoliert.

### Beispiel 69: (nicht erfindungsgemäß)

### 4-(((1H-Indol-5-yl)methoxy)methyl)-N,N-dimethyl-1-phenylcyclohexanamin (Eines von zwei möglichen Diastereomeren)

Eine Lösung von N,N-Dimethyl-1-phenyl-4-(((1-(phenytsulfonyl)=1 H-indol-5-yl)methoxy)methyl)cyclohexanamin (50 mg, 0.1 mmol) in Methanol (4 mL) wurde mit 2 *N* Natronlauge (1 mL) versetzt und 6.5 h unter Rückfluss gerührt. Anschließend wurde Methanol i. Vak. abdestilliert, der wässrige Rückstand mit Essigsäure auf pH ∼6-7 eingestellt und mit Dichlormethan (2 × 10 mL) extrahiert. Die vereinigten organischen Phasen wurden mit Natriumsulfat getrocknet und i. Vak. eingeengt. Die Lösung des Rohprodukt in Dichlormethan / Methanol wurde mit wässrigem Ammoniak basisch gestellt, i. Vak. eingeengt und getrocknet. Der Rückstand wurde mittels Flashchromatographie (10 g, 17 × 1.5 cm) mit Dichlormethan / Methanol (10:1) gereinigt.

### Ausbeute: 35 mg (96 %), gelbes Öl

¹H-NMR (CDCl₃): 0.81-0.93 (m, 2H); 1.26 (s, 3H); 1.65-1.83 (m, 2H); 2.09 (br s, 6H); 2.46-2.57 (m, 2H); 3.36-3.49 (m, 2H); 4.63 (s, 2H); 6.52-6.55 (m, 1 H); 7.19-7.42 (m, 8H); 7.61-7.63 (m, 1H); 8.26 (s, 1H).

### Beispiel 70: (nicht erfindungsgemäß)

### N,N-Dimethyl-1-phenyl-4-(((1-(phenylsulfonyl)-1H-indol-4-yl)methoxy)methyl)cyclo-hexanamin (Eines von zwei möglichen Diastereomeren)

### Stufe 1: 1-Benzolsulfonyl-4-methyl-1H-indol

Zu einer Lösung von 4-Methylindol (4.80 g, 36.6 mmol) in wasserfreiem Dichlormethan (40 mL) wurde pulverisiertes Natriumhydroxid (2.93 g, 73.2 mmol) und Tetra-*n*-butylammoniumhydrogensulfat (20 mg) gegeben und 1 h bei Raumtemperatur gerührt. Anschließend wurde das Gemisch bei 0 °C innerhalb von 20 min tropfenweise mit Benzolsulfonylchlorid (7.11 g, 5.2 mL, 40.3 mmol) versetzt (heftige Reaktion!) und danach über Nacht bei Raumtemperatur gerührt. Das Reaktionsgemisch wurde dann mit Wasser und Dichlormethan (je 50 mL) versetzt. Die organische Phase wurde abgetrennt, mit Natriumchloridlösung gewaschen, mit Natriumsulfat getrocknet und i. Vak. eingeengt.

### Ausbeute: 4.86 g (49 %), hellgelber Feststoff

¹H-NMR (DMSO-d₆): 2.42 (s, 3H); 6.89 (d, 1H, J = 3.4 Hz); 7.05 (d, 1H, J = 7.3 Hz); 7.23 (t, 1H, J = 7.9 Hz); 7.57 (t, 2H, J = 7.7 Hz); 7.67 (t, 1H, J = 7.3 Hz); 7.76 (d, 1 H, J = 8.4 Hz); 7.8 (d, 1 H J = 3.67 Hz); 7.96 (d, 2H, J = 7.8 Hz).

### Stufe 2: 1-Benzolsulfonyl-4-brommethyl-1H-indol

Eine Lösung von 1-Benzolsulfonyl-4-methyl-1*H*-indol (3.90 g, 14.4 mmol) in Tetrachlorkohlenstoff (120 mL) wurde unter Rückfluss mit Azoisobutyronitril (50 mg) und *N-*Bromsuccinimid (3.10 g, 17.3 mmol) versetzt und weitere 4 h unter Rückfluss erhitzt. Der ausgefallene Niederschlag wurde abfiltriert, das Filtrat i. Vak. eingeengt und der Rückstand (5.8 g) mittels Flashchromatographie mit Ethylacetat / Cyclohexan (1:9) gereinigt.

### Ausbeute: 4.20 g (83 %), weißer Feststoff

¹H-NMR (DMSO-d₆): 4.95 (s, 2H); 7.05 (dd, 1H, J =0.8, 3.7 Hz); 7.30-7.35 (m, 2H); 7.55-7.66 (m, 3H); 7.89-8.04 (m, 4H).

### Stufe 3: 1-Benzolsulfonyl-4-(1,4-dioxaspiro[4.5]dec-8-ylmethoxyrnethyl)-1H-indol

Ein Gemisch von 1-Benzolsulfonyl-4-brommethyl-1*H*-indol (4.00 g, 11.4 mmol), (1,4-Dioxaspiro[4.5]dec-8-yl)methanol (2.90 g, 17.1 mmol), Ethyldiisopropylamin (1.47 g, 1.9 mL, 11.4 mmol) und Kupfer(II)-acetylacetonat (149 mg, 0.57 mmol) in *p*-Xylol (70 mL) wurde in einem Teflondruckgefäß 24 h bei 140 °C gerührt. Das Lösungsmittel wurde anschließend i. Vak. entfernt. Der Rückstand wurde in Dichlormethan (50 mL) aufgenommen und die Lösung mit 0.05 *N* Salzsäure und Wasser (je 50 mL) gewaschen. Die organische Phase wurde mit Natriumsulfat getrocknet und eingeengt. Das Rohprodukt (5.8 g) wurde mittels Flashchromatographie (400 g, 21 × 7.0 cm) mit Ethylacetat / Cyclohexan (1:4) gereinigt.

### Ausbeute: 912mg (18 %), weißer wachsartiger Feststoff

¹H-NMR (DMSO-d₆): 1.10-1.20 (m, 2H); 1.35-1.45 (m, 2H); 1.57-1.67 (m, 5H); 3.26 (d, 2H, J = 6.3 Hz); 3.82 (s, 4H); 4.64 (s, 2H); 6.89 (dd, 1 H, J = 0.8, 3.7 Hz); 7.19-7.22 (m, 1H); 7.29-7.34 (m, 1 H); 7.56-7.61 (m, 2H); 7.66-7.71 (m, 1 H); 7.82 (d, 1H, J = 3.7 Hz); 7.88 (d, 1 H, J = 8.3 Hz); 7.95-7.99 (m, 2H).

### Stufe 4: 4-[1-Benzolsulfonyl-1H-indol-4-ylmethoxymethyl)cyclohexanon

Eine Lösung von 1-Benzolsulfonyl-4-(1,4-dioxaspiro[4.5]dec-8-ylmethoxymethyl)-1*H-*indol (920 mg, 2.1 mmol) in Tetrahydrofuran (25 mL) wurde mit 2 *N* Salzsäure (5 mL) versetzt und 5 h bei 70 °C gerührt. Das Reaktionsgemisch wurde anschließend mit Natriumhydrogencarbonat-Lösung auf pH 8 eingestellt. Die Phasen wurde getrennt und die wässrige Phase mit Dichlormethan (2 × 20 mL) extrahiert. Die vereinigten organische Phasen wurden mit Natriumsulfat getrocknet und i. Vak. eingeengt.

### Ausbeute: 776 mg (92 %), gelbes Öl

¹H-NMR (DMSO-d₆): 1.23-1.40 (m, 2H); 1.87-2.08 (m, 3H); 2.09-2.20 (m, 2H); 2.34 (dt, 2H, J = 5.9, 13.7 Hz); 3.35 (d, 2H, J = 6.4 Hz); 4.68 (s, 2H); 6.91 (dd, 1H, J = 0.8. 3.72 Hz); 7.20-7.25 (m, 1H); 7.29-7.36 (m, 1 H); 7.54-7.72 (m, 3H); 7.84 (d, 1 H,J = 3.72 Hz); 7.89 (d, 1H, J = 8.2 Hz,); 7.95-8.01 (m, 2H).

### Stufe 5: 4-[1-Benzolsulfonyl-1H-indol-4-ylmethoxymethyl)-1-dimethylaminocyclohexancarbonitril

Zu einer auf 0 °C gekühlten 40 % wässrigen Dimethylaminlösung (1.1 mL, 8.7 mmol) und Methanol (0.3 mL) wurden eine Lösung von 4 *N* Salzsäure (0.87 mL, 3.48 mmol), anschließend Kaliumcyanid (227 mg, 3.48 mmol) und dann eine Lösung von 4-[1-Benzolsulfonyl-1*H*-indol-4-ylmethoxymethyl)cyclohexanon (578 mg, 1.45 mmol) in Methanol / Tetrahydrofuran (2 mL / 1 mL) gegeben. Dieses Gemisch wurde über Nacht bei Raumtemperatur gerührt. Nach Zugabe von Wasser (3 mL) wurde mit Diethylether (2 × 10 mL) extrahiert. Die vereinigten organischen Phasen wurden mit Natriumsulfat getrocknet und i. Vak. eingeengt.

### Ausbeute: 630 mg (96 %), gelbes dickflüssiges Öl

¹H-NMR (DMSO-d₆): 1.01-1.37(m, 4H); 1.39-1.83 (m, 3H); 1.97-2.11 und 2.11-2.21 (2 m, 2H); 2.18 und 2.23 (2 s, 6H); 3.25 (d, 0.33H, J = 6.5 Hz); 3.28 (d, 1.67H, J = 6.3 Hz); 4.64 und 4.66 (2 s, 2H); 6.88-6.92 (m, 1 H); 7.18-7.24 (m, 1H); 7.28 -7.35 (m, 1 H); 7.55-7.63 (m, 2H); 7.65-7.72 (m, 1H); 7.83 (d, 1H, J = 3.7 Hz); 7.88 (d, 1H, J = 8.25 Hz); 7.95-8.01 (m, 2H).

Das Diastereoisomerenverhältnis beträgt ca. 1:5.

### Stufe 6: N,N-Dimethyl-1-phenyl-4-(((1-(phenylsulfonyl)-1H-indol-4-yl)methoxy)methyl)-cyclohexanamin (Eines von zwei möglichen Diastereomeren)

Zu einer eisgekühlten 2 *M* Lösung von Phenylmagnesiumchlorid in Tetrahydrofuran (2.8 mL, 5.58 mmol) wurde unter Argon eine Lösung von 4-(1-Benzolsulfonyl-1*H*-indol-4-ylmethoxymethyl)-1-dimethylaminocyclohexancarbonitril (630 mg, 1.4 mmol) in wasserfreiem Tetrahydrofuran (4 mL) getropft und anschließend über Nacht bei Raumtemperatur gerührt. Danach wurde das Reaktionsgemisch mit 20 % Ammoniumchloridlösung (5 mL) versetzt. Die Phasen wurden getrennt und die wässrige mit Diethylether (2 × 10 mL) extrahiert. Die vereinigten organischen Phasen wurden mit Natriumchloridlösung (20 mL) gewaschen, mit Natriumsulfat getrocknet und i. Vak. eingeengt. Das Rohprodukt (554 mg) wurde mittels Flashchromatographie (30 g, 18 × 2.5 cm) zuerst mit Ethylacetat und dann nochmals (20 g, 22 × 2.0 cm) mit Ethylacetat / Cyclohexan (2:1) gereinigt.

### Ausbeute: 296 mg (42 %), farbloses Öl

¹H-NMR (DMSO-d₆): 1.26-1.46 (m, 4H); 1.45-1.56 (m, 2H); 1.54-1.68 (m, 1H); 1.89 (s, 6H); 2.51-2.58 (m, 2H); 3.30-3.32 (m, 2H); 4.66 (s, 2H); 6.93 (dd, 1H, J = 0.8, 3.7 Hz); 7.20-7.25 (m, 2H); 7.27-7.36 (m, 5H); 7.54-7.61 (m, 2H); 7.65-7.70 (m, 1 H); 7.84 (d, 1 H, J = 3.7 Hz); 7.89 (d, 1H, J = 8.3 Hz); 7.96-8.00 (m, 2H).

¹³C-NMR (DMSO-d₆): 24.4 (2C); 32.0 (2C); 36.9; 37.4 (2C); 58.5; 70.1; 75.3; 108.0; 112.3; 122.4; 124.5; 126.1; 126.5 (2C); 126.6 (2C); 126.7; 127.2 (2C); 129.1; 129.8 (2C); 131.7; 134.1; 134.5; 137.0; 139.4.

Es handelt sich um ein einheitliches Diastereoisomer.

### Beispiel 71: (nicht erfindungsgemäß)

### 4-(((1H-Indol-4-yl)methoxy)methyl)-N,N-dimethyl-1-phenylcyclohexanamin (Eines von zwei möglichen Diastereomeren)

Eine Lösung von N,N-Dimethyl-1-phenyl-4-(((1-(phenylsulfonyl)-1 H-indol-4-yl)methoxy)methyl)cyclohexanamin (230 mg, 0.46 mmol) in Methanol (20 mL) wurde mit 2 *N* Natronlauge (2 mL) versetzt und 4.5 h unter Rückfluss gerührt. Das Reaktionsgemisch wurde anschließend mit 2*N* Salzsäure auf pH -5-6 eingestellt und Methanol i. Vak. abdestilliert. Die wässrige Lösung wurde mit Natriumhydrogencarbonat auf pH ∼8-9 eingestellt und mit Dichlormethan (2 × 15 mL) extrahiert. Die vereinigten organischen Phasen wurden mit Natriumsulfat getrocknet und i. Vak. eingeengt. Der Rückstand (159 mg) wurde mittels Flashchromatographie (10 g, 16 × 1.5 cm) mit Dichlormethan / Methanol (9:1) gereinigt.

### Ausbeute: 87 mg (52 %), farbloses Öl

¹H-NMR (CDCl₃): 1.48-1.84 (m, 7H); 2.05 (s, 6H); 2.46-2.56 (m, 2H); 3.48 (d, 2H, J = 6.8 Hz); 4.85 (s, 2H); 6.68-6.71 (m, 1H); 7.10-7.14 (m, 1H); 7.15-7.18 (m, 1 H); 7.19-7.22 (m, 1H); 7.30-7.39 (m, 6H); 8.34 (br s, 1H).

¹C-NMR (CDCl₃)- 24.7; 32.0 (sehr br); 37.0 (sehr br); 37.8; 59.7 (sehr br); 71.9; 75.3 (sehr br); 101.2; 110.6; 119.1; 121.7; 124.0; 126.4; 126.7; 127.0; 127.5; 128.4; 130.5; 135.9. LC-MS (Methode 7): m/z: [M+H]⁺ = 363.3, Rₜ 2.9 min.

### Beispiel 72: (nicht erfindungsgemäß)

### N,N-Dimethyl-1-phenyt-4-(((2-(triethylsilyl)-5-(trifluoromethoxy)-1H-indol-3-yl)-methoxy)methyl)cyclohexanamin (Eines von zwei möglichen Diastereomeren)

Eine Mischung von **Ain-1** (1.10 g, 2.8 mmol), 4-Trifluormethoxy-2-iodanilin **(Ian-12)** (1.13 g, 3.7 mmol), [1,3-Bis-(2,6-diisopropylphenyl)imidazol-2-yliden]-(3-chlorpyridyl)palladium(II)-chlorid (PEPPSI, 380 mg, 0.56 mmol) und Natriumcarbonat (1.48 g, 14 mmol) in wasserfreiem *N,N*-Dimethylformamid (15 mL) wurde bei 100 °C 18 h gerührt. Das Lösungsmittel wurde i. Vak. entfernt und der Rückstand zwischen Wasser und Diethylether (je 60 mL) verteilt. Die wässrige Phase wurde mit Diethylether (3 × 20 mL) extrahiert. Die vereinigten organischen Phasen wurden mit Wasser und Natriumthiosulfatlösung (je 30 mL) gewaschen, mit Natriumsulfat getrocknet und i. Vak. eingeengt. Das Rohprodukt (2.70 g) wurde durch Flashchromatographie (100 g, 20 × 4.0 cm) mit Ethylacetat / Cyclohexan (1:6) gereinigt.

### Ausbeute: 1.33 g (85 %), bräunliches Öl

¹H-NMR (DMSO-d₆): 0.86-0.99 (m, 15H); 1.28-1.65 (m, 9H); 1.87 (s, 6H); 2.51-2.58 (m, 2H); 4.63 (s, 2H); 7.06 (d, 1H, J = 8.3 Hz); 7.18-7.24 (m, 1 H); 7.27-7.37 (m, 4H), 7.47 (d, 1H, J = 8.7 Hz); 7.52 (s, 1H); 10.92 (s, 1 H).

¹³C-NMR (DMSO-d₆): 3.1; 7.2; 24.5; 32.0; 37.0; 37.4, 58.5; 64.4; 74.7; 110.8; 11.2.3; 115.1; 120.4 (q, J = 254 Hz); 121.9; 126.1; 126.5; 127.2; 128.4; 136.2; 137.1; 139.4; 141.5. m/z: [M+H]⁺ = 561.4

### Beispiel 73: (nicht erfindungsgemäß)

### N,N-Dimethyl-1-phenyl-4-(((5-(trifluoromethoxy)-1H-indol-3-yl)methoxy)methyl)-cyclohexanamin (Eines von zwei möglichen Diastereomeren)

Eine Suspension von Benzyltrimethylammoniumfluorid-Monohydrat (966 mg, 5.7 mmol) in wasserfreiem Tetrahydrofuran (60 mL) wurde mit aktiviertem Molsieb 4 A (4.00 g) versetzt und 1 h bei Raumtemperatur gerührt. Anschließend wurde zu dieser Suspension eine Lösung von Beispiel 72 (1.08 g, 1.9 mmol) in wasserfreiem Tetrahydrofuran (30 mL) gegeben und 1 h unter Rückfluss gerührt. Das Reaktionsgemisch wurde fltriert, das Filtrat i. Vak. eingeengt und der Rückstand (1.13 g) durch Flashchromatographie (100 g, 20 × 4.0 cm) mit Ethylacetat / Methanol (95:5) gereinigt.

### Ausbeute: 546 mg (64 %), gelbliches Öl

¹H-NMR (DMSO-d₆): 1.28-1.64 (m, 7H); 1.89 (s, 6H); 2.54 (br d, 2H, J = 14.3 Hz); 3.29 (br d, 2H, J = 6.3 Hz); 4.62 (s, 2H); 7.04-7.09 (m, 1H); 7.19-7.25 (m, 1H); 7.27-7.36 (m, 4H); 7.44-7.53 (m, 3H), 11.25 (s, 1 H).

¹³C-NMR (DMSO-d₆): 24.5; 32.0; 37.0; 37.4; 58.5; 64.2; 74.3; 111.0; 112.5; 112.8; 114.7; 120.4 (q, J = 254 Hz); 126.1; 126.5; 126.9; 127.0; 127.2; 134.8; 139.4; 141.6 (br).

m/z: [M+H]⁺ = 447.3

### Untersuchungen zur Wirksamkeit der erfindungsgemäßen Verbindungen:

### Messung der ORL1-Bindung

Die Cyclohexan-Derivate der allgemeinen Formel I wurden in einem Rezeptorbindungsassay mit ³H-Nociceptin/Orphanin FQ mit Membranen von rekombinanten CHO-ORL1 Zellen untersucht. Diese Messungen wurde gemäß der von Ardati et al. (Mol. Pharmacol., 51, 1997, S. 816-824) vorgestellten Methode durchgeführt. Die Konzentration von ³H-Noci-ceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM M₉Cl₂ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei Raumtemperatur und anschliessende Messung im Szintillationscounter Trilux (Wallac, Finnland), bestimmt.

### Messung der µ-Bindung

Die Rezeptoraffinität zum humanen µ-Opiatrezeptor wurde in einem homogenen Ansatz in Mikrotiterplatten bestimmt. Hierzu wurden Verdünnungsreihen des jeweils zu prüfenden substituierten 4-Aminocyclohexan-Derivates mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 µmol/l Naloxon zugegeben. Nach Beendigung der neunzigminütigen Inkubationszeit wurden die Mikrotiterplatten für 20 Minuten bei 1000 g abzentrifugiert und die Radioaktivität in einem ß-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen µ-Opiatrezeptor bei einer Konzentration der Prüfsubstanzen von 1 µmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I IC₅₀ Hemmkonzentrationen berechnet, die eine 50-prozentige Verdrängung des radioaktiven Liganden bewirken. Durch Umrechnung mittels der Cheng-Prusoff-Beziehung werden Ki-Werte für die Prüfsubstanzen erhalten.

Die erfindungsgemäßen Verbindungen binden an den ORL1-Rezeptor und/oder den µ-Rezeptor.

### Chung-Modell: Mononeuropathieschmerz nach spinaler Nervenligatur

Tiere: Männliche Sprague Dawley Ratten (140-160g), von einem kommerziellen Züchter (Janvier, Genest St. Isle, Frankreich), wurden unter einem 12:12h Licht-Dunkel Rhythmus gehalten. Die Tiere wurden mit Futter und Leitungswasser ad libitum gehalten. Zwischen der Lieferung der Tiere und der Operation wurde eine Pause von einer Woche eingehalten. Die Tiere wurden nach Operation über einen Zeitraum von 4-5 Wochen mehrfach getestet, wobei eine Auswaschzeit von mindestens einer Woche eingehalten wurde.

Modellbeschreibung: Unter Pentobarbital Narkose (Narcoren ^{®}, 60 mg/kg i.p., Merial GmbH, Hallbergmoos, Deutschland), wurden die linken L5, L6 Spinalnerven exponiert, indem ein Stück des paravertebralen Muskels und ein Teil des linken spinalen Prozesses des L5 lumbalen Wirbelkörpers entfernt wurden. Die Spinalnerven L5 und L6 wurden vorsichtig isoliert und mit einer festen Ligatur abgebunden (NC-silk black,USP 5/0, metric 1, Braun Melsungen AG, Melsungen, Deutschland) (Kim and Chung 1992). Nach Ligatur wurden Muskel und benachbarte Gewebe zugenäht und die Wunde mittels Metallklammern geschlossen.

Nach einer Woche Erholungszeit wurden die Tiere zur Messung der mechanischen Allodynie in Käfige mit einem Drahtboden gesetzt. An ipsi- und kontralateraler Hinterpfote wurde jeweils die Wegziehschwelle mittels eines elektronischen von Frey Filamentes (Somedic AB, Malmö, Schweden) bestimmt. Zu jedem Messzeitpunkt wurde der Median von fünf konsekutiven Stimulierungen ermittelt. Die Tiere wurden 30 min vor und zu verschiedenen Zeiten nach Applikation von Testsubstanz- oder Vehikellösung getestet. Die Daten wurden als % maximal mögliche Wirkung (%MPE) aus den Vortesten der Einzeltiere (=0%MPE) und den Testwerten einer unabhängigen Sham-Kontrollgruppe (=100%MPE) bestimmt. Alternativ wurden die Wegziehschwellen in Gramm dargestellt.

Statistische Auswertung: ED₅₀ Werte und 95% Vertrauensbereiche wurden über semilogarithmische Regressionsanalyse zum Zeitpunkt der maximalen Wirkung bestimmt. Die Daten wurden über eine Varianzanalyse mit wiederholten Messungen sowie einer post hoc Analyse nach Bonferroni analysiert. Die Gruppengröße betrug üblicherweise n=10.

Referenzen: Kim,S.H. and Chung,J.M., An experimental model forperipheral neuropathy produced by segmental spinal nerve ligation in the rat, Pain, 50 (1992) 355-363.

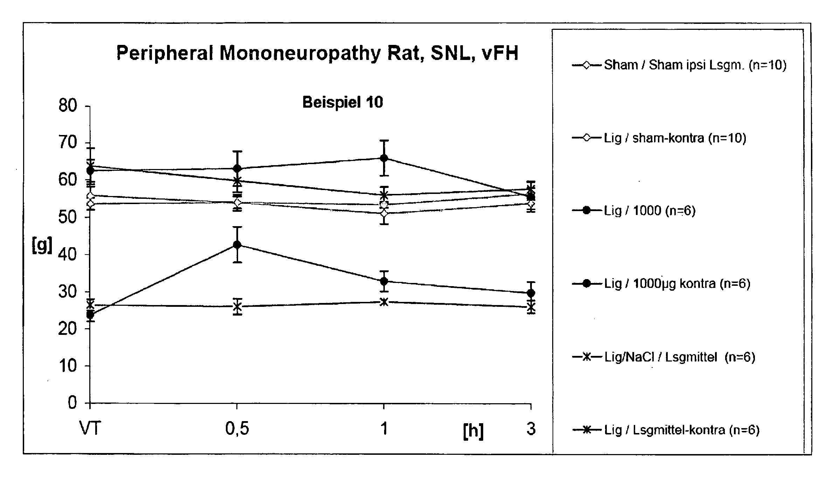

Die Untersuchung von Beispiel 10 im Chung-Modell zeigt, dass die Verbindung primär auf die ipsilaterale Seite (d. h. im Neuropathischmerz) wirkt (vgl. auch Figur 1). Dagegen wurde auf der nicht-geschädigten, kontralateralen Seite nur ein sehr geringer und statistisch nicht signifikanter Effekt beobachtet. Der Test zeigt, dass innerhalb eines Tieres die Verbindung eine sehr gute Wirksamkeit im Neuropathieschmerz hat, da der Zustand der Allodynie (sichtbar in der Absenkung der Wegziehschwelle von ca. 55 Gramm auf 25 Gramm) nahezu vollständig aufgehoben wird (sichtbar an der Anhebung der Wegzeischwelle auf über 40 Gramm). Das Fehlen eines Effektes auf der kontralateralen Seite zeigt an, dass die Verbindung in der getesteten Dosierung per-se keine anti-nozizeptive - also generell schmerzunterdrückende Wirkung hat. daraus lässt sich folgern, dass die Verbindung pathologischen Zustand der Allodynie in der neuropathischen Situation aufhebt ohne direkt analgetisch zu wirken. Dies bedeutet, dass zur Therapie von neuropathischem Schmerz niedriger dosiert werden kann als dies zur Behandlung von Akutschmerz notwendig wäre.

### Parenterale Lösung eines erfindungsgemäßen substituierten 4-Aminocyclohexan-Derivats

38 g eines der erfindungsgemäßen substituierten 4-Aminocyclohexan-Derivate, hier Beispiel 3, wird in 1 I Wasser für Injektionszwecke bei Raumtemperatur gelöst und anschließend durch Zugabe von wasserfreier Glukose für Injektionszwecke auf isotone Bedingungen eingestellt.

## Patentansprüche

1. Substituierte 4-Aminocyclohexan-Derivate der allgemeinen formel I, 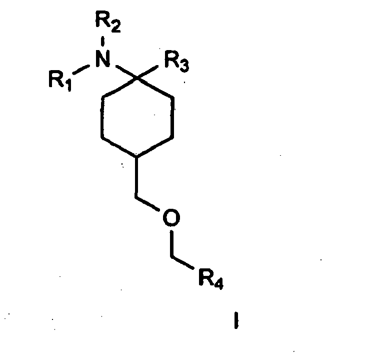 worin
R¹ und R² unabhängig voneinander methyl oder H bedeuten oder die Reste R¹ und R² unter Einschluss des N-Atoms einen Ring bilden und gemeinsam (CH₂)₃
bedeuten;
R³ für Phenyl, unsubstituiert oder einfach substituiert mit F, Cl, CN, CH₃; Thienyl; oder n-Butyl, unsubstituiert oder einfach oder mehrfach substituiert mit OCH₃, OH oder OC₂H₅, insbesondere mit OCH₃; steht.
R⁴ für 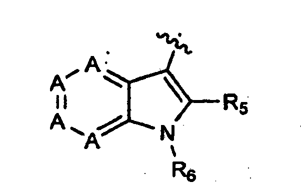 steht,
worin
A für N oder CR⁷⁻¹⁰ steht, wobei A höchstens einmal N bedeutet;
R⁵, R⁷, R⁸, R⁹ und R¹⁰ unabhängig voneinander für H, F, Cl. Br, CN, CH₃, C₂H₅·NH₂, *tert*.-Butyl, Si(Ethyl)₃, Si(Methyl)₂(*tert*.-Butyl), SO₂CH₃, C(O)CH₃, NO₂, SH, CF₃, OH, OCH₃, OC₂HS oder N(CH₃)₂, insbesondere Si(Ethyl)₃, Si(Methyl)₂(*tert*.-Butyl), CN, CF₃, F, C(O)CH₃, SO₂CH₃ oder CH₃, und
R⁶ für H, CH₃ oder C(O)CH₃ steht.
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

2. Substituierte 4-Aminocyclohexan-Derivate gemäß Anspruch 1, worin R⁴ eine der Strukturen L, M oder N bedeutet 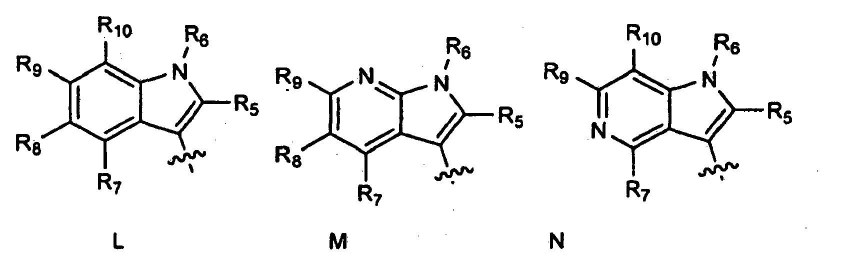 wobei die Reste
R⁵, R⁷, R⁸, R⁹ und R¹⁰ unabhängig voneinander für H, F, Cl, Br, CN, CH₃, C₂H₅' NH₂, tert.-Butyl, Si(Ethyl)₃, Si(Methyl)₂(*tert*.-Butyl), SO₂CH₃, C(O)CH₃, NO₂, SH, CF₃, OH,
OCH₃. OC₂H₅ oder N(CH₃)₂, insbesondere Si(Ethyl)₃, Si(Methyl)₂(*tert*.-Butyl), CN, CF₃, F, C(O)CH₃, SO₂CH₃ oder CH₃, und
R⁶ für H, CH₃ oder C(O)CH₃ steht.

3. Substituierte 4-Aminocyclohexan-Derivate gemäß einem der vorstehenden Ansprüche aus der Gruppe:
1 [1-Phenyl-4-(2-triethylsilanyl-1H-indol-3-ylmethoxymethyl)cyclohexyl]dimethyl-amin
2 [1-Phenyl-4-(1H-indol-3-ylmethoxymethyl)cyclohexyl]dimethylamin
3 1-Phenyl-4-(((2-(tert-butyldimethylsilyl)-5-cyano-1H-indol-3-yl)methoxy)methyl)-N,N-dimethylcyclohexanamin
4 1-Phenyl-4-(((5-cyano-2-(triethylsilyl)-1H-indol-3-yl)methoxy)methyl)-N,N-dimethylcyclohexanamin
5 1-Phenyl-4-(((5-cyano-1H-indol-3-yl)methoxy)methyl)-N,N-dimethyl-cyclohexanamin
6 1-Phenyl-4-(((2-(tert-butyldimethylsilyl)-5-trifluormethyl-1H-indol-3-yl)methoxy)methyl)-N,N-dimethylcyclohexanamin
7 1-Phenyl-4-(((5-trifluormethyl-2-(triethylsilyl)-1H-indol-3-yl)methoxy)methyl)-N,N-dimethylcyclohexanamin
8 1-Phenyl-4-(((5-trifluormethyl-1H-indol-3-yl)methoxy)methyl)-N,N-dimethyl-cyclohexanamin
9 1-Phenyl-4-(((5-fluor-2-(triethylsilyl)-1H-indol-3-yl)methoxy)methyl)-N,N-dimethylcyclohexanamin
10 1-Phenyl-4-(((5-fluor-1H-indol-3-yl)methoxy)methyl)-N,N-dimethyl-cyclohexanamin
11 1-Phenyl-N,N-dimethyl-4-(((2-(triethylsilyl)-1H-pyrrolo[2,3-b]pyridin-3-yl)methoxy)methyl)cyclohexanamin
12 4-(((1H-Pyrrolo[2,3-b]pyridin-3-yl)methoxy)methy)-1-phenyl-N,N-dimethylcyclohexanamin
13 1-Phenyl-4-(((2-(tert-butyldimethylsilyil)-1H-pyrrolo[3,2-c]pyridin-3-yl)methoxy)methyl)-N,N-dimethylcyclohexanamin
14 1-Phenyl-N,N-dimethyl-4-(((2-(triethylsily)-1H-pyrrolo[3,2-c]pyridin-3-yl)methoxy)methyl)cyclohexanamin
15 4-(((1H-Pyrrolo[3,2-c]pyridin-3-yl)methoxy)methyl)-1-phenyl-N,N-dimethylcyclohexanamin
16 1-(3-(((4-(Dimethylamino)-4-phenylcyclohexyl)methoxy)methyl-5-fluor-2-(triethylsilyl)-1H-indol-yl)ethanon
17 4-(((5-Fluor-1-(methylsulfonyl)-2-(triethylsilyl)-1H-indol-3-yl)methoxy)methyl)-N,N-dimethyl-1-phenylcyclohexanamin
18 1-Phenyll-4-[2-(*tert*-butyldimethylsilanyl)1*H*-indol-3-ylmethoxy-methyl]cyclohexyldimethylamin
19 1-Phenyl-4-(((2-(tert-butyldimethylsilyl)-5-fluor-1H-indol-3-yl)methoxy)methyl)-N,N-dimethylcyclohexanamin
20 1-Phenyl-4-(((2-(tert-butyldimethylsilyl)-1H-pyrrolo[2,3-b]pyridin-3-yl)methoxy)methyl)-N,N-dimethylcyclohexanamin
22 {1-Benzyl-4-[2-(*tert*-butyldimethylsilanyl)-1*H*-indol-3-ylmethoxymethyl]cyclohexyl}-dimethylamin
23 [1-Benzyl-4-(2-triethylsllanyl-1*H*-indol-3-ylmethoxymethyl)cyclohexyl] dimethylamin
24 [1-Benzyl-4-(1*H*-indol-3-ylmethoxymethyl)cyclohexyl]dilmethylamin
25 1-Benzyl-4-(((5-fluor-2-(triethylsilyl)-1H-indol-3-yl)methoxy)methyl)-N,N-dimethylcyclohexanamin
26 1-Benzyl-4-(((2-(tert-butyldimethylsilyl)-5-fluor-1H-indol-3-yl)methoxy)methyl)-N,N-dimethylcyclohexanamin
27 1-Benzyl-4-(((5-fluor-1H-indol-3-yl)methoxy)methyl)-N,N-dimethylcyclohexanamin
28 1-Benzyl-N,N-dimethyl-4-(((2-(triethylsilyl)-1H-pyrrolo[2,3-b]pyridin-3-yl)methoxy)methyl)cyclohexanamin
29 1-Benzyl-4-(((2-(tert-butyldimethylsilyl)-1H-pyrrolo[2,3-b]pyridin-3-yl)methoxy)methyl)-N,N-dimethylcyclohexanamin
30 4-(((1H-Pyrrolo[2,3-b]pyridin-3-yl)methoxy)methyl)-1-benzyl-N,N-dimethylcyclohexanamin
31 1-Benzyl-N,N-dimethyl-4-(((2-(triethylsilyl)-1H-pyrrolo[3,2-c]pyridin-3-yl)methoxy)methyl)cyclohexanamin
32 1-Benzyl-4-(((2-(tert-butyldimethylsilyl)-1H-pyrrolo[3,2-c]pyridin-3-yl)methoxy)methyl)-N,N-dimethylcyclohexanamin
33 4-(((1H-Pyrrolo[3,2-c]pyridin-3-yl)methoxy)methyl)-1-benzyl-N,N-dimethylcyclohexanamin
34 1-Benzyl-N,N-dimethyl-4-(((2-(triethylsilyl)-1H-indol-3-yl)methoxy)methyl)cyclohexanamin
35 1-Benzyl-4-(((2-(tert-butyldimethylsilyl)-1H-indol-3-yl)methoxy)methyl)-N,N-dimethylcyclohexanamin
36 4-(((1H-indol-3-yl)methoxy)methyl)-1-benzyl-N,N-dimethylcyclohexanamin
37 1-Benzyl-4-(((5-fluor-2-(triethylsilyl)-1H-indol-3-yl)methoxy)methyl)-N,N-dimethylcyclohexanamin
38 1-Benzyl-4-(((2-(tert-butyldimethylsilyl)-5-fluor-1H-indol-3-yl)methoxy)methyl)-N,N-dimethylcyclohexanamin
39 1-Benzyl-4-(((5-fluor-1H-indol-3-yl)methoxy)methyl)-N,N-dimethylcyclohexanamin
40 1-Benzyl-N,N-dimethyl-4-(((2-(triethylsilyl)-1H-pyrrolo[2,3-b]pyridin-3-yl)methoxy)methyl)cyclohexanamin hydrochlorid
41 4-(((1H-Pyrrolo[2,3-b]pyridin-3-yl)methoxy)methyl)-1-benzyl-N,N-dimethylcyclohexanamin
42 1-Benzyl-4-(((2-(tert-butyldimethylsilyl)-1H-pyrrolo[3,2-c]pyridin-3-yl)methoxy)methyl)-N,N-dimethylcyclohexanamin
43 1-Butyl-N,N-dimethyl-4-(((2-(triethylsilyl)-1H-indol-3-yl)methoxy)methyl)cyclohexanamin
44 4-(((1H-Indol-3-yl)methoxy)methyl)-1-butyl-N,N-dimethylcyclohexanamin
45 1-Butyl-4-(((5-fluor-2-(triethylsilyl)-1H-indol-3-yl)methoxy)methyl)-N,N-dimethylcyclohexanamin
46 1-Butyl-4-(((5-fluor-1H-indol-3-yl)methoxy)methyl)-N,N-dimethylcyclohexanamin
47 1-Butyl-N,N-dimethyl-4-(((2-(triethylsilyl)-1H-pyrrolo[2,3-b]pyridin-3-yl)methoxy)methyl)cyclohexanamin
48 4-(((1H-Pyrrolo[2,3-b]pyridin-3-yl)methoxy)methyl)-1-butyl-N,N-dimethylcyclohexanamin ;
49 1-Butyl-N,N-dimethyl-4-(((2-(triethylsilyl)-1H-pyrrolo[3,2-c]pyridin-3-yl)methoxy)methyl)cyclohexanamin
50 4-(((1H-Pyrrolo[3,2-c]pyridin-3-yl)methoxy)methyl)-1-butyl-N,N-dimethylcyclohexanamin
51 1-Butyl-4-(((5-fluor-2-(triethylsilyl)-1H-indol-3-yl)methoxy)methyl)-N-methylcyclohexanamin
52 1-Butyl-N-methyl-4-(((2-(triethylsilyl)-1H-indol-3-yl)methoxy)methyl)cyclohexanamin
53 1-Butyl-N-methyl-4-(((2-(triethylsilyl)-5-(trifluormethyl)-1H-indol-3-yl)methoxy)methyl)cyclohexanamin
54 1-Butyl-N-methyl-4-(((5-(trifluormethyl)-1H-indol-3-yl)methoxy)methyl)cyclohexanamin
55 4-(((5-Fluor-2-(triethylsilyl)-1H-indol-3-yl)methoxy)methyl)-N,N-dimethyl-1-(thiophen-2-yl)cyclohexanamin
56 4-(((5-Fluor-1H-indol-3-yl)methoxy)methyl)-N,N-dimethyl-1-(thiophen-2-yl)cyclohexanamin
57 3-(((4-(Azetidin-1-yl)-4-phenylcyclohexyl)methoxy)methyl)-5-fluor-2-(triethylsilyl)-1H-indol
58 3-(((4-(Azetidin-1-yl)-4-phenylcyclohexyl)methoxy)methyl)-5-fluor-1H-indol 60 4-(((5-Fluor-1-methyl-2-(triethylsilyl)-1H-indol-3-yl)methoxy)methyl)-N,N-dimethyl-1-phenylcyclohexanamin
61 4-(((5-Fluor-1-methyl-1H-indol-3-yl)methoxy)methyl)-N,N-dimethyl-1-phenylcyclohexanamin (GRTE9052; WW447)
62 4-(((5-Fluor-1-(methylsulfonyl)-1H-indol-3-yl)methoxy)methyl)-N,N-dimethyl-1-phenylcyclohexanamin
63 1-(3-(((4-(Dimethylamino)-4-phenylcyclohexy)methoxy)methyl)-5-fluor-1H-indol-1-yl)ethanon
64 4-(((2-*tert*-Butyl-5-fluor-1H-indol-3-yl)methoxy)methyl)-N,N-dimethyl-1-phenylcyclohexanamin
65 3-(((4-(Azetidin-1-yl)-4-phenylcyclohexyl)methoxy)methyl)-2-(triethylsilyl)-1H-pyrrolo[2,3-b]pyridin
66 3-(((4-(Azetidin-1-yl)-4-phenylcyclohexyl)methoxy)methyl)-1H-pyrrolo[2,3-b]pyridin
67 4-(((2-*tert*-Butyl-1H-pyrrolo[2,3-b]pyridin-3-yl)methoxy)methyl)-N,N-dimethyl-1-phenylcyclohexanamin in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

4. Verfahren zur Herstellung von substituierten 4-Aminocyclohexan-Derivaten gemäß einem der vorstehenden Ansprüche, 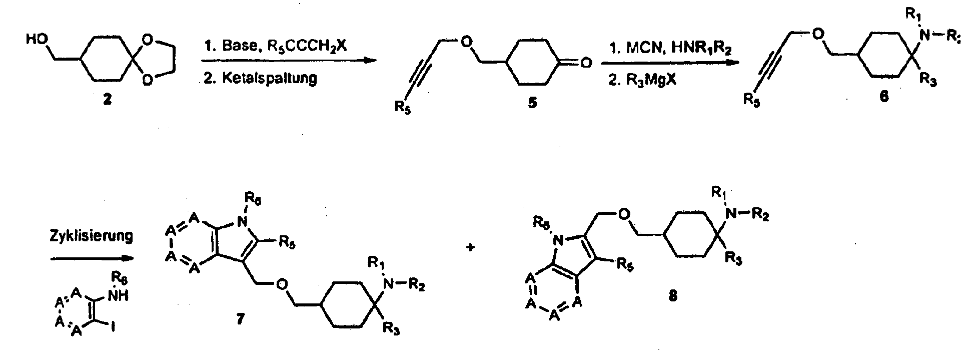 worin
a) das ketalgeschützten 4-Hydroxymethyl-cyclohexanon **2** in organischen Lösungsmitteln, beispielsweise Ether, Tetrahydrofuran, Dimethylformamid, Methanol, Ethanol oder Dichlormethan in Gegenwart von einer anorganischen oder organischen Base, beispielsweise Kalium-tert-butanolat, Natriummethanolat oder -ethanolat, Lithium- oder Natriumhydrid, Kalium- oder Natriumhydroxid, Protonenschwamm, Butyllithium, oder anderen basischen Organometallverbindungen wie Grignardreagenzien, beispielsweise Ethylmagnesiumchlorid oder -bromid, gegebenenfalls in Anwesenheit von Phasentransferkatalysatoren, beispielsweise Tetra-*n*-butylammoniumchlorid, Tetra-*n-*butylammoniumhydroxid oder Tetra-*n*-butylammoniumiodid und mit einem Alkylierungsmittel
R⁵CCCH₂X mit X = Cl, Br
bei Temperaturen zwischen -10 und 120 °C und anschließender Ketalspaltung zu substituierten Cyclohexanonen der allgemeinen Formel 5 umgesetzt wird
b) die substituierten Cyclohexanone der allgemeinen Formel 5 in wässrigem Medium oder organischen Lösungsmitteln, beispielsweise Methanol, Ethanol, n-Propanol, Tetrahydrofuran, Acetonitril oder Dichlormethan, oder Mischungen dieser beiden Medien in Gegenwart einer anorganischen Säure, beispielsweise HCl, HBr, H₂SO₄, HClO₄ oder H₃PO₄, einer Cyanidquelle, beispielsweise Natrium- oder Kaliumcyanid, Zinkcyanid, Trimethylsilylcyanid, Acetoncyanhydrin, und einem Amin
HNR¹R²
oder dessen Hydrochlorid
HCl·HNR¹R²
bei Temperaturen zwischen 20 und 60 °C umgesetzt werden und die erhaltene Zwischenstufe in organischen Lösungsmitteln, beispielsweise Hexan, Pentan, Toluol, Ether, Düsopropylether, Tetrahydrofuran oder Methyl-*t*-Butylether, mit einem Grignardreagenz
MgXR³ mit X = Cl, Br, I
bei Temperaturen zwischen -10 °C und 40 °C zu 1-substituierten-4-Alkinyloxymethyl-Cyclohexylamin-Derivaten der allgemeinen Formel **6** umgesetzt werden;
c) 1-sübstituierte-4-Alkinyloxymethyl-Cyclohexylamin-Derivate der allgemeinen Formel **6** in organischen Lösungsmitteln, beispielsweise Tetrahydrofuran, Dimethylformamid, Benzol, Toluol, Xylolen, Dimethoxyethan oder Diethylenglykol-dimethylether, in Gegenwart einer anorganischen Base, beispielsweise Natrium-, Kalium- oder Cäsiumcarbonat oder Kaliumphosphat, in Anwesenheit von PdCl₂, Pd(OAC)₂, PdCl₂(MeCN)₂, PdCl₂(PPh₃)₂ oder [1,3- Bis-(2,6-diisopropylphenyl)imidazol-2-yliden]-(3-chlorpyridyl)palladium(II)-chlorid (PEPPSI^{®}), gegebenenfalls in Anwesenheit von zusätzlichen Liganden, beispielsweise Triphenyl-, Tri-*o-*tolyl-, Tricyclohexyl oder Tri-*t*-butylphosphin, gegebenenfalls in Anwesenheit von Phasentransferkatalysätoren, beispielsweise Tetra-*n*-butylammoniumchlorid, Tetra-n-butylammoniumhydroxid oder Tetra-*n*-butylammoniumiodid, bei Temperaturen zwischen 60 °C und 180 °C, auch mikrowellenunterstützt, zu den erfindungsgemäßen 4-Aminocyclohexan-Derivaten der allgemeinen Formeln **7** und **8** umgesetzt werden.

5. Arzneimittel enthaltend wenigstens ein substituiertes 4-Aminocyclohexan-Derivat gemäß einem der Ansprüche 1 bis 3 sowie gegebenenfalls enthaltend geeignete Zusatz- und/oder Hilfsstoffe und/oder gegebenenfalls weiterer Wirkstoffe.

6. Verwendung eines substituierten 4-Aminocyclohexan-Derivates gemäß einem der Ansprüche 1 bis 3 zur Herstellung eines Arzneimittels zur Behandlung von Schmerz, insbesondere neuropathischem Schmerz, diabetischem Polyneuropathieschmerz oder Schmerzen bei postzosterischer Neuralgie.

## Claims

1. Substituted 4-aminocyclohexane derivatives having the general formula I, 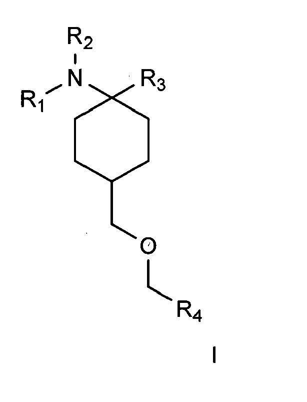 wherein
R¹ and R² mutually independently denote methyl or H or the radicals R¹ and R² form a ring with inclusion of the N atom and together denote (CH₂)₃;
R³ stands for phenyl, unsubstituted or monosubstituted with F, Cl, CN, CH₃; thienyl; or n-butyl, unsubstituted or mono- or polysubstituted with OCH₃, OH or OC₂H₅, in particular with OCH₃;
R⁴ stands for 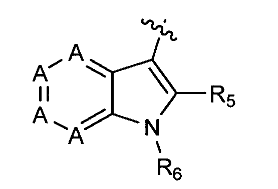 wherein
A stands for N or CR⁷⁻¹⁰, wherein A denotes at most one N;
R⁵, R⁷, R⁸, R⁹ and R¹⁰ mutually independently stand for H, F, Cl, Br, CN, CH₃, C₂H₅, NH₂, *tert*-butyl, Si(ethyl)₃, Si(methyl)₂(*tert*-butyl), SO₂CH₃, C(O)CH₃, NO₂, SH, CF₃, OH, OCH₃, OC₂H₅ or N(CH₃)₂, in particular Si(ethyl)₃, Si(methyl)₂(*tert*-butyl), CN, CF₃, F, C(O)CH₃, SO₂CH₃ or CH₃, and
R⁶ stands for H, CH₃ or C(O)CH₃;
in the form of the racemate; the enantiomers, diastereomers, mixtures of enantiomers or diastereomers or a single enantiomer or diastereomer; the bases and/or salts of physiologically acceptable acids or cations.

2. Substituted 4-aminocyclohexane derivatives according to claim 1, wherein R⁴ denotes one of the structures L, M or N 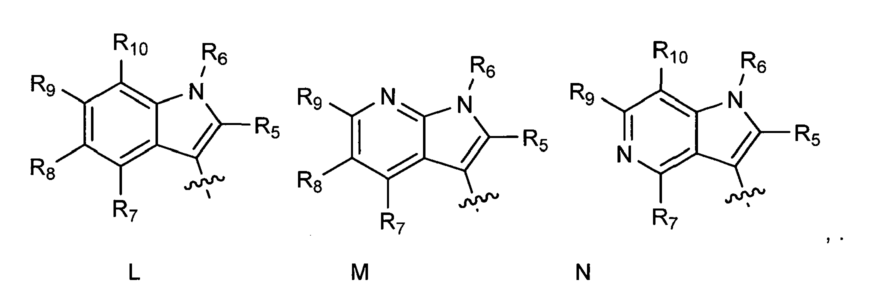 wherein the radicals
R⁵, R⁷, R⁸, R⁹ and R¹⁰ mutually independently stand for H, F, Cl, Br, CN, CH₃, C₂H₅, NH₂, *tert*-butyl, Si(ethyl)₃, Si(methyl)₂(*tert*-butyl), SO₂CH₃, C(O)CH₃, NO₂, SH, CF₃, OH, OCH₃, OC₂H₅ or N(CH₃)₂, in particular Si(ethyl)₃, Si(methyl)₂(*tert*-butyl), CN, CF₃, F, C(O)CH₃, SO₂CH₃ or CH₃, and
R⁶ stands for H, CH₃ or C(O)CH₃.

3. Substituted 4-aminocyclohexane derivatives according to one of the preceding claims, selected from the group comprising:
1 [1-Phenyl-4-(2-triethylsilanyl-1H-indol-3-ylmethoxymethyl)cyclohexyl]dimethylamine
2 [1-Phenyl-4-(1H-indol-3-ylmethoxymethyl)cyclohexyl]dimethylamine
3 1-Phenyl-4-(((2-(*tert*-butyldimethylsilyl)-5-cyano-1H-indol-3-yl)methoxy)methyl)-N,N-dimethylcyclohexanamine
4 1-Phenyl-4-(((5-cyano-2-(triethylsilyl)-1H-indol-3-yl)methoxy)methyl)-N, N-dimethylcyclohexanamine
5 1-Phenyl-4-(((5-cyano-1H-indol-3-yl)methoxy)methyl)-N,N-dimethylcyclohexanamine
6 1-Phenyl-4-(((2-(*tert*-butyldimethylsilyl)-5-trifluoromethyl-1H-indol-3-yl)methoxy)methyl)-N,N-dimethylcyclohexanamine
7 1-Phenyl-4-(((5-trifluoromethyl-2-(triethylsilyl)-1H-indol-3-yl)methoxy)metbyl)-N,N-dimethylcyclohexanamine
8 1-Phenyl-4-(((5-trifluoromethyl-1H-indol-3-yl)methoxy)methyl)-N,N-dimethylcyclohexanamine
9 1-Phenyl-4-(((5-fluoro-2-(triethylsilyl)-1H-indol-3-yl)methoxy)methyl)-N,N-dimethylcyclohexanamine
10 1-Phenyl-4-(((5-fluoro-1H-indol-3-yl)methoxy)methyl)-N,N-dimethylcyclohexanamine
11 1-Phenyl-N,N-dimethyl-4-(((2-(triethylsilyl)-1H-pyrrolo[2,3-b]pyridin-3-yl)methoxy)methyl)cyclohexanamine
12 4-(((1H-Pyrrolo[2,3-b]pyridin-3-yl)methoxy)methyl)-1-phenyl-N,N-dimethylcyclohexanamine
13 1-Phenyl-4-(((2-(*tert*-butyldimethylsilyl)-1H-pyrrolo[3,2-c]pyridin-3-yl)methoxy)methyl)-N,N-dimethylcyclohexanamine
14 1-Phenyl-N,N-dimethyl-4-(((2-(triethylsilyl)-1H-pyrrolo[3,2-c]pyridin-3-yl)methoxy)methyl)cyclohexanamine
15 4-(((1H-Pyrrolo[3,2-c]pyridin-3-yl)methoxy)methyl)-1-phenyl-N,N-dimethylcyclohexanamine
16 1-(3-(((4-(Dimethylamino)-4-phenylcyclohexyl)methoxy)methyl)-5-fluoro-2-(triethylsilyl)-1H-indol-1-yl)ethanone
17 4-(((5-Fluoro-1-(methylsulfonyl)-2-(triethylsilyl)-1H-indol-3-yl)methoxy)methyl)-N,N-dimethyl-1-phenylcyclohexanamine
18 1-Phenyl-4-[2-(*tert*-butyldimethylsilanyl)-1*H*-indol-3-ylmethoxymethyl]cyclohexyldimethylamine
19 1-Phenyl-4-(((2-(*tert*-butyldimethylsilyl)-5-fluoro-1H-indol-3-yl)methoxy)methyl)-N,N-dimethylcyclohexanamine
20 1-Phenyl-4-(((2-(*tert*-butyldimethylsilyi)-1H-pyrrolo[2,3-b]pyridin-3-yl)methoxy)methyl)-N,N-dimethylcyclohexanamine
22 {1-Benzyl-4-[2-(*tert*-butyldimethylsilanyl)-1*H*-indol-3-ylmethoxymethyl]cyclohexyl}dimethylamine
23 [1-Benzyl-4-(2-triethylsilanyl-1*H*-indol-3-ylmethoxymethyl)cyclohexyl]dimethylamine
24 [1-Benzyl-4-(1*H*-indol-3-ylmethoxymethyl)cyclohexyl]dimethylamine
25 1-Benzyl-4-(((5-fluoro-2-(triethylsilyl)-1H-indol-3-yl)methoxy)methyl)-N,N-dimethylcyclohexanamine
26 1-Benzyl-4-(((2-(*tert*-butyldimethylsilyl)-5-fluoro-1H-indol-3-yl)methoxy)methyl)-N,N-dimethylcyclohexanamine
27 1-Benzyl-4-(((5-fluoro-1H-indol-3-yl)methoxy)methy)-N,N-dimethylcyclohexanamine
28 1-Benzyl-N,N-dimethyl-4-(((2-(triethylsilyl)-1H-pyrrolo[2,3-b]pyridin-3-yl)methoxy)methyl)cyclohexanamine
29 1-Benzyl-4-(((2-(*tert*-butyldimethylsilyl)-1H-pyrrolo[2,3-b]pyridin-3-yl)methoxy)methyl)-N,N-dimethylcyclohexanamine
30 4-(((1H-Pyrrolo[2,3-b]pyridin-3-yl)methoxy)methyl)-1-benzyl-N,N-dimethylcyclohexanamine
31 1-Benzyl-N,N-dimethyl-4-(((2-(triethylsilyl)-1H-pyrrolo[3,2-c]pyridin-3-yl)methoxy)methyl)cyclohexanamine
32 1-Benzyl-4-(((2-(*tert*-butyldimethylsilyl)-1H-pyrrolo[3,2-c]pyridin-3-yl)methoxy)methyl)-N, N-dimethylcyclohexanamine
33 4-(((1H-Pyrrolo[3,2-c]pyridin-3-yl)methoxy)methyl)-1-benzyl-N,N-dimethylcyclohexanamine
34 1-Benzyl-N,N-dimethyl-4-(((2-(triethylsilyl)-1H-indol-3-yl)methoxy)methyl)cyclohexanamine
35 1-Benzyl-4-(((2-(*tert*-butyldimethylsilyl)-1H-indol-3-yl)methoxy)methyl)-N,N-dimethylcyclohexanamine
36 4-(((1H-Indol-3-yl)methoxy)methyl)-1-benzyl-N,N-dimethylcyclohexanamine
37 1-Benzyl-4-(((5-fluoro-2-(triethylsilyl)-1H-indol-3-yl)methoxy)methyl)-N,N-dimethylcyclohexanamine
38 1-Benzyl-4-(((2-(*tert*-butyldimethylsilyl)-5-fluoro-1H-indol-3-yl)methoxy)methyl)-N,N-dimethylcyclohexanamine
39 1-Benzyl-4-(((5-fluoro-1H-indol-3-yl)methoxy)methyl)-N,N-dimethylcyclohexanamine
40 1-Benzyl-N,N-dimethyl-4-(((2-(triethylsilyl)-1H-pyrrolo[2,3-b]pyridin-3-yl)methoxy)methyl)cyclohexanamine hydrochloride
41 4-(((1H-Pyrrolo[2,3-b]pyridin-3-yl)methoxy)methyl)-1-benzyl-N,N-dimethylcyclohexanamine
42 1-Benzyl-4-(((2-(*tert*-butyldimethylsilyl)-1H-pyrrolo[3,2-c]pyridin-3-yl)methoxy)methyl)-N,N-dimethylcyclohexanamine
43 1-Butyl-N,N-dimethyl-4-(((2-(triethylsilyl)-1H-indol-3-yl)methoxy)methyl)cyclohexanamine
44 4-(((1H-Indol-3-yl)methoxy)methyl)-1-butyl-N,N-dimethylcyclohexanamine
45 1-Butyl-4-(((5-fluoro-2-(triethylsilyl)-1H-indol-3-yl)methoxy)methyl)-N,N-dimethylcyclohexanamine
46 1-Butyl-4-(((5-fluoro-1H-indol-3-yl)methoxy)methyl)-N,N-dimethylcyclohexanamine
47 1-Butyl-N,N-dimethyl-4-(((2-(triethylsilyl)-1H-pyrrolo[2,3-b]pyridin-3-yl)methoxy)methyl)cyclohexanamine
48 4-(((1H-Pyrrolo[2,3-b]pyridin-3-yl)methoxy)methyl)-1-butyl-N,N-dimethylcyclohexanamine
49 1-Butyl-N,N-dimethyl-4-(((2-(triethylsilyl)-1H-pyrrolo[3,2-c]pyridin-3-yl)methoxy)methyl)cyclohexanamine
50 4-(((1H-Pyrrolo[3,2-c]pyridin-3-yl)methoxy)methyl)-1-butyl-N,N-dimethylcyclohexanamine
51 1-Butyl-4-(((5-fluoro-2-(triethylsilyl)-1H-indol-3-yl)methoxy)methyl)-N-methylcyclohexanamine
52 1-Butyl-N-methyl-4-(((2-(triethylsilyl)-1H-indol-3-yl)methoxy)methyl)cyclohexanamine
53 1-Butyl-N-methyl-4-(((2-(triethylsilyl)-5-(trifluoromethyl)-1H-indol-3-yl)methoxy)methyl)cyclohexanamine
54 1-Butyl-N-methyl-4-(((5-(trifluoromethyl)-1H-indol-3-yl)methoxy)methyl)cyclohexanamine
55 4-(((5-Fluoro-2-(triethylsilyl)-1H-indol-3-yl)methoxy)methyl)-N,N-dimethyl-1-(thiophen-2-yl)cyclohexanamine
56 4-(((5-Fluoro-1H-indol-3-yl)methoxy)methyl)-N,N-dimethyl-1-(thiophen-2-yl)cyclohexanamine
57 3-(((4-(Azetidin-1-yl)-4-phenylcyclohexyl)methoxy)methyl)-5-fluoro-2-(triethylsilyl)-1H-indole
58 3-(((4-(Azetidin-1-yl)-4-phenylcyclohexyl)methoxy)methyl)-5-fluoro-1H-indole 60 4-(((5-Fluoro-1-methyl-2-(triethylsilyl)-1H-indol-3-yl)methoxy)methyl)-N,N-dimethyl-1-phenylcyclohexanamine
61 4-(((5-Fluoro-1-methyl-1H-indol-3-yl)methoxy)methyl)-N,N-dimethyl-1-phenylcyclohexanamine (GRTE9052; WW447)
62 4-(((5-Fluoro-1-(methylsulfonyl)-1H-indol-3-yl)methoxy)methyl)-N,N-dimethyl-1-phenylcyclohexanamine
63 1-(3-(((4-(Dimethylamino)-4-phenylcyclohexyl)methoxy)methyl)-5-fluoro-1H-indol-1-yl)ethanone
64 4-(((2-*tert*-Butyl-5-fluoro-1H-indol-3-yl)methoxy)methyl)-N,N-dimethyl-1-phenylcyclohexanamine
65 3-(((4-(Azetidin-1-yl)-4-phenylcyclohexyl)methoxy)methyl)-2-(triethylsilyl)-1H-pyrrolo[2,3-b]pyridine
66 3-(((4-(Azetidin-1-yl)-4-phenylcyclohexyl)methoxy)methyl)-1H-pyrrolo[2,3-b]pyridine
67 4-(((2-*tert*-Butyl-1H-pyrrolo[2,3-b]pyridin-3-yl)methoxy)methyl)-N,N-dimethyl-1-phenylcyclohexanamine
in the form of the racemate; the enantiomers, diastereomers, mixtures of enantiomers or diastereomers or a single enantiomer or diastereomer; the bases and/or salts of physiologically acceptable acids or cations.

4. Method for preparing substituted 4-aminocyclohexane derivatives according to one of the preceding claims, 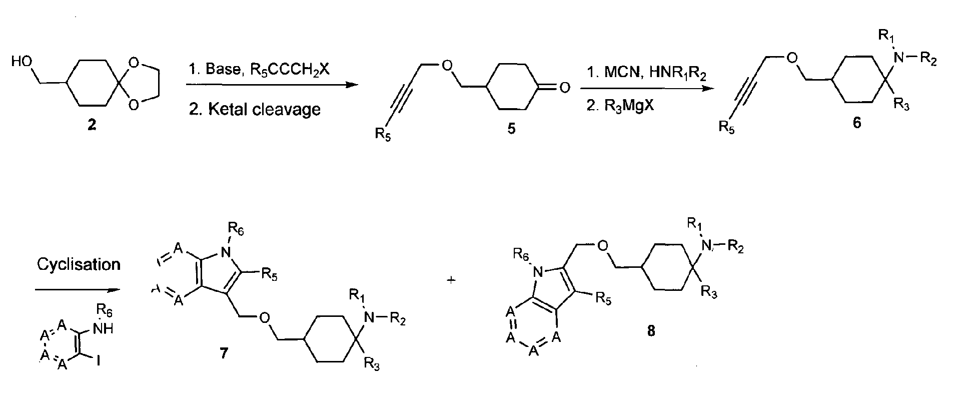 wherein
a) the ketal-protected 4-hydroxymethyl cyclohexanone **2** is reacted to substituted cyclohexanones having the general formula 5 in organic solvents, for example ether, tetrahydrofuran, dimethyl formamide, methanol, ethanol or dichloromethane, in the presence of an inorganic or organic base, for example potassium *tert*-butanolate, sodium methanolate or ethanolate, lithium or sodium hydride, potassium or sodium hydroxide, Proton Sponge, butyl lithium or other basic organometallic compounds such as Grignard reagents, for example ethyl magnesium chloride or bromide, optionally in the presence of phase-transfer catalysts, for example tetra-*n*-butylammonium chloride, tetra-*n*-butylammonium hydroxide or tetra-*n*-butylammonium iodide, and with an alkylating agent
R⁵CCCH₂X where X = Cl, Br
at temperatures of between -10 and 120°C with subsequent ketal cleavage
b) the substituted cyclohexanones having the general formula **5** are reacted in an aqueous medium or organic solvents, for example methanol, ethanol, *n*-propanol, tetrahydrofuran, acetonitrile or dichloromethane, or mixtures of these two media, in the presence of an inorganic acid, for example HCl, HBr, H₂SO₄, HClO₄ or H₃PO₄, a cyanide source, for example sodium or potassium cyanide, zinc cyanide, trimethylsilyl cyanide, acetone cyanohydrin, and an amine
HNR¹R²
or its hydrochloride
HCl-HNR¹R²
at temperatures of between 20 and 60°C and the intermediate that is obtained is reacted in organic solvents, for example hexane, pentane, toluene, ether, diisopropyl ether, tetrahydrofuran or methyl-*t*-butyl ether, with a Grignard reagent
MgXR³ where X = Cl, Br, I
at temperatures of between -10°C and 40°C to monosubstituted 4-alkynyloxymethyl cyclohexylamine derivatives having the general formula **6;**
c) monosubstituted 4-alkynyloxymethyl cyclohexylamine derivatives having the general formula **6** are reacted to the 4-aminocyclohexane derivatives according to the invention having the general formulae **7** and **8** in organic solvents, for example tetrahydrofuran, dimethyl formamide, benzene, toluene, xylenes, dimethoxyethane or diethylene glycol dimethyl ether, in the presence of an inorganic base, for example sodium, potassium or caesium carbonate or potassium phosphate, in the presence of PdCl₂, Pd(OAc)₂, PdCl₂(MeCN)₂, PdCl₂(PPh₃)₂ or [1,3-bis-(2,6-diisopropylphenyl)imidazol-2-ylidene]-(3-chloropyridyl)palladium(II) chloride (PEPPSI^{®}), optionally in the presence of additional ligands, for example triphenyl, tri-*o*-tolyl, tricyclohexyl or tri-*t*-butyl phosphine, optionally in the presence of phase-transfer catalysts, for example tetra-*n*-butylammonium chloride, tetra-*n-*butylammonium hydroxide or tetra-*n*-butylammonium iodide, at temperatures of between 60°C and 180°C, also with microwave assistance.

5. Medicinal product containing at least one substituted 4-aminocyclohexane derivative according to one of claims 1 to 3 and optionally containing suitable additives and/or auxiliary substances and/or optionally further active ingredients.

6. Use of a substituted 4-aminocyclohexane derivative according to one of claims 1 to 3 to prepare a medicinal product for the treatment of pain, in particular neuropathic pain, diabetic polyneuropathic pain or pain in postzosteric neuralgia.

## Revendications

1. Dérivés de 4-aminocyclohexane substitués de formule générale I 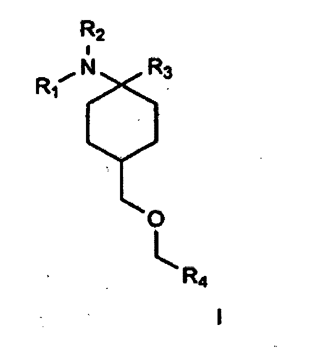 dans laquelle
R¹ et R² signifient indépendamment l'un de l'autre méthyle ou H, ou les radicaux R¹ et R² forment un cycle en incluant l'atome N et signifient ensemble (CH₂)₃ ;
R³ représente phényle, non substitué ou substitué une fois avec F, Cl, CN, CH₃ ; thiényle ; ou n-butyle, non substitué ou substitué une ou plusieurs fois avec OCH₃, OH ou OC₂Hₛ, notamment avec OCH₃ ;
R⁴ représente 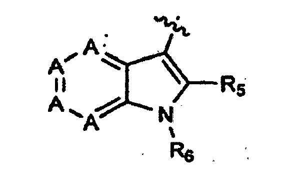 A représentant N ou CR⁷⁻¹⁰, A signifiant au plus une fois N ;
R⁵, R⁷, R⁸, R⁹ et R¹⁰ représentant indépendamment les uns des autres H, F, Cl, Br, CN, CH₃, C₂H₅, NH₂, tert.-butyle, Si(éthyle)₃, Si(méthyle)₂(tert.-butyle), SO₂CH₃, C(O)CH₃, NO₂, SH, CF₃, OH, OCH₃, OC₂H₅ ou N(CH₃)₂, notamment Si(éthyle)₃, Si(méthyle)₂(tert.-butyle), CN, CF₃, F, C(O)CH₃, SO₂CH₃ ou CH₃, et
R⁶ représentant H, CH₃ ou C(O)CH₃,
sous la forme du racémat ; des énantiomères, diastéréomères, mélanges des énantiomères ou diastéréomères ou d'un énantiomère ou diastéréomère individuel ; des bases et/ou des sels d'acides ou de cations physiologiquement compatibles.

2. Dérivés de 4-aminocyclohexane substitués selon la revendication 1, dans lesquels R⁴ signifie une des structures L, M ou N 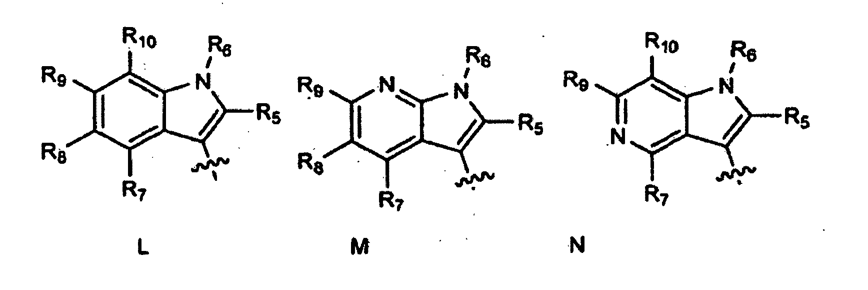 dans lesquelles les radicaux
R⁵ ,R⁷, R⁸, R⁹ et R¹⁰ représentent indépendamment les uns des autres H, F, Cl, Br, CN, CH₃, C₂H₅, NH₂, tert.-butyle, Si(éthyle)₃, Si(méthyle)₂(tert.-butyle), SO₂CH₃, C(0)CH₃, NO₂, SH, CF₃, OH, OCH₃, OC₂H₅ ou N(CH₃)₂, notamment Si(éthyle)₃, Si(méthyle)₂(tert.-butyle), CN, CF₃, F, C(O)CH₃, SO₂CH₃ ou CH₃, et
R⁶ représente H, CH₃ ou C(O)CH₃,

3. Dérivés de 4-aminocyclohexane substitués selon l'une quelconque des revendications précédentes, du groupe constitué par :
1 la [1-phényl-4-(2-triéthylsilanyl-1H-indol-3-ylméthoxyméthyl)cyclohexyl)diméthyl-amine
2 la [1-phényl-4-(1H-indol-3-ylméthoxyméthyl)cyclohexyl]diméthylamine
3 la 1-phényl-4-(((2-(tert-butyldiméthylsilyl)-5-cyano-1H-indol-3-yl)méthoxy)méthyl)-N,N-diméthylcyclohexanamine
4 la 1-phényl-4-(((5-cyano-2-(triéthylsilyl)-1H-indol-3-yl)méthoxy)méthyl)-N,N-diméthylcyclohexanamine
5 la 1-phényl-4-(((5-cyano-1H-indol-3-yl)méthoxy)méthyl)-N,N-diméthyl-cyclohexanamine
6 la 1-phényl-4-(((2-(tert-butyldiméthylsilyl)-5-trifluorométhyl-1H-indol-3-yl)méthoxy)méthyl)-N,N-diméthylcyclohexanamine
7 la 1-phényl-4-(((5-trifluorométhyl-2-(triéthylsilyl)-1H-indol-3-yl)méthoxy)méthyl)-N,N-diméthylcyclohexanamine
8 la 1-phényl-4-(((5-trifluorométhyl-1H-indol-3-yl)méthoxy)méthyl)-N,N-diméthyl-cyclohexanamine
9 la 1-phényl-4-(((5-fluoro-2-(triéthylsilyl)-1H-indol-3-yl)méthoxy)méthyl)-N,N-diméthylcyclohexanamine
10 la 1-phényl-4-(((5-fluoro-1H-indol-3-yl)méthoxy)méthyl)-N,N-diméthyl-cyclohexanamine
11 la 1-phényl-N,N-diméthyl-4-(((2,-triéthylsilyl)-1H-pyrrolo[2,3-bipyridin-3-yl)méthoxy)méthyl)cyclohexanamine
12 la 4-(((1H-pyrrolo[2,3-b]pyridin-3-yl)méthoxy)méthyl)-1-phényl-N,N-diméthylcyclohexanamine
13 la 1-phényl-4-(((2-(tert-butyldiméthylsilyl)-1H-pyrrolo[3,2-c]pyridin-3-yl)méthoxy)méthyl)-N,N-diméthylcyclohexanamine
14 la 1-phényl-N,N-diméthyl-4-(((2-(triéthylsilyl)-1H-pyrrolo[3,2-c]pyridin-3-yl)méthoxy)méthyl)cyclohexanamine
15 la 4-(((1H-pyrrolo[3,2-c]pyridin-3-yl)méthoxy)méthyl)-1-phény-N,N-diméthylcydohexanamine
16 la 1-(3-(((4-(diméthylamino)-4-phénylcyclohexyl)méthoxy)méthyl)-5-fluoro-2-(triéthylsilyl)-1H-indol-1-yl)éthanone
17 la 4-(((5-fluoro-1-(méthylsulfonyl)-2-(triéthylsilyl)-1H-indol-3-yl)méthoxy)méthyl)-N,N-diméthyl-1-phénylcyclohexanamine
18 la 1-phényl-4-[2-(tert-butyldiméthylsilanyl)-1H-indol-3-ylméthoxy-méthyl]cyclohexyl-diméthylamine
19 la 1-phényl-4-(((2-(tert-butyldiméthylsilyl)-5-fluoro-1H-indol-3-yl)méthoxy)méthyl)-N,N-diméthylcyclohexanamine
20 la 1-phényl-4-(((2-(tert-butyldiméthylsilyl)-1H-pyrrolo[2,3-b]pyridin-3-yl)méthoxy)méthyl)-N,N-diméthylcyclohexanamine
22 la {1-benzyl-4-[2-(tert-butyldiméthylsilanyl)-1H-indol-3-ylméthoxyméthyl]cyclohexyl}-diméthylamine
23 la [1-benzyl-4-(2-triéthylsilanyl-1H-indol-3-ylméthoxyméthyl)cyclohexyl]diméthylamine
24 la [1-benzyl-4-(1H-indol-3-ylméthoxyméthyl)cyclohexyl]diméthylamine
25 la 1-benzyl-4-(((5-fluoro-2-(triéthylsilyl)-1H-indol-3-yl)méthoxy)méthyl)-N,N-diméthylcyclohexanamine
26 la 1-benzyl-4-(((2-(tert-butyldiméthylsilyl)-5-fluoro-1H-indol-3-yl)méthoxy)méthyl)-N,N-diméthylcyclohexanamine
27 la 1-benzyl-4-(((5-fluoro-1H-indol-3-yl)méthoxy)méthyl)-N,N-diméthylcyclohexanamine
28 la 1-benzyl-N,N-diméthyl-4-(((2-(triéthylsilyl)-1H-pyrrolo[2,3-b]pyridin-3-yl)méthoxy)méthyl)cyclohexanamine
29 la 1-benzyl-4-(((2-(tert-butyldiméthylsilyl)-1H-pyrrolo[2,3-b]pyridin-3-yl)méthoxy)méthyl)-N,N-diméthylcyclohexanamine
30 la 4-(((1H-pyrrolo[2,3-d]pyridin-3-yl)méthoxy)méthyl)-1-benzyl-N,N-diméthylcyclohexanamine
31 la 1-benzyl-N,N-diméthyl-4-(((2-(triéthylsilyl)-1H-pyrrolo[3,2-c]pyridin-3-yl)méthoxy)méthyl)cyclohexanamine
32 la 1-benzyl-4-(((2-(tert-butyldiméthylsilyl)-1H-pyrrolo[3,2-c]pyridin-3-yl)méthoxy)méthyl)-N,N-diméthylcyclohexanamine
33 la 4-(((1H-pyrrolo[3,2-c]pyridin-3-yl)méthoxy)méthyl)-1-benzyl-N,N-diméthylcyclohexanamine
34 la 1-benzyl-N,N-diméthyl-4-(((2-(triéthylsilyl)-1H-indol-3-yl)méthoxy)méthyl)cyclohexanamine
35 la 1-benzyl-4-(((2-(tert-butyldiméthylsilyl)-1H-indol-3-yl)méthoxy)méthyl)-N,N-diméthylcyclohexanamine
36 la 4-(((1H-indol-3-yl)méthoxy)méthyl)-1-benzyl-N,N-diméthylcyclohexanamine
37 la 1-benzyl-4-(((5-fluoro-2-(triéthylsilyl)-1H-indol-3-yl)méthoxy)méthyl)-N,N-diméthylcyclohexanamine
38 la 1-benzyl-4-(((2-(tert-butyldiméthylsilyl)-5-fluoro-1H-indol-3-yl)méthoxy)méthyl)-N,N-diméthylcyclohexanamine
39 la 1-benzyl-4-(((5-fluoro-1H-indol-3-yl)méthoxy)méthyl)-N,N-diméthylcyclohexanamine
40 le chlorhydrate de 1-benzyl-N,N-diméthyl-4-(((2-(triéthylsilyl)-1H-pyrrolo[2,3-b]pyridin-3-yl)méthoxy)méthyl)cyclohexanamine
41 la 4-(((1H-pyrrolo[2,3-b]pyridin-3-yl)méthoxy)méthyl)-1-benzyl-N,N-diméthylcyclohexanamine
42 la 1-benzyl-4-(((2-(tert-butyldiméthylsilyl)-1H-pyrrolo[3,2-c]pyridin-3-yl)méthoxy)méthyl)-N,N-diméthylcyclohexanamine
43 la 1-butyl-N,N-diméthyl-4-(((2-(triéthylsilyl)-1H-indol-3-yl)méthoxy)méthyl)cyclohexanamine
44 la 4-(((1H-indol-3-yl)méthoxy)méthyl)-1-butyl-N,N-diméthylcydohexanamine
45 la 1-butyl-4-(((5-fluoro-2-(triéthylsilyl)-1H-indol-3-yl)méthoxy)méthyl)-N,N-diméthylcyclohexanamine
46 la 1-butyl-4-(((5-fluoro-1H-indol-3-yl)méthoxy)méthyl)-N,N-diméthylcyclohexanamine
47 la 1-butyl-N,N-diméthyl-4-(((2-(triéthylsilyl)-1H-pyrrolo[2,3-b]pyridin-3-yl)méthoxy)méthyl)cyclohexanamine
48 la 4-(((1H-pyrrolo[2,3-b]pyridin-3-yl)méthoxy)méthyl)-1-butyl-N,N-diméthylcyclohexanamine
49 la 1-butyl-N,N-diméthyl-4-(((2-(triéthylsilyl)-1H-pyrrolo[3,2-c]pyridin-3-yl)méthoxy)méthyl)cyclohexanamine
50 la 4-(((1H-pyrrolo[3,2-c]pyridin-3-yl)méthoxy)méthyl)-1-butyl-N,N-diméthylcyclohexanamine
51 la 1-butyl-4-(((5-fluoro-2-(triéthylsilyl)-1H-indol-3-yl)méthoxy)méthyl)-N-méthylcyclohexanamine
52 la 1-butyl-N-méthyl-4-(((2-(triéthylsilyl)-1H-indol-3-yl)méthoxy)méthyl)cyclohexanamine
53 la 1-butyl-N-méthyl-4-(((2-(triéthylsilyl)-5-(trifluorométhyl)-1H-indol-3-yl)méthoxy)méthyl)cyclohexanamine
54 la 1-butyl-N-méthyl-4-(((5-(trifluorométhyl)-1H-indol-3-yl)méthoxy)méthyl)cyclohexanamine
55 la 4-(((5-fluoro-2-(triéthylsilyl)-1H-indol-3-yl)méthoxy)méthyl)-N,N-diméthyl-1-(thiophén-2-yl)cyclohexanamine
56 la 4-(((5-fluoro-1H-indol-3-yl)méthoxy)méthyl)-N,N-diméthyl-1-(thiophén-2-yl)cyclohexanamine
57 le 3-(((4-(azétidin-1-yl)-4-phénylcyclohexyl)méthoxy)méthyl)-5-fluoro-2-(triéthylsilyl)-1H-indol
58 le 3-(((4-(azétidin-1-yl)-4-phénylcyclohexyl)méthoxy)méthyl)-5-fluoro-1H-indol 60 la 4-(((5-fluoro-1-méthyl-2-(triéthylsilyl)-1H-indol-3-yl)méthoxy)méthyl)-N,N-diméthyl-1-phénylcyclohexanamine
61 la 4-(((5-fluoro-1-méthyl-1H-indol-3-yl)méthoxy)méthyl)-N,N-diméthyl-1-phénylcyclohexanamine (GRTE9052 ; WW447)
62 la 4-(((5-fluoro-1-(méthylsulfonyl)-1H-indol-3-yl)méthoxy)méthyl)-N,N-diméthyl-1-phénylcyclohexanamine
63 la 1-(3-(((4-(diméthylamino)-4-phénylcyclohexyl)méthoxy)méthyl)-5-fluoro-1H-indol-1-yl)éthanone
64 la 4-(((2-tert-butyl-5-fluoro-1H-indol-3-yl)méthoxy)méthyl)-N,N-diméthyl-1-phénylcyclohexanamine
65 la 3-(((4-(azétidin-1-yl)-4-phénylcyclohexyl)méthoxy)méthyl)-2-(triéthylsilyl)-1H-pyrrolo[2,3-b]pyridine
66 la 3-(((4-(azétidin-1-yl)-4-phénylcyclohexyl)méthoxy)méthyl)-1H-pyrrolo[2,3-b]pyridine
67 la 4-(((2-tert-butyl-1H-pyrrolo[2,3-b]pyridin-3-yl)méthoxy)méthyl)-N,N-diméthyl-1-phénylcyclohexanamine sous la forme du racémat ; des énantiomères, diastéréomères, mélanges des énantiomères ou diastéréomères ou d'un énantiomère ou diastéréomère individuel ; des bases et/ou des sels d'acides ou de cations physiologiquement compatibles.

4. Procédé de fabrication de dérivés de 4-aminocyclohexane substitués selon l'une quelconque des revendications précédentes, 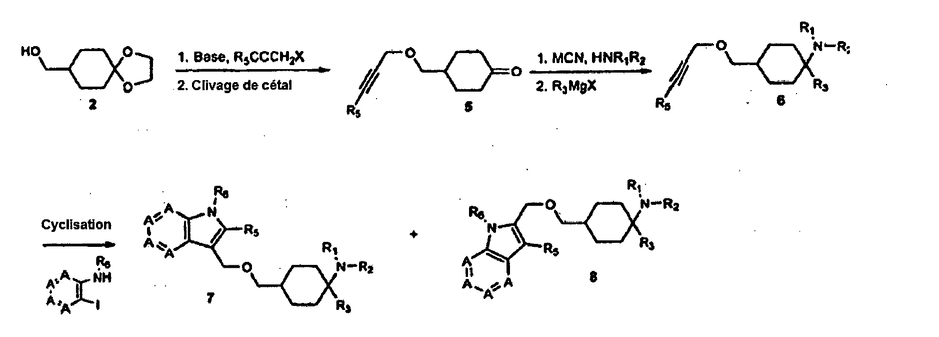 selon lequel
a) la 4-hydroxyméthyl-cyclohexanone à protection cétal 2 est mise en réaction dans des solvants organiques, par exemple un éther, le tétrahydrofurane, le diméthylformamide, le méthanol, l'éthanol ou le dichlorométhane, en présence d'une base inorganique ou organique, par exemple le tert-butanolate de potassium, le méthanolate ou l'éthanolate de sodium, l'hydrure de lithium ou de sodium, l'hydroxyde de potassium ou de sodium, une éponge à protons, le butyllithium ou d'autres composés organométalliques basiques tels que les réactifs de Grignard, par exemple le chlorure ou le bromure d'éthylmagnésium, éventuellement en présence de catalyseurs de transfert de phase, par exemple le chlorure de tétra-n-butylammonium, l'hydroxyde de tétra-n-butylammonium ou l'iodure de tétra-n-butylammonium, et avec un agent d'alkylation
R⁵CCCH₂X avec X = Cl, Br
à des températures comprises entre -10 et 120 °C, puis le cétal est clivé pour former des cyclohexanones substituées de formule générale 5,
b) les cyclohexanones substituées de formule générale 5 sont mises en réaction dans un milieu aqueux ou dans des solvants organiques, par exemple le méthanol, l'éthanol, le n-propanol, le tétrahydrofurane, l'acétonitrile ou le dichlorométhane, ou des mélanges de ces deux milieux, en présence d'un acide inorganique, par exemple HCl, HBr, H₂SO₄, HClO₄ ou H₃PO₄, d'une source de cyanure, par exemple le cyanure de sodium ou de potassium, le cyanure de zinc, le cyanure de triméthylsilyle, la cyanhydrine d'acétone, et d'une amine
HNR¹R²
ou son chlorhydrate
HCl ·HNR¹R²
à des températures comprises entre 20 et 60 °C, et les intermédiaires obtenus sont mis en réaction dans des solvants organiques, par exemple l'hexane, le pentane, le toluène, un éther, l'éther diisopropylique, le tétrahydrofurane ou l'éther de méthyle et de t-butyle, avec un réactif de Grignard
MgXR³ avec X = Cl, Br, I
à des températures comprises entre -10 °C et 40 °C pour former des dérivés de 4-alcynyloxyméthyl-cyclohexylamine 1-substitués de formule générale 6 ;
c) les dérivés de 4-alcynyloxyméthyl-cyclohexylamine 1-substitués de formule générale 6 sont mis en réaction dans des solvants organiques, par exemple le tétrahydrofurane, le diméthylformamide, le benzène, le toluène, des xylènes, le diméthoxyéthane ou l'éther diméthylique de diéthylène glycol, en présence d'une base inorganique, par exemple le carbonate de sodium, de potassium ou de césium, ou le phosphate de potassium, en présence de PdCl₂, Pd(OAc)₂, PdCl₂(MeCN)₂, PdCl₂ (PPh₃)₂ ou du chlorure de [1,3-bis-(2,6-diisopropylphényl)imidazol-2-ylidène]-(3-chloropyridyl)palladium (II) (PEPPSI^{®}), éventuellement en présence de ligands supplémentaires, par exemple la triphényl-, tri-o-tolyl-, tricyclohexyl- ou tri-t-butylphosphine, éventuellement en présence de catalyseurs de transfert de phase, par exemple le chlorure de tétra-n-butylammonium, l'hydroxyde de tétra-n-butylammonium ou l'iodure de tétra-n-butylammonium, à des températures comprises entre 60 °C et 180 °C, également avec l'assistance de micro-ondes, pour former les dérivés de 4-aminocyclohexane de formules générales 7 et 8 selon l'invention.

5. Médicament contenant au moins un dérivé de 4-aminocyclohexane substitué selon l'une quelconque des revendications 1 à 3, ainsi qu'éventuellement contenant des additifs et/ou adjuvants appropriés et/ou éventuellement d'autres agents actifs.

6. Utilisation d'un dérivé de 4-aminocyclohexane substitué selon l'une quelconque des revendications 1 à 3 pour la fabrication d'un médicament pour le traitement de la douleur, notamment de la douleur neuropathique, de la douleur polyneuropathique diabétique ou de la douleur dans le cadre d'une névralgie post-herpétique.
